(19) Europäisches Patentamt
European Patent Office
Office européen des brevets

(11) EP 0 893 423 B1

(12) EUROPEAN PATENT SPECIFICATION

(45) Date of publication and mention of the grant of the patent:
**01.09.2004 Bulletin 2004/36**

(21) Application number: **97916638.6**

(22) Date of filing: **09.04.1997**

(51) Int Cl.[7]: **C07C 43/12**, C07C 43/172, C07C 43/174, C07C 43/176, C07C 43/225, C07C 255/54, C09K 19/20, C09K 19/30, C09K 19/42, G02F 1/13

(86) International application number:
**PCT/JP1997/001213**

(87) International publication number:
**WO 1997/037959 (16.10.1997 Gazette 1997/44)**

(54) **LIQUID CRYSTAL COMPOUNDS HAVING FLUORINATED LINKAGES, LIQUID CRYSTAL COMPOSITIONS, AND LIQUID CRYSTAL DISPLAY DEVICES**

FLÜSSIGKRISTALLINE VERBINDUNGEN MIT FLUORIERTEN BINDUNGEN, FLÜSSIGKRISTALLINE ZUSAMMENSETZUNGEN UND FLÜSSIGKRISTALLINES ANZEIGEELEMENT

COMPOSES DE CRISTAUX LIQUIDES COMPORTANT DES LIAISONS FLUOREES, COMPOSITIONS DE CRISTAUX LIQUIDES ET DISPOSITIFS D'AFFICHAGE A CRISTAUX LIQUIDES

(84) Designated Contracting States:
**DE FR GB**

(30) Priority: **10.04.1996 JP 8850796**

(43) Date of publication of application:
**27.01.1999 Bulletin 1999/04**

(73) Proprietor: **CHISSO CORPORATION**
**Osaka-shi Osaka 530-6591 (JP)**

(72) Inventors:
- **MATSUI SHUICHI**
  **Ichihara-shi, Chiba 290 (JP)**
- **KOIZUMI, Yasuyuki**
  **6358-1, Goi, Ichihara-shi, Chiba 290 (JP)**
- **MIYAZAWA, Kazutoshi**
  **Ichihara-shi, Chiba 290-01 (JP)**
- **SEKIGUCHI, Yasuko**
  **Ichihara-shi, Chiba 290 (JP)**
- **NAKAGAWA, Etsuo**
  **Ichihara-shi, Chiba 290 (JP)**

(74) Representative: **HOFFMANN - EITLE**
**Patent- und Rechtsanwälte**
**Arabellastrasse 4**
**81925 München (DE)**

(56) References cited:
**DE-A- 19 531 165**
**JP-A- 2 289 529**
**JP-A- 5 112 778**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 893 423 B1

## Description

TECHNICAL FIELD

The present invention relates to liquid crystalline compounds having a fluorine substituted bonding group and used as novel liquid crystalline compounds which develop preferable physical properties in liquid crystal compositions principally for TN, STN, or TFT; relates to liquid crystal compositions comprising the liquid crystalline compound; and further relates to liquid crystal display devices.

BACKGROUND ART

Liquid crystal display devices utilize optical anisotropy and dielectric anisotropy of liquid crystal substances. The liquid crystal display devices are widely used in tabletop calculators, word processors, and television sets, including watches, and demand for the display devices is in a trend to increase year by year. Liquid crystal phase exists between solid phase and liquid phase, and is divided broadly into nematic phase, smectic phase, and cholesteric phase. Among them, nematic phase is most widely employed for display devices at present. On the other hand, while many display modes were devised up to date, three types of twist nematic (TN) mode, super twist nematic (STN) mode, and thin film transistor (TFT) mode have now become main currents. Properties required of liquid crystal substances for these various liquid crystal display devices are different depending on their uses, but any of the liquid crystal substances is required to be stable against outside environmental factors such as moisture, air, heat, and light; to exhibit liquid crystal phase at a temperature range as wide as possible with room temperature being its center; to be in a low viscosity; and to have a low driving voltage. However, no liquid crystal substances which satisfy such requirements at the same time by a single compound have been found.

With respect to liquid crystal substances used for liquid crystal display devices, it is an actual circumstance that several kind or twenty-odd kind of liquid crystalline compounds, and further several kind of liquid non-crystalline compounds when necessary, are mixed to produce liquid crystal compositions and used for display devices, in order to obtain most suitable physical properties such as dielectric anisotropy ($\Delta\varepsilon$), optical anisotropy ($\Delta n$), viscosity, and elastic constant ratio $K_{33}/K_{11}$ ($K_{33}$: bend elastic constant, $K_{11}$: splay elastic constant) usually required of each display device. Accordingly, liquid crystalline compounds are required to be excellent in miscibility with other liquid crystal compounds, and recently in particular required to be excellent in the solubility even at low temperatures from the requirement of being used in various environments.

Meanwhile, active matrix mode, especially thin film transistor (TFT) mode is extensively adopted in recent years as display mode, for example, for television sets and viewfinders from the aspect of display performances such as contrast, display capacity, and response time. Also, STN mode which has a large display capacity and display devices of which mode are simple in structure and can be produced at a low production cost compared with display devices of active matrix mode is largely adopted in displays, for example, for personal computers.

Recent development in these fields is being progressed while placing stress on

(a) downsizing of liquid crystal display devices into a portable size as shown by the development of small size TV sets and notebook size personal computers both of which are characterized by being portable;
(b) display on a screen by using a mouse; and
(c) high speed response to cope with dynamic image display such as the images on television screen.

With these facts for a background, liquid crystal substances which have a low driving voltage, that is, a low threshold voltage, and are capable of responding at a high speed are required. Further, since the uses of liquid crystal display devices with the uses in outdoor life being their center are now rapidly increasing, liquid crystal materials which are stable against the changes in outside environments such as atmospheric temperature, moisture, and ultraviolet light are also required.

It is known that threshold voltage ($V_{th}$) can be expressed by the following equation (H. J. Deuling et al., Mol. Cryst. Liq. Cryst., 27 (1975) 81):

$$V_{th} = \pi\ (K/\ \varepsilon_0 \Delta\ \varepsilon)^{1/2}$$

In the equation described above, K is an elastic constant and $\varepsilon_0$ is a dielectric constant in vacuum. As will be seen from this equation, two methods, that is, a method of increasing dielectric anisotropy ($\Delta\varepsilon$) and a method of lowering elastic constant can be considered to lower the threshold voltage. However, since actual control of elastic constant is very difficult, liquid crystal compositions with a purpose of being driven at a low voltage are usually produced by using

a large amount of liquid crystalline compounds having a high dielectric anisotropy.

With respect to the response speed, it has already been reported that the viscosity is an important factor which controls the response speed of liquid crystal molecules oriented in a liquid crystal panel to electric field (Phys. Lett., 39A, 69 (1972)). That is, it is preferable to use a large amount of a liquid crystalline compound having an extremely low viscosity in order to produce liquid crystal compositions having a high speed responsibility.

From the facts described above, it can be considered that liquid crystal compositions which have a low driving voltage and are capable of responding at a high speed are readily obtained by using a liquid crystalline compound having a high dielectric anisotropy and a low viscosity. However, liquid crystalline compounds having a high dielectric anisotropy and a low viscosity are not known as a matter of fact at present. This is because the value of dielectric anisotropy is known in the art to be usually in proportional relation with viscosity though it is not simple, and thus the viscosity increases as the value of the dielectric anisotropy increases.

As liquid crystalline compounds for low voltage used in TFT mode, the compounds of the following formula (10) or (11) are described in Japanese Patent Publication No. Sho 63-44132 and U.S. Patent No. 5,032,313, respectively:

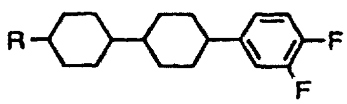

(10) Japanese Patent Publication No. Sho 63-44132

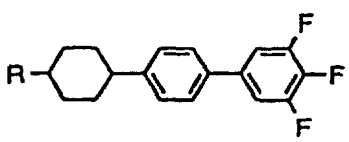

(11) U.S. Patent No. 5,032,313

in the structural formula described above, R represents an alkyl group.

Compounds of the formula (10) or (11) have fluorine atom as a substituent at a terminal of the molecule, and it is disclosed in these publications that the compounds exhibit a comparatively large dielectric anisotropy value. However, according to the consideration by the present inventors, both of the compounds have a problem in the miscibility with other liquid crystalline compounds, particularly in the miscibility at cryogenic temperatures around -40°C since they have a straight ring structure, and such defects that crystals are separated or smectic phase is developed in liquid crystal compositions at low temperatures are anticipated with the compounds. Compounds of the formula (11) are also anticipated to exhibit a comparatively high viscosity. In order to overcome these problems, the compounds expressed by one of the following formulas (12) to (16) are proposed in which compounds various bonding groups are inserted between two six-membered rings which form the skeleton structure of the molecule with the purpose of reducing the rigidity of the skeleton structure and increasing the miscibility:

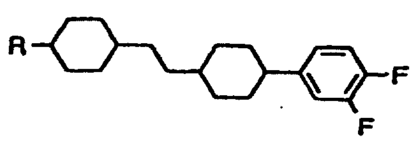

(12) Japanese Patent Publication No. Hei 2-44818

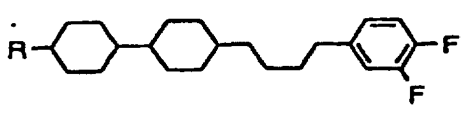

(13) Laid-open Japanese Patent Publication No. Hei 3-66632

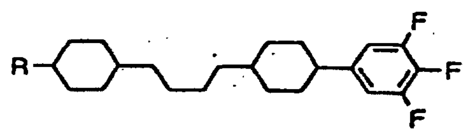

(14) Laid-open Japanese Patent Publication No. Hei 5-310605

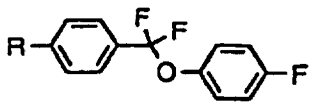

(15) Laid-open Japanese Patent Publication No. Hei 5-112778

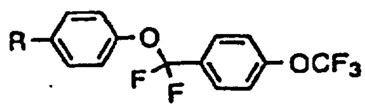

(16) Laid-open Japanese Patent Publication No. Hei 2-289529

in the structural formula described above, R represents an alkyl group.

Among these compounds, the compounds of the formula (12) have 1,2-ethylene group as bonding group, and the compounds of the formula (13) or (14) have 1,4-butylene group. Compounds of the formulas (15) or (16) have difluoromethyleneoxy ($-CF_2O-$) group as bonding group. According to the study by the present inventors, whereas the compounds of the formula (12) exhibit a comparatively excellent miscibility, their miscibility at cryogenic temperatures is still insufficient. Whereas the compounds of the formula (13) or (14) exhibit a remarkably excellent miscibility at low temperatures owing to 1,4-butylene group inserted therein, they have a considerably low clearing point since the bonding group remarkably deteriorates the rigidity, and they can not be expected to have a wide temperature range of liquid crystal phase. With respect to the compounds of the formula (15) or (16), physical data of the compounds and descriptions for evaluating their utility as liquid crystalline compounds are not included in the patent publications. In the compounds of the formula (15), the dipole moment of two fluorine atoms of difluoromethyleneoxy group, as bonding group, effectively contributes to the increase of the dipole moment of fluorine atom, as substituent, at a terminal of the molecule, and thus, the compounds have a comparatively high dielectric anisotropy and are anticipated to exhibit a comparatively excellent miscibility at low temperatures as well. However, a wide temperature range of liquid crystal phase can not be expected from the compounds as in the case of the compounds of the formula (13) or (14), since they have a two ring structure and difluoromethyleneoxy group, as bonding group, largely deteriorates the rigidity and straightness of the molecule. In the compounds of the formula (16), the direction of dipole moment of difluoromethyleneoxy group, as bonding group, faces to the direction opposite to that of trifluoromethoxy group, as substituent at a terminal, and thus, a high dielectric anisotropy can not be expected from the compounds. Any compounds expressed by one of the formulas (12) to (16) can readily be anticipated to have a high viscosity compared with the compounds of a directly-linked ring structure (for example, the compounds of the formula (10)) having no bonding group.

As described above, compounds which exhibit a comparatively wide temperature range of liquid crystal phase, have a high dielectric anisotropy and a low viscosity, and are remarkably excellent in solubility at low temperatures at the same time have not yet been found. Accordingly, compounds having improved characteristics for solving these problems have been long-awaited.

DISCLOSURE OF THE INVENTION

An object of the present invention is to provide novel liquid crystalline compounds

1) exhibiting a low threshold voltage,
2) having a low viscosity, and
3) having an excellent property in miscibility with other known liquid crystalline compounds, particularly in the miscibility at low temperatures,

as liquid crystal material capable of being driven at a low voltage and capable of responding at a high speed; liquid crystal compositions comprising the compound; and liquid crystal display devices comprising the composition therein.

With respect to the compounds having difluoromethyleneoxy group as bonding group, the compounds of the formula (15) or (16) described in the section of BACKGROUND ART are disclosed in Laid-open Japanese Patent Publication No. Hei 5-112778 and Laid-open Japanese Patent Publication No. Hei 2-289529, respectively. Whereas the structural formula for several compounds is described in the publications, physical data of the compounds and specific values of physical properties of the compounds necessary for evaluating their utility as liquid crystalline compound are not disclosed at all in the publications, and thus, the characteristics of the bonding group were not known in the least.

Under such circumstances, 1,4-butylene group, which deteriorates the liquid crystallinity of such compounds, but remarkably improve miscibility at low temperatures, was noticed by the present inventors; and diligent investigation was continued on the compounds which have a bonding group formed by substituting difluoromethyleneoxy group for two adjacent methylene ($CH_2$) groups, and are expressed by the general formula (1) shown below, to find out that the compounds not only maintain an excellent miscibility at low temperatures and exhibit a remarkably high dielectric anisotropy which are characteristics of 1,4-butylene group, but also have an extremely low viscosity than expected at first by the present inventors. Besides, it was found out that the liquid crystallinity which is a disadvantage of 1,4-butylene group is also improved by the substitution of difluoromethyleneoxy group, that the compounds exhibit a comparatively wide temperature range of liquid crystal phase, and that the compounds are remarkably stable against the changes of outside environments such as ultraviolet light and heat as well.

Further, it was found out that whereas the compounds of the present invention expressed by the general formula (1) wherein an alkyl group is selected as substituent $R^1$, and fluorine atom, chlorine atom, $CF_3$ group, $OCF_3$ group, or $OCHF_2$ group is selected as R2 have a low viscosity, the compounds exhibit a large dielectric anisotropy value, a high voltage holding ratio, and properties preferable for liquid crystal materials used for TFT mode display devices, and particularly preferable for those used for low voltage driving or a high speed response; and, on the other hand, that the compounds of the present invention expressed by the general formula (1) wherein an alkyl group or alkenyl group is selected as substituent $R^1$, and an alkenyl group or CN group is selected as R2 have an extremely low viscosity and a high clearing point, and thus are very useful as viscosity reducing agent for STN display devices, leading to the discovery of compounds of the present invention as novel liquid crystalline substance

In order to achieve the subjects described above, the present invention is summarized as follows:

(1) A liquid crystalline compound having a fluorine substituted bonding group and expressed by the general formula (1)

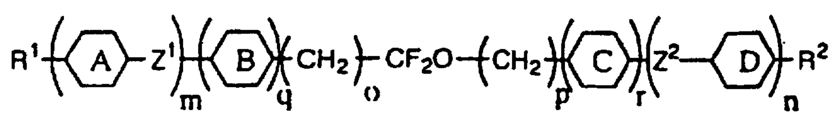
(1)

wherein $R^1$ and $R^2$ independently represent a straight chain or branched alkyl group having 1 to 15 carbon atoms or an alkenyl group having 2 to 15 carbon atoms, a halogen atom, CN group, $CF_3$ group, $OCF_3$ group, or $OCHF_2$ group, one or more hydrogen atoms in the alkyl or alkenyl group may be replaced by a halogen atom, and one or not-adjacent two or more $CH_2$ groups in the alkyl or alkenyl group may be replaced by oxygen atom, ring A, ring B, ring C, and ring D independently represent 1,4-cyclohexylene group or 1,4-phenylene group one or more hydrogen atoms on the ring may be replaced by a fluorine atom; $Z^1$ and $Z^2$ independently represent $-(CH_2)_2-$, -CH=CH-, -C≡C-, or single bond; m and n are independently an integer of 0 or 1; q and r are each 1; o and p are independently an integer of 0 to 2 provided that o + p = 2; and an element which constitutes the compound may be replaced by its isotope.

(2) The compound recited in paragraph (1) above wherein o is 2 and p is 0 in the general formula (1).

(3) The compound recited in paragraph (1) above wherein o is 1 and p is 1 in the general formula (1).

(4) The compound recited in paragraph (1) above wherein o is 0 and p is 2 in the general formula (1).

(5) The compound recited in paragraph (1) above wherein both ring B and ring C are 1,4-phenylene group.

(6) The compound recited in paragraph (4) above wherein ring B is 1,4-phenylene group in the general formula (1).

(7) A liquid crystal composition comprising at least two components and comprising at least one liquid crystalline compound recited in any of the paragraphs (1) to (6) above.

(8) A liquid crystal composition comprising, as a first component, at least one liquid crystalline compound recited in any one of paragraphs (1) to (6) above, and comprising, as a second component, at least one compound selected from the group consisting of the compounds expressed by any one of the general formulas (2), (3), and (4)

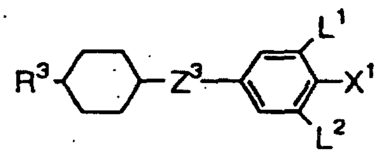

(2)

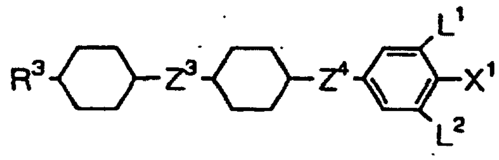

(3)

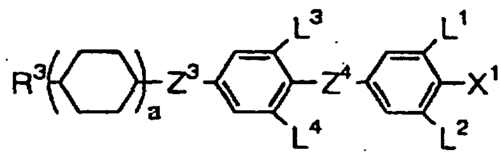

(4)

wherein $R^3$ represents an alkyl group having 1 to 10 carbon atoms; $X^1$ represents F, Cl, $OCF_3$, $OCF_2H$, $CF_3$, $CF_2H$, or $CFH_2$; $L^1$, $L^2$, $L^3$, and $L^4$ independently represent H or F; $Z^3$ and $Z^4$ independently represent $-(CH_2)_2-$, -CH=CH-, or single bond; and a is 1 or 2.

(9) A liquid crystal composition comprising, as a first component, at least one liquid crystalline compound recited in any one of paragraphs (1) to (6), and comprising, as a second compound, at least one compound selected from the group consisting of the compounds expressed by any one of the general formulas (5), (6), (7), (8), and (9)

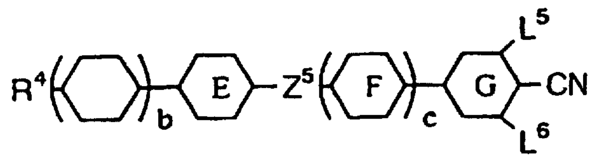

(5)

wherein $R^4$ represents F, an alkyl group having 1 to 10 carbon atoms, or an alkenyl group having 2 to 10 carbon atoms any methylene group ($-CH_2-$) in the alkyl or alkenyl group may be replaced by oxygen atom (-O-), but in no case two or more methylene groups are continuously replaced by oxygen atom; ring E represents trans-1,4-cyclohexylene group, 1,4-phenylene group, pyrimidine-2,5-diyl group, or 1,3-dioxane-2,5-diyl group; ring F represents trans-1,4-cyclohexylene group, 1,4-phenylene group, or pyrimidine-2,5-diyl group; ring G represents trans-1,4-cyclohexylene group or 1,4-phenylene group; $Z^5$ represents - $(CH_2)_2-$, -COO-, or single bond; $L^5$ and $L^6$ independently represent H or F; and b and c are independently 0 or 1,

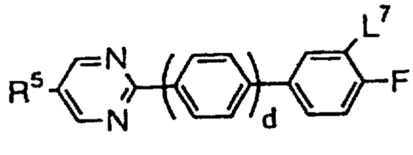

(6)

wherein $R^5$ represents an alkyl group having 1 to 10 carbon atoms, $L^7$ represents H or F; and d is 0 or 1,

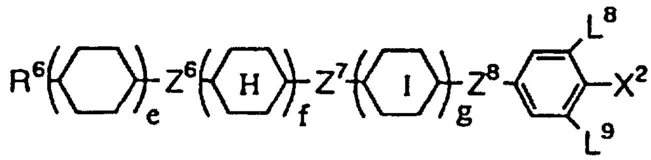

(7)

wherein R6 represents an alkyl group having 1 to 10 carbon atoms; ring H and ring I independently represent trans-1,4-cyclohexylene group or 1,4-phenylene group; $Z^6$ and $Z^7$ independently represent -COO- or single bond; $Z^8$ represents - COO- or -C≡C-; $L^8$ and $L^9$ independently represent H or F; $X^2$ represents F, $OCF_3$, $OCF_2H$, $CF_3$, $CF_2H$, or $CFH_2$ provided that when $X^2$ represents $OCF_3$, $OCF_2H$, $CF_3$, $CF_2H$, or $CFH_2$, then both $L^8$ and $L^9$ represent H; and e, f, and g are independently 0 or 1,

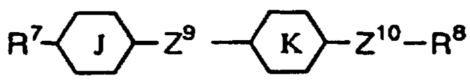

(8)

wherein $R^7$ and $R^8$ independently represent an alkyl group having 1 to 10 carbon atoms or an alkenyl group having 2 to 10 carbon atoms any methylene group ($-CH_2-$) in the alkyl or alkenyl group may be replaced by oxygen atom (-O-), but in no case two or more methylene groups are continuously replaced by oxygen atom; ring J represents trans-1,4-cyclohexylene group, 1,4-phenylene group, or pyrimidine-2,5-diyl group; ring K represents trans-1,4-cyclohexylene group or 1,4-phenylene group; $Z^9$ represents -C≡C-. -COO-, $-(CH_2)_2-$, -CH=CH-C≡C-, or single bond; and $Z^{10}$ represents - COO- or single bond;

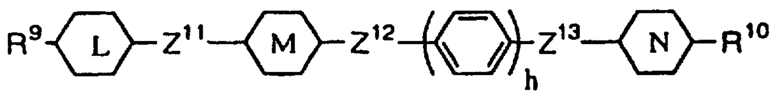

(9)

wherein $R^9$ and $R^{10}$ independently represent an alkyl group having 1 to 10 carbon atoms or an alkenyl group having 2 to 10 carbon atoms any methylene group ($-CH_2-$) in the alkyl or alkenyl group may be replaced by oxygen atom (-O-), but in no case two or more methylene groups are continuously replaced by oxygen atom; ring L represents trans-1,4-cyclohexylene group, 1,4-phenylene group, or pyrimidine-2,5-diyl group, ring M represents trans-1,4-cyclohexylene group, 1,4-phenylene group one or more hydrogen atoms on the ring may be replaced by F, or pyrimidine-2,5-diyl group; ring N represents trans-1,4-cyclohexylene group or 1,4-phenylene group; $Z^{11}$ and $Z^{13}$ independently represent -COO-, $-(CH_2)_2-$, or single bond; $Z^{12}$ represents -CH=CH-, -C≡C-, -COO-, or single bond; and h is 0 or 1.

(10) A liquid crystal composition comprising, as a first component, at least one liquid crystalline compound recited in any one of paragraphs (1) to (6), comprising, as a part of a second component, at least one compound selected from the group consisting of the compounds expressed by any one of the general formulas (2), (3), and (4), and comprising, as another part of the second component, at least one compound selected from the group consisting of the compounds expressed by any one of the general formulas (5), (6), (7), (8), and (9).

(11) A liquid crystal display device comprising a liquid crystal composition recited in any one of paragraphs (7) to (10).

Compounds of the present invention expressed by the general formula (1) are very stable against outside environments, are exceedingly low in viscosity, and have a high dielectric anisotropy. The compounds of the general formula (1) are excellent in miscibility with other liquid crystal compounds, particularly in the miscibility at low temperatures. Further, the compounds of the present invention expressed by the general formula (1) wherein an alkyl group is selected for $R^1$, and a group other than CN group, or a halogen atom is selected for substituent $R^2$ exhibit a high voltage holding ratio and properties preferable for liquid crystal materials used for TFT mode display devices, particularly for those used for low voltage driving or high speed response; and on the other hand, the compounds of the present invention expressed by the general formula (1) wherein an alkenyl group or an alkyl group is selected for substituent $R^1$ and an alkenyl group or CN group is selected for $R^2$ have an exceedingly low viscosity or a high clearing point, and thus are very useful as viscosity reducing agent for STN display devices.

By the use of the compounds of the present invention, novel liquid crystal compositions and liquid crystal

display devices can be provided which are stable against outside environments, capable of being driven at a low voltage, and capable of responding at a high speed.

As will be clear from the examples described below, excellent properties, particularly a low viscosity, high dielectric anisotropy, and excellent miscibility at low temperatures of the compounds of the present invention can be considered to be caused by a central bonding group formed by substituting difluoromethyleneoxy ($-CF_2O-$) group for adjacent two methylene groups in 1,4-butylene group.

As described in the section of BACKGROUND ART, compounds having no central bonding group or compounds having 1,2-ethylen group, 1,4-butylene group, or difluoromethyleneoxy group are already disclosed in Japanese Patent Publication No. Sho 63-44132, U.S. Patent No. 5,032,313, Laid-open Japanese Patent Publication No. Hei 2-289529, Laid-open Japanese Patent Publication No. Hei 3-66632, Laid-open Japanese Patent Publication No. 5-112778, or Laid-open Japanese Patent Publication No. Hei 5-310605. However, those compounds can not be said to be excellent as liquid crystal materials in the comprehensive balance of the following 1) to 3):

1) capability of being driven at a low voltage
2) capability of responding at a high speed
3) miscibility, particularly miscibility at low temperatures,

and thus, liquid crystal materials excellent in the balance of these characteristics were necessary.

Compounds of the present invention can be used to produce liquid crystal compositions meeting various purposes by selecting proper $R^1$, $R^2$, rings A, B, C, and D, $Z^1$, $Z^2$, m, n, o, and p in the general formula (1).

That is, when the compounds are used to produce liquid crystal compositions which have the temperature range of liquid crystal phase at high temperature side, it is sufficient to use four ring compounds wherein m and n are 1, and when the circumstance is not so, it is sufficient to use two ring or three ring compounds.

When an especially high voltage holding ratio is required of liquid crystal compositions as in the case of those for active matrix, it is sufficient to select an alkyl group as side chain $R^1$, and select a group other than CN group, or a halogen atom as $R^2$ in the general formula (1). When the compounds are used as viscosity reducing agent, it is sufficient to select an alkyl group or alkenyl group as side chain $R^1$ and select an alkyl group, alkenyl group, or CN group as $R^2$.

In order to obtain compounds having a comparatively large dielectric anisotropy value, it is sufficient to select 1,4-phenylene group for ring C or ring D, and select a halogen atom, CN group, $CF_3$ group, $OCF_3$ group, or $OCHF_2$ group for $R^2$. In order to cope with the demand for larger dielectric anisotropy value, it is sufficient to substitute one or two fluorine atoms at the ortho position of R2 on ring C or ring D, and the purpose can be attained by introducing fluorine atoms so that the dipoles of substituents face the same direction. Further, the compounds in which fluorine atom substituted at a lateral position of ring A, ring B, ring C, or ring D exhibit excellent characteristics in miscibility, particularly in that at low temperatures compared with the compounds having no substituted fluorine atom.

Optical anisotropy value can also optionally be adjusted by selecting proper $R^1$, $R^2$, rings A, B, C, and D, $Z^1$, $Z^2$, m, n, o, and p in the general formula (1). That is, when a large optical anisotropy value is necessary, it is sufficient to select compounds having many 1,4-phenylene rings, and having single bond for $Z^1$ and $Z^2$. When a small optical anisotropy value is required, it is sufficient to select compounds having many trans-1,4-cyclohexylene.

For the purpose of the present invention, the term "alkyl group" means a straight chain or branched alkyl group having 1 to 15 carbon atoms, and the alkyl group having 1 to 5 carbon atoms is preferable particularly from the viewpoint of low viscosity. Specifically, methyl group, ethyl group, propyl group, butyl group, pentyl group, hexyl group, heptyl group, octyl group, nonyl group, isopropyl group, isobutyl group, isoamyl group, isohexyl group, 2-methylbutyl group, 2-methylpentyl group, and 3-methylpentyl group are preferable, and racemic modifications, S isomers, and R isomers are comprehended.

For the purpose of the present invention, the term "alkenyl group" means a straight chain alkenyl group having 2 to 15 carbon atoms, and vinyl, 1E-alkenyl, 2Z-alkenyl, 3-alkenyl, 3E-alkenyl, and 4-alkenyl group are preferable. More specifically, 1-ethenyl, 1E-propenyl, 1E-butenyl, 1E-hexenyl 2-propenyl, 2Z-butenyl, 2Z-pentenyl, 2Z-hexenyl, 3-butenyl, 3E-pentenyl, and 3E-hexenyl can be mentioned.

The rings A, B, C, and D represent a cyclohexane ring, a benzene ring or a fluorine-substituted benzene ring.

BEST MODE FOR CARRYING OUT THE INVENTION

In the present invention, preferable embodiments of the compounds expressed by the general formula (1) are those expressed by one of the following general formulas (1-a) to (1-c)

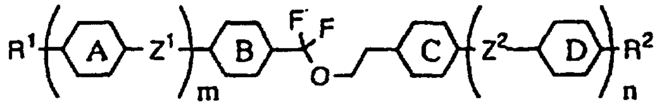

(1－a)

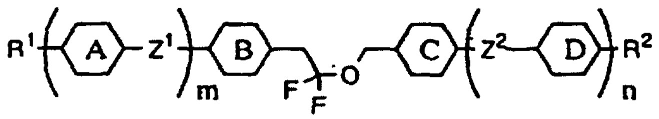

(1－b)

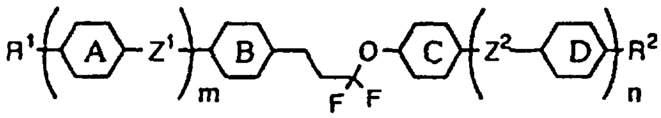

(1－c)

wherein $R^1$, $R^2$, ring A, ring B, ring C, ring D, $Z^1$, $Z^2$, m, and n have the same meaning as described above.

Further, the compounds which are particularly preferable in the aspect of skeleton structure are those expressed by one of the group of the general formulas (1-1) to (1-117)

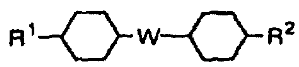

(1-1)

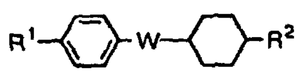

(1-2)

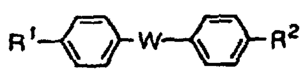

(1-3)

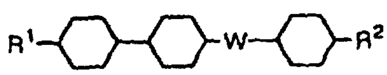

(1-4)

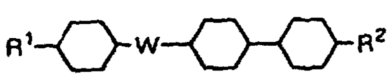

(1-5)

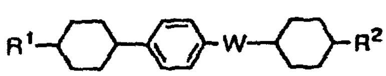

(1-6)

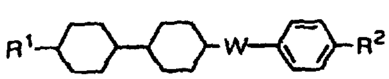

(1-7)

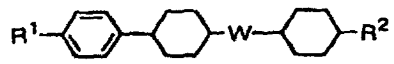

(1-8)

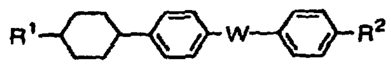

(1-9)

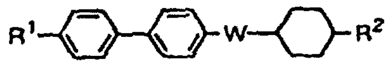

(1-10)

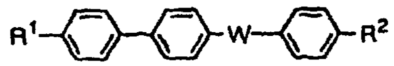

(1-11)

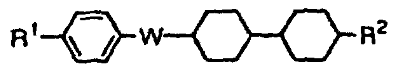

(1-12)

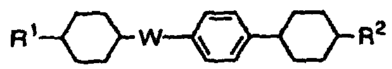

(1-13)

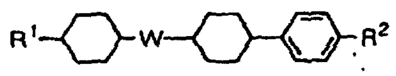

(1-14)

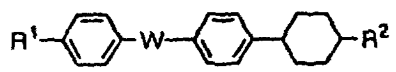

(1-15)

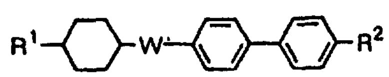

(1-16)

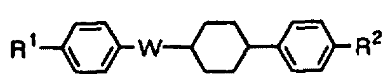

(1-17)

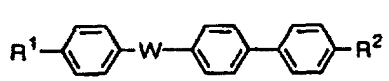

(1-18)

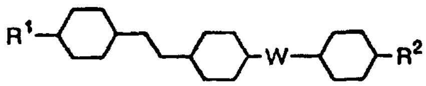

(1-19)

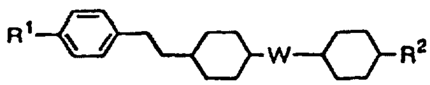

(1-20)

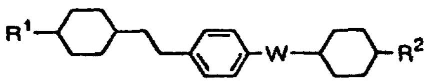

(1-21)

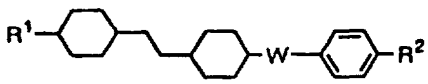

(1-22)

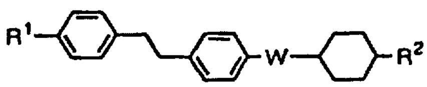

(1-23)

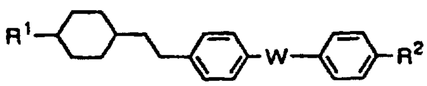

(1-24)

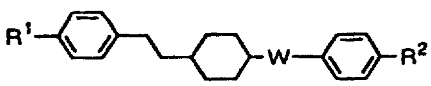

(1-25)

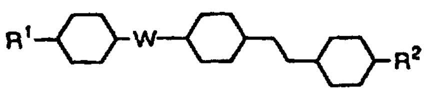

(1-26)

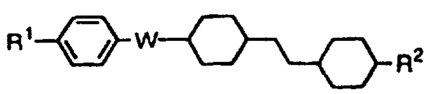

(1-27)

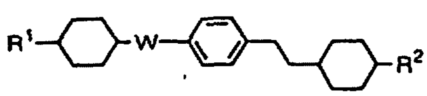

(1-28)

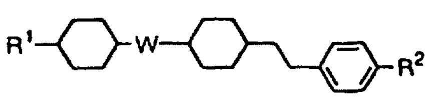

(1-29)

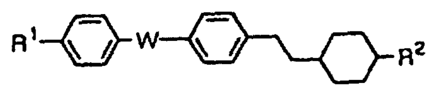

(1-30)

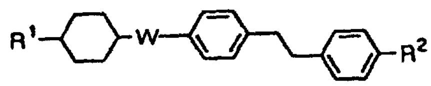

(1-31)

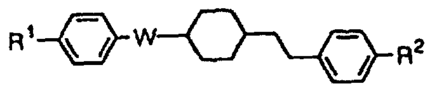

(1-32)

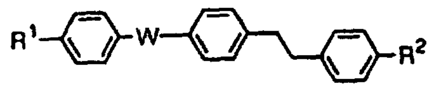

(1-33)

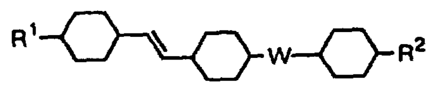

(1-34)

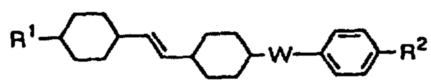

(1-35)

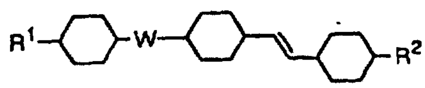

(1-36)

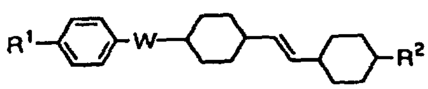

(1-37)

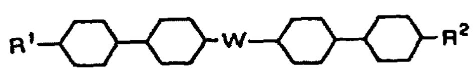

(1-38)

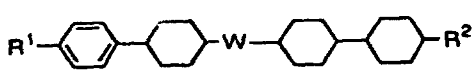

(1-39)

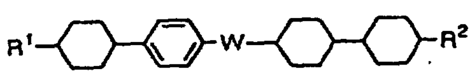

(1-40)

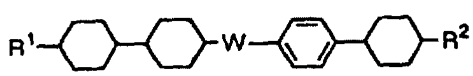

(1-41)

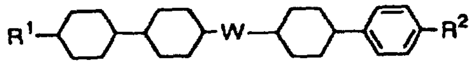

(1-42)

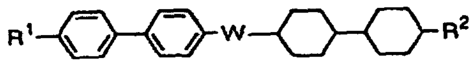

(1-43)

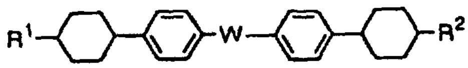

(1-44)

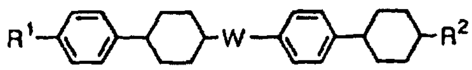

(1-45)

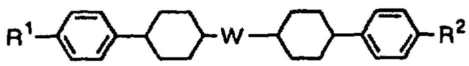

(1-46)

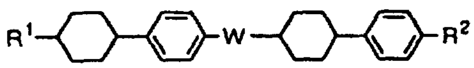

(1-47)

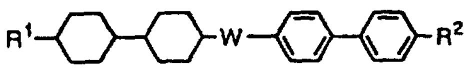

(1-48)

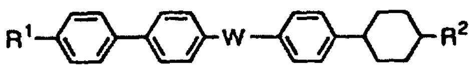

(1-49)

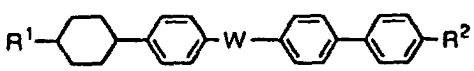

(1-50)

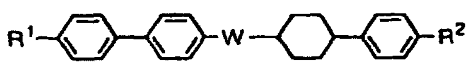

(1-51)

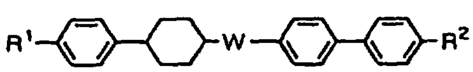

(1-52)

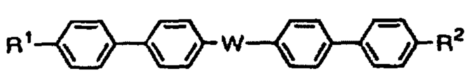

(1-53)

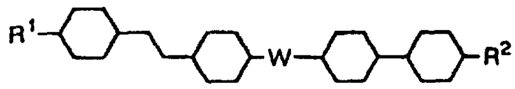
(1-54)

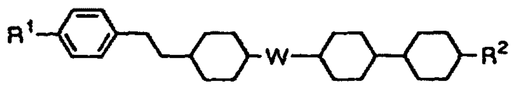
(1-55)

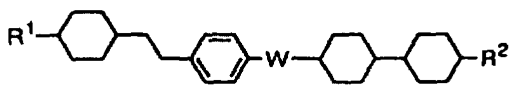
(1-56)

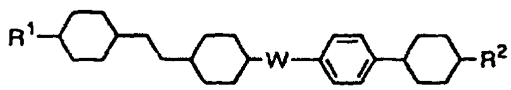
(1-57)

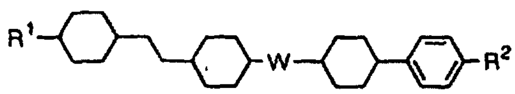
(1-58)

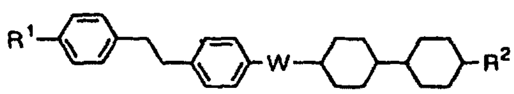
(1-59)

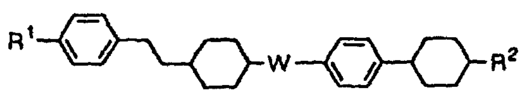
(1-60)

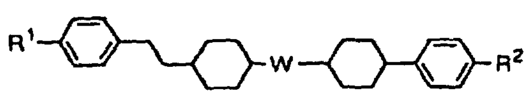
(1-61)

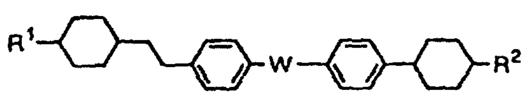
(1-62)

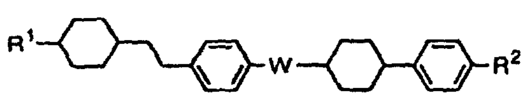
(1-63)

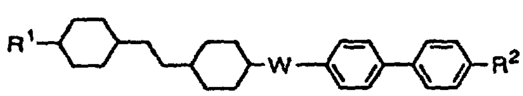
(1-64)

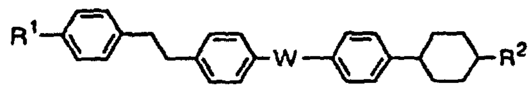

(1-65)

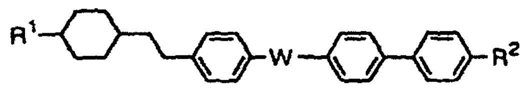

(1-66)

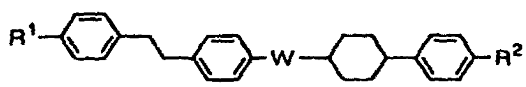

(1-67)

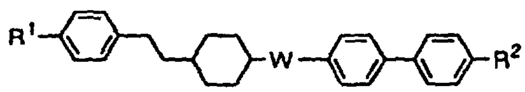

(1-68)

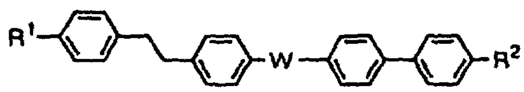

(1-69)

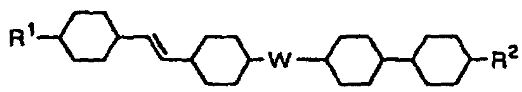

(1-70)

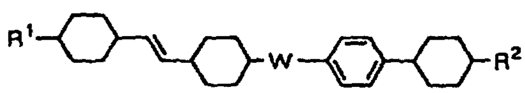

(1-71)

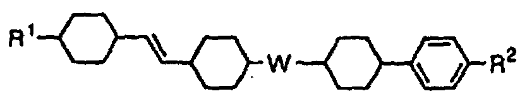

(1-72)

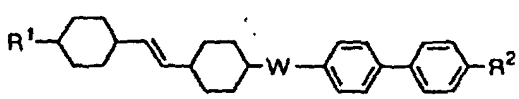

(1-73)

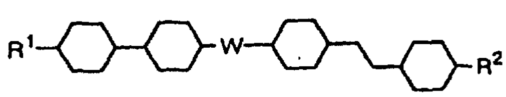

(1-74)

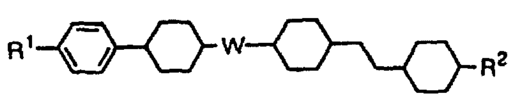

(1-75)

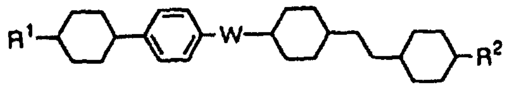
(1-76)

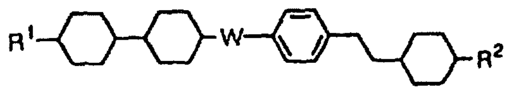
(1-77)

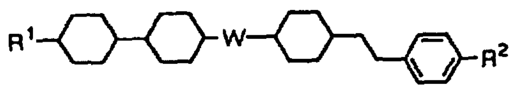
(1-78)

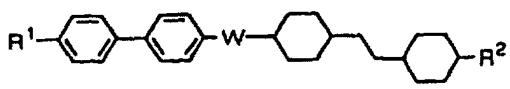
(1-79)

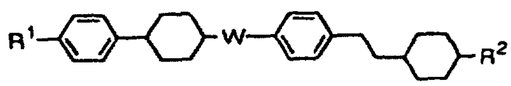
(1-80)

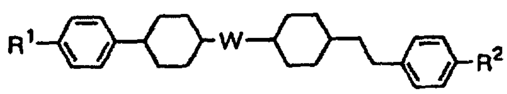
(1-81)

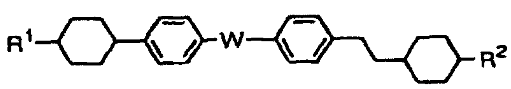
(1-82)

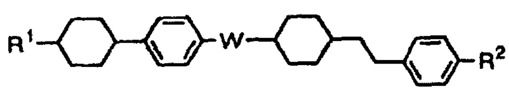
(1-83)

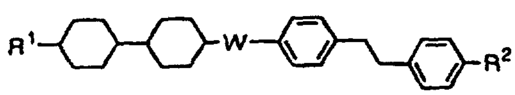
(1-84)

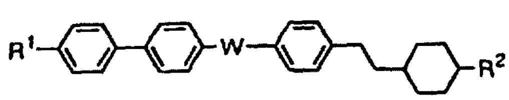
(1-85)

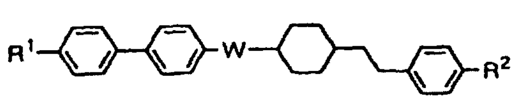
(1-86)

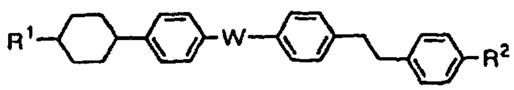
(1-87)

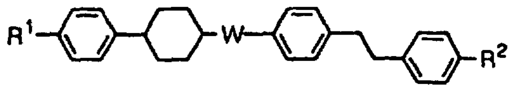
(1-88)

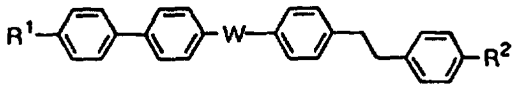
(1-89)

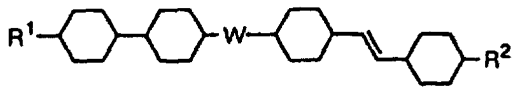
(1-90)

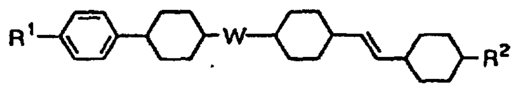
(1-91)

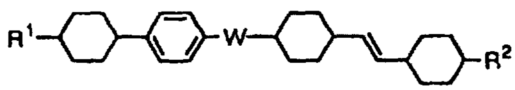
(1-92)

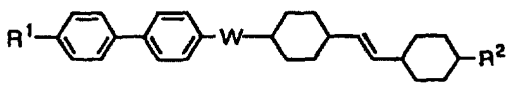
(1-93)

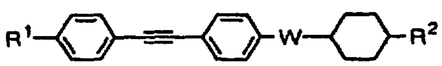
(1-94)

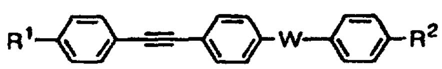
(1-95)

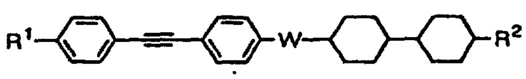
(1-96)

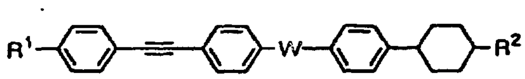
(1-97)

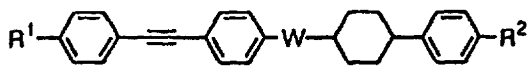
(1-98)

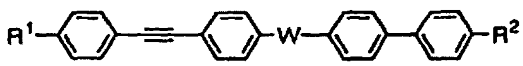
(1-99)

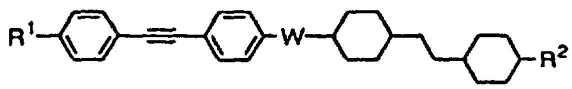
(1-100)

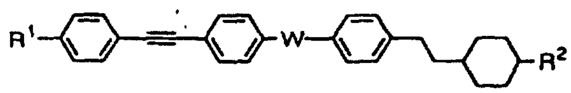
(1-101)

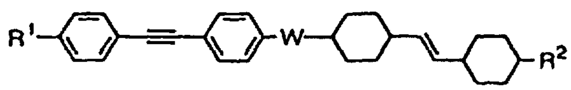
(1-102)

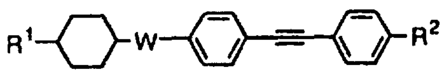
(1-103)

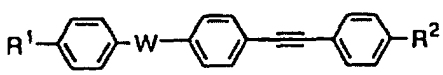
(1-104)

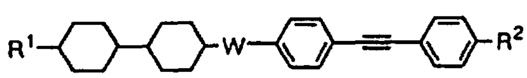
(1-105)

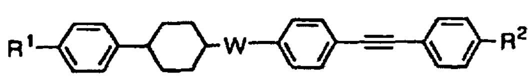
(1-106)

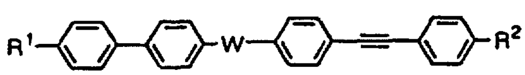
(1-107)

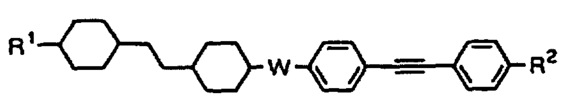
(1-108)

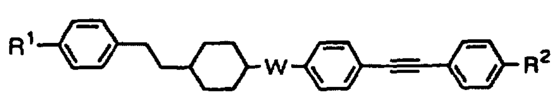
(1-109)

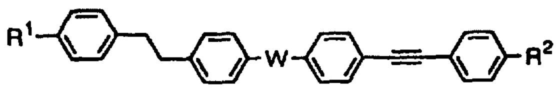

(1-110)

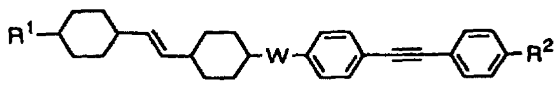

(1-111)

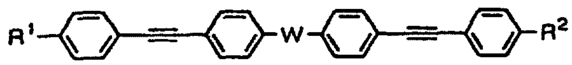

(1-112)

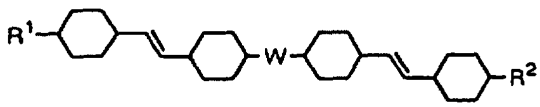

(1-113)

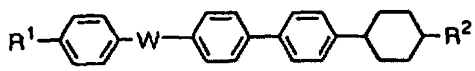

(1-114)

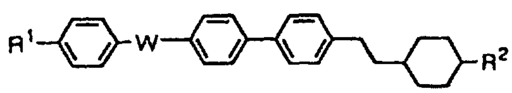

(1-115)

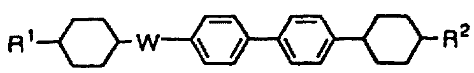

(1-116)

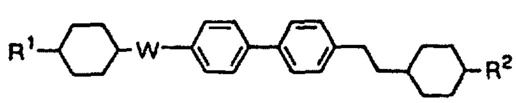

(1-117)

wherein $R^1$ and $R^2$ have the same meaning as described above, W represents one of the following bonding groups, and any hydrogen atom on benzene ring in the group of the general formulas may be replaced by fluorine atom.

W :

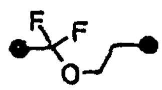

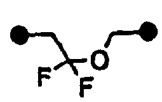

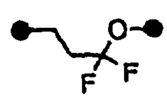

Any compounds expressed by one of the general formulas (1-1) to (1-117) above have a low viscosity and remarkably excellent characteristics in miscibility at low temperatures. When the $R^1$ or $R^2$ is a halogen atom, $CF_3$ group, $OCF_3$ group, $CHF_2$ group, or CN group, the compounds exhibit a high dielectric anisotropy compared with known compounds having the same substituent.

Since the two ring or three ring compounds expressed by one of the general formulas (1-1) to (1-37), (1-94), (1-95), (1-103), and (1-104) are extremely low in viscosity while having a comparatively high clearing point, it is possible

to reduce the viscosity of liquid crystal compositions while maintaining their clearing point when the compounds are added as a component of liquid crystal compositions. Further, since the four ring compounds expressed by one of the general formulas (1-38) to (1-93), (1-96) to (1-102), and (1-105) to (1-117) are comparatively low in viscosity while having a remarkably high clearing point, it is possible to considerably raise the clearing point of liquid crystal compositions while maintaining their viscosity when the compounds are added as a component of liquid crystal compositions.

As described above, liquid crystal compositions and liquid crystal display devices which are very stable against outside environments, capable of being driven at a low voltage, and capable of responding at a high speed can be provided by producing compositions comprising only the compounds of the present invention (two ring to four ring compounds) or by using the compounds of the present invention together with known liquid crystalline compounds.

Liquid crystal compositions of the present invention preferably comprise at least one liquid crystalline compound expressed by the general formula (1) in a ratio of 0.1 to 99 % by weight to develop excellent characteristics.

Further, the liquid crystal compositions of the present invention preferably comprise, as a second component, at least one compound (hereinafter referred to as second component A) selected from the group consisting of the compounds expressed by one of the general formulas (2), (3), and (4) described above and/or at least one compound (hereinafter referred to as second component B) selected from the group consisting of the compounds expressed by one of the general formulas (5), (6), (7), (8), and (9) in addition to the liquid crystalline compound expressed by the general formula (1). Furthermore, known compounds may be mixed in the liquid crystal compositions as a third component for the purpose of adjusting threshold voltage, temperature range of liquid crystal phase, optical anisotropy value, dielectric anisotropy value, and viscosity.

Among the second component A, the compounds of the formulas (2-1) to (2-15), (3-1) to (3-48), and (4-1) to (4-55) can be mentioned as preferable examples of the compounds included in the general formulas (2), (3), or (4).

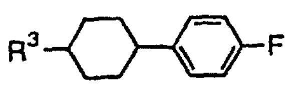
(2-1)

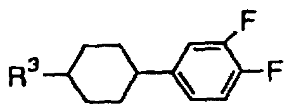
(2-2)

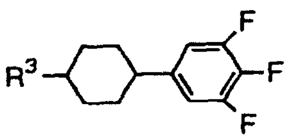
(2-3)

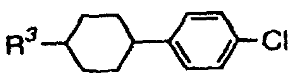
(2-4)

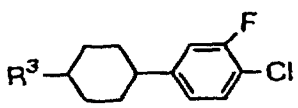
(2-5)

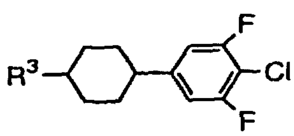
(2-6)

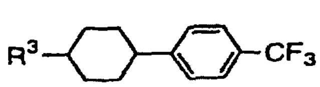
(2-7)

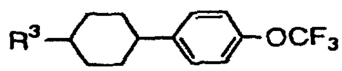

(2-8)

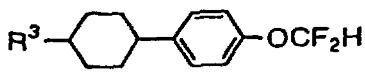

(2-9)

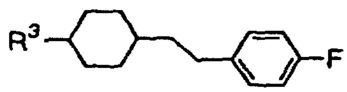

(2-10)

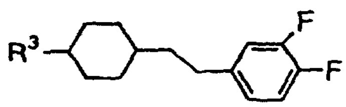

(2-11)

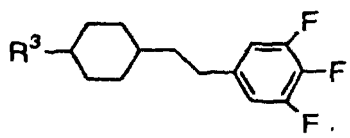

(2-12)

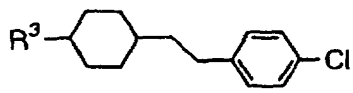

(2-13)

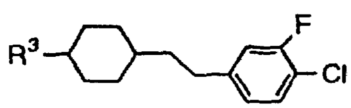

(2-14)

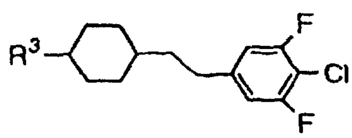

(2-15)

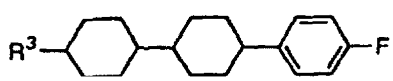

(3-1)

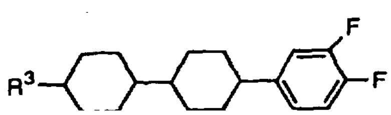

(3-2)

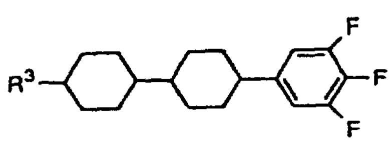

(3-3)

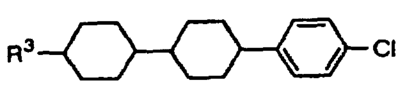

(3-4)

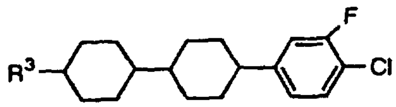
(3-5)

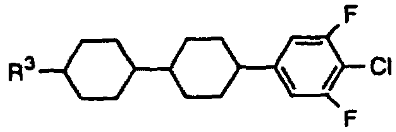
(3-6)

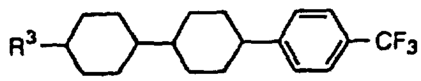
(3-7)

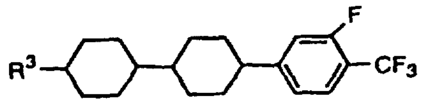
(3-8)

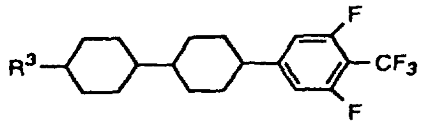
(3-9)

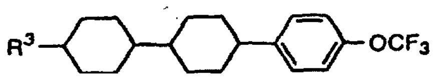
(3-10)

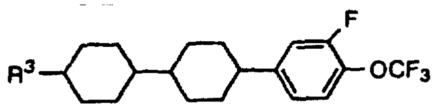
(3-11)

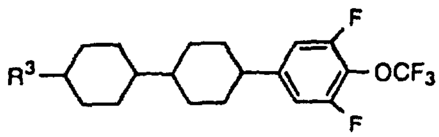
(3-12)

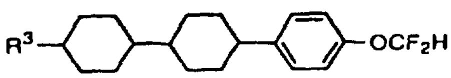
(3-13)

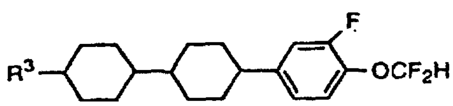
(3-14)

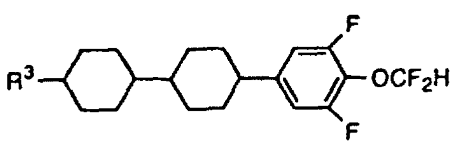
(3-15)

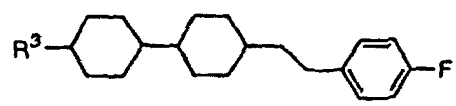
(3-16)

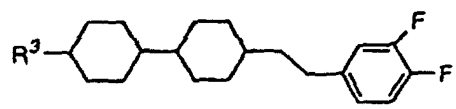
(3-17)

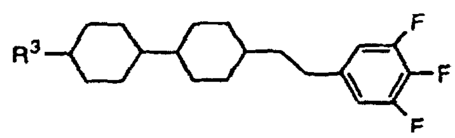
(3-18)

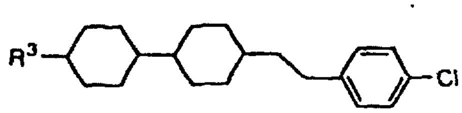
(3-19)

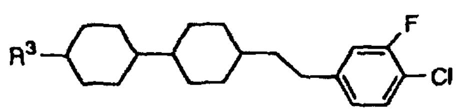
(3-20)

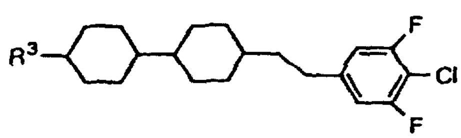
(3-21)

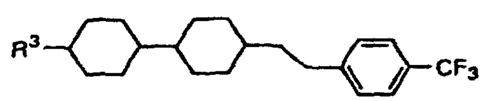
(3-22)

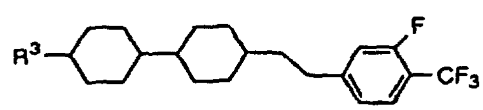
(3-23)

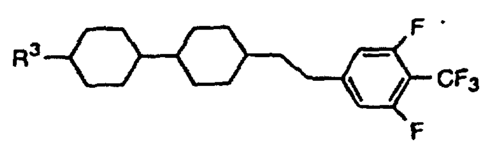
(3-24)

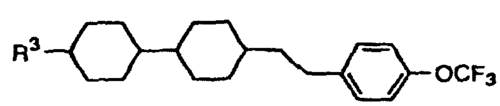
(3-25)

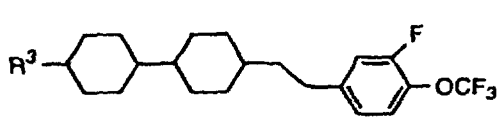
(3-26)

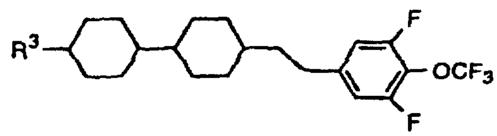
(3-27)

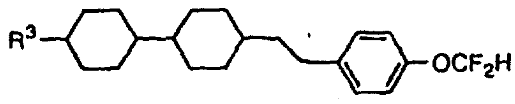
(3-28)

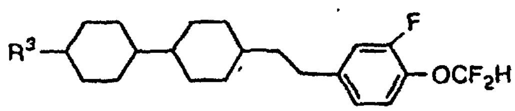
(3-29)

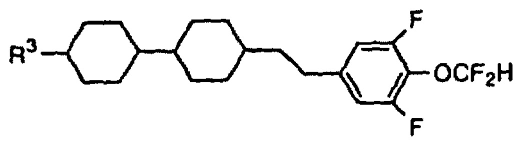
(3-30)

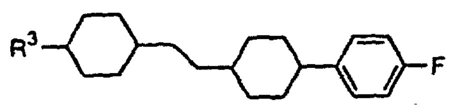
(3-31)

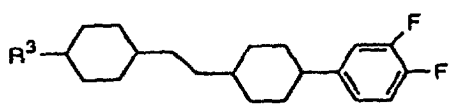
(3-32)

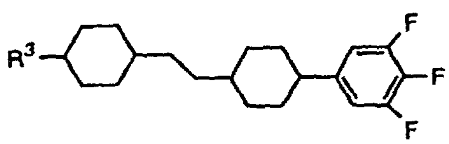
(3-33)

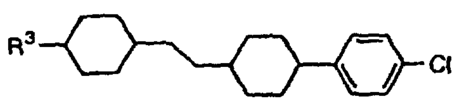
(3-34)

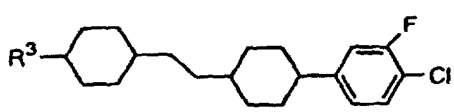
(3-35)

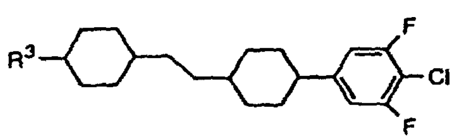
(3-36)

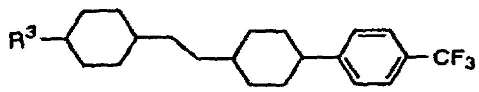

(3-37)

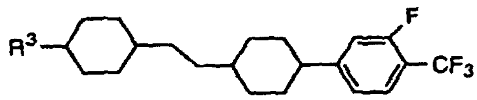

(3-38)

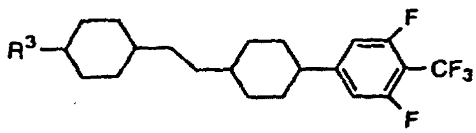

(3-39)

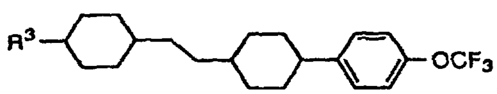

(3-40)

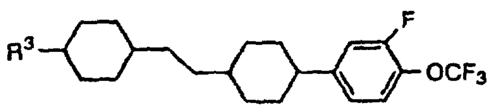

(3-41)

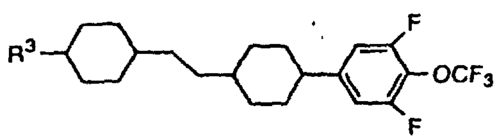

(3-42)

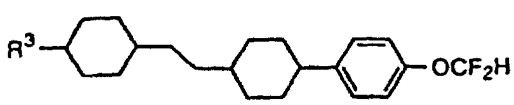

(3-43)

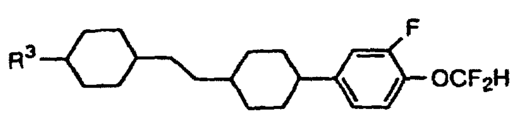

(3-44)

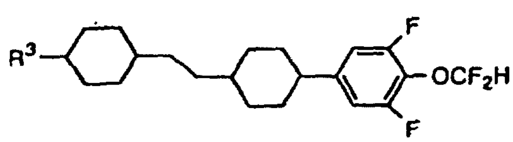

(3-45)

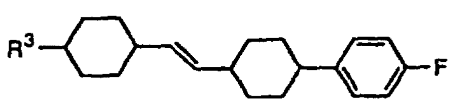

(3-46)

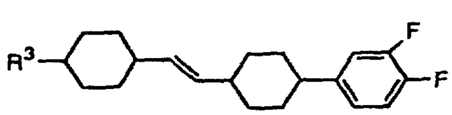

(3-47)

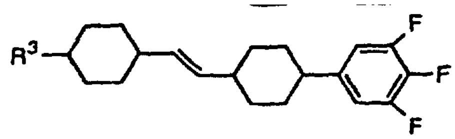

(3-48)

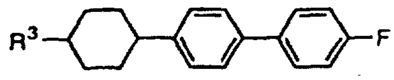

(4-1)

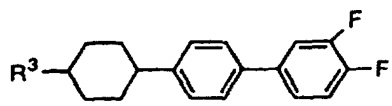

(4-2)

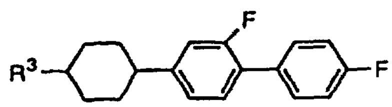

(4-3)

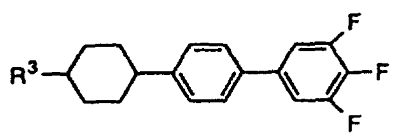

(4-4)

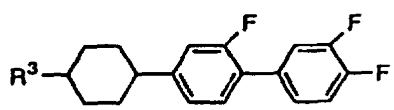

(4-5)

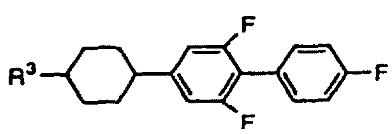

(4-6)

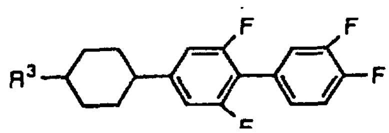

(4-7)

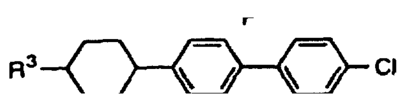

(4-8)

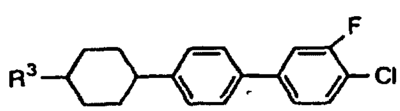

(4-9)

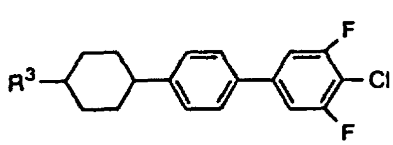

(4-10)

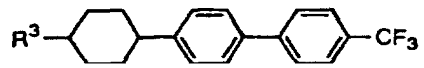

(4-11)

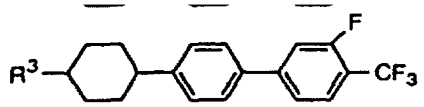

(4-12)

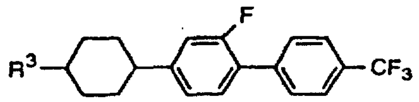

(4-13)

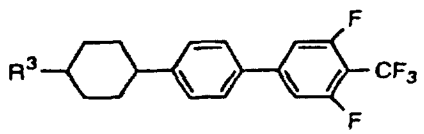

(4-14)

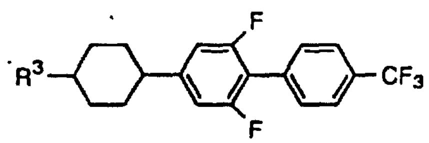

(4-15)

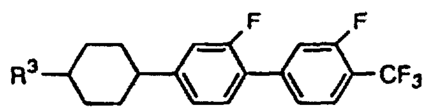

(4-16)

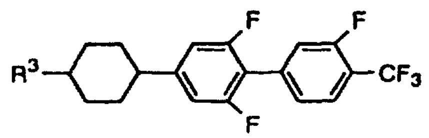

(4-17)

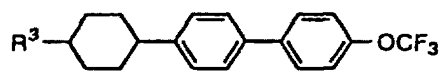

(4-18)

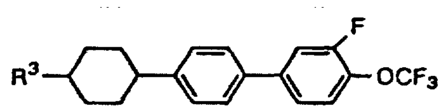

(4-19)

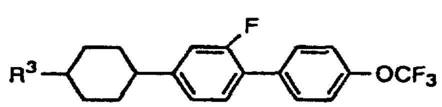

(4-20)

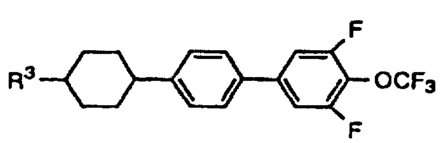

(4-21)

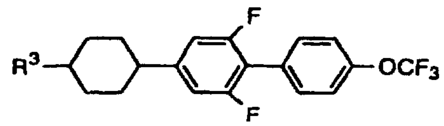

(4-22)

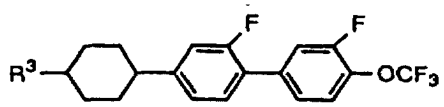

(4-23)

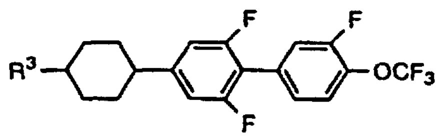

(4-24)

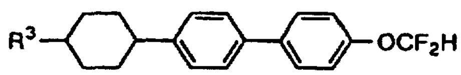

(4-25)

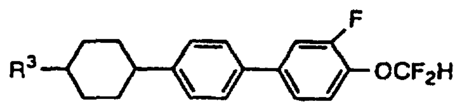

(4-26)

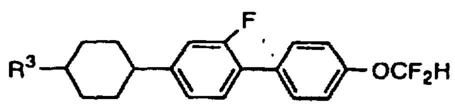

(4-27)

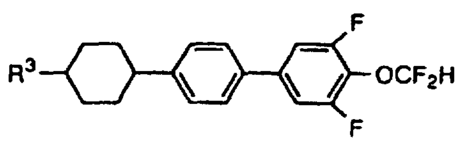

(4-28)

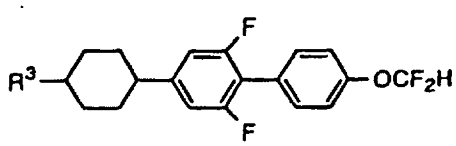

(4-29)

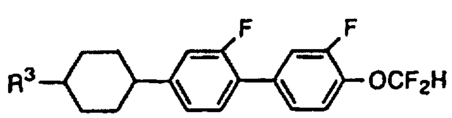

(4-30)

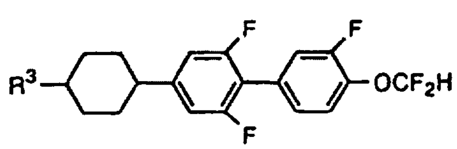

(4-31)

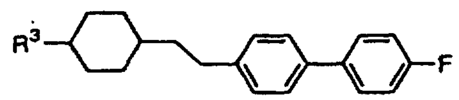
(4-32)

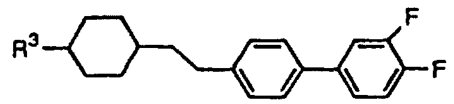
(4-33)

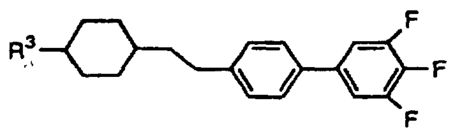
(4-34)

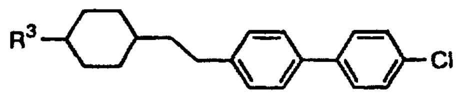
(4-35)

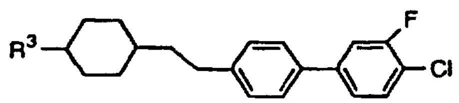
(4-36)

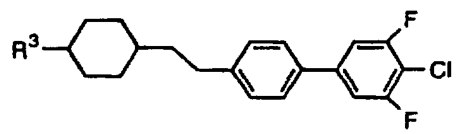
(4-37)

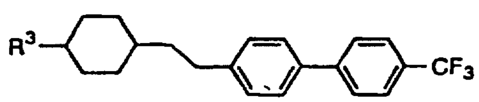
(4-38)

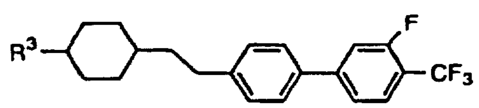
(4-39)

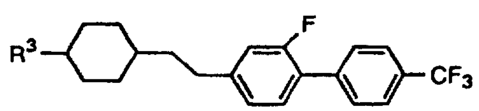
(4-40)

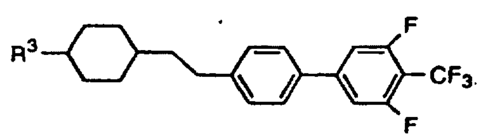
(4-41)

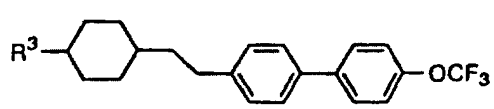
(4-42)

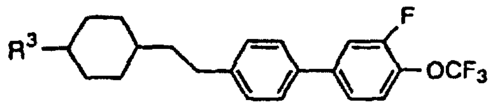

(4-43)

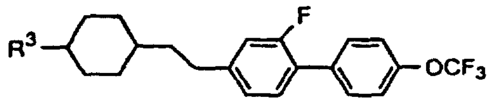

(4-44)

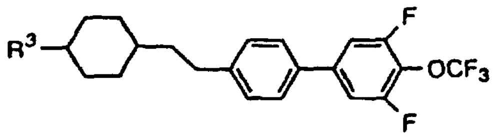

(4-45)

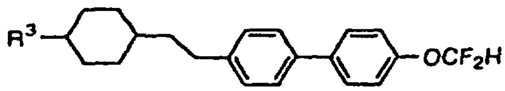

(4-46)

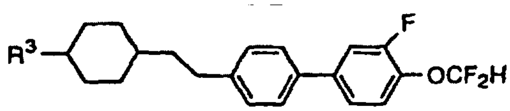

(4-47)

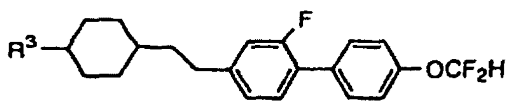

(4-48)

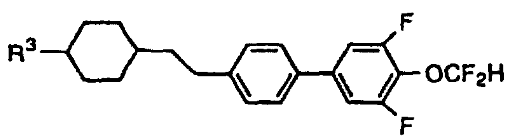

(4-49)

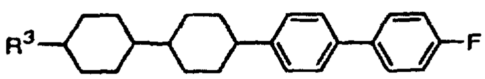

(4-50)

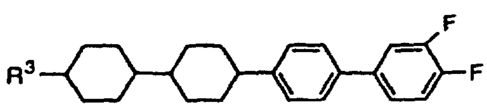

(4-51)

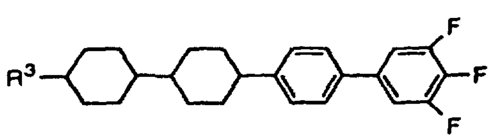

(4-52)

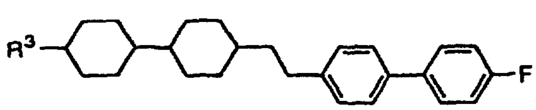

(4-53)

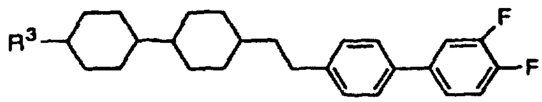

(4-54)

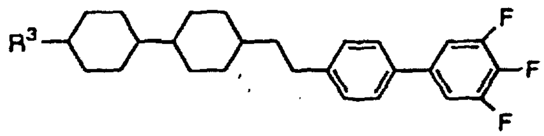

(4-55)

Compounds expressed by one of these general formulas (2-1) to (4-55) exhibit a positive dielectric anisotropy value and are remarkably excellent in thermal stability and chemical stability.

Amount of the compounds to be used is suitably in the range of 1 to 99 % by weight, preferably 10 to 97 % by weight, and more desirably 40 to 95 % by weight based on the total amount of liquid crystal composition.

Next, among the second component B, the compounds of one of the formulas (5-1) to (5-24), (6-1) to (6-3), and (7-1) to (7-28) can be mentioned as preferable examples of the compounds included in the general formula (5), (6), or (7).

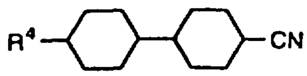

(5-1)

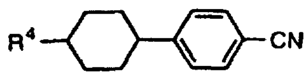

(5-2)

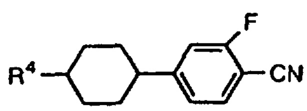

(5-3)

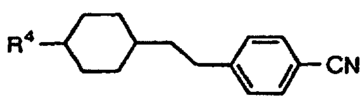

(5-4)

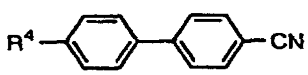

(5-5)

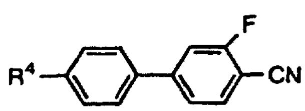

(5-6)

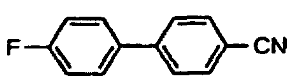

(5-7)

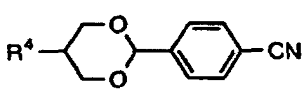

(5-8)

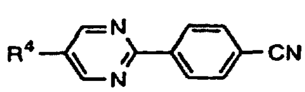

(5-9)

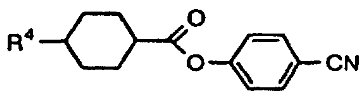
(5-10)

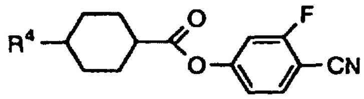
(5-11)

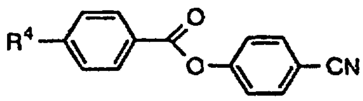
(5-12)

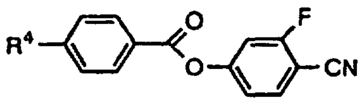
(5-13)

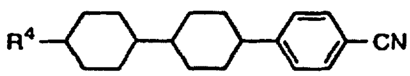
(5-14)

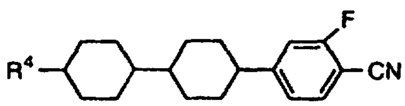
(5-15)

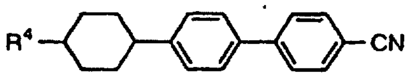
(5-16)

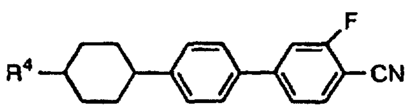
(5-17)

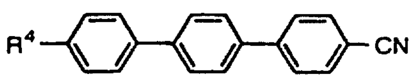
(5-18)

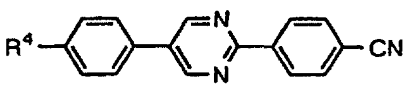
(5-19)

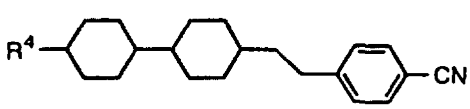
(5-20)

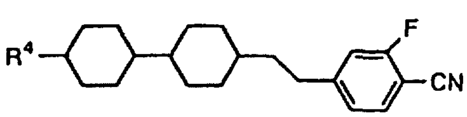
(5-21)

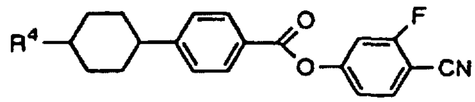

(5-22)

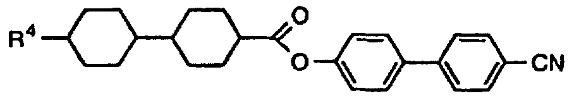

(5-23)

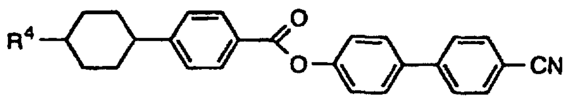

(5-24)

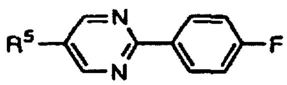

(6-1)

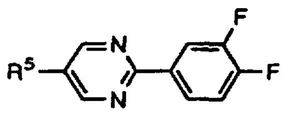

(6-2)

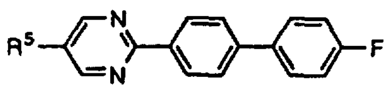

(6-3)

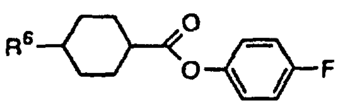

(7-1)

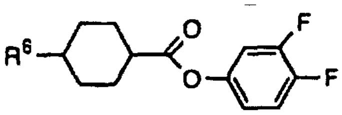

(7-2)

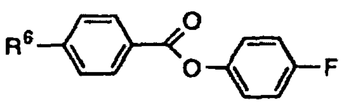

(7-3)

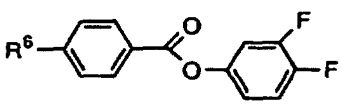

(7-4)

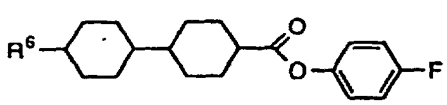

(7-5)

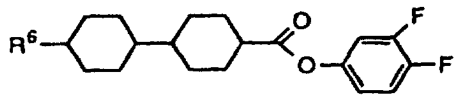

(7-6)

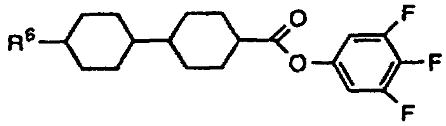

(7-7)

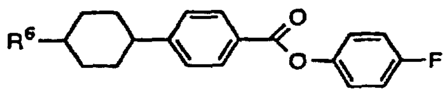

(7-8)

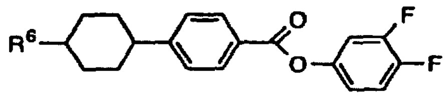

(7-9)

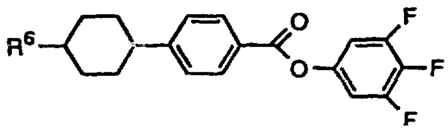

(7-10)

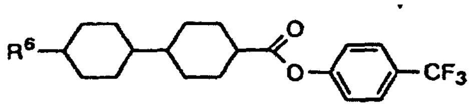

(7-11)

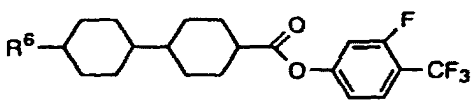

(7-12)

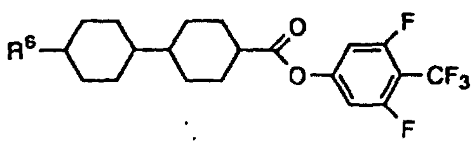

(7-13)

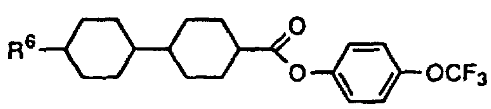

(7-14)

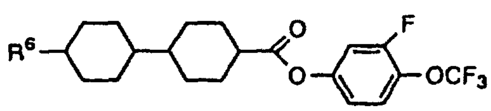

(7-15)

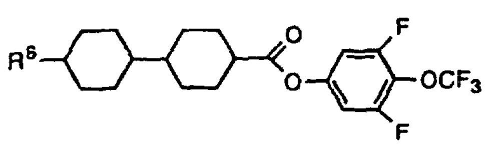

(7-16)

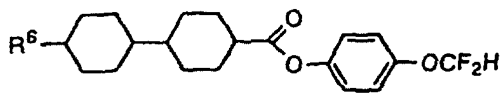

(7-17)

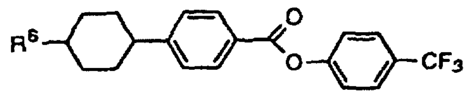

(7-18)

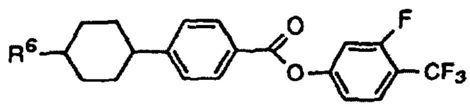

(7-19)

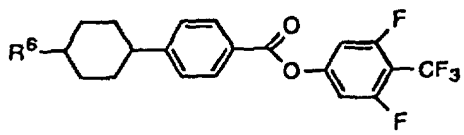

(7-20)

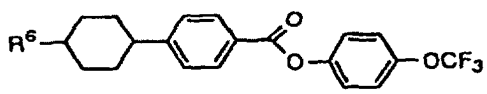

(7-21)

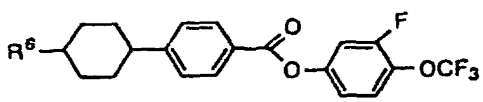

(7-22)

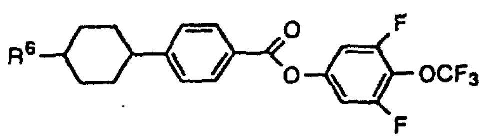

(7-23)

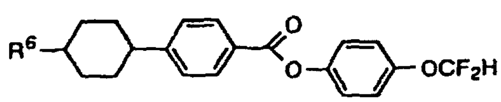

(7-24)

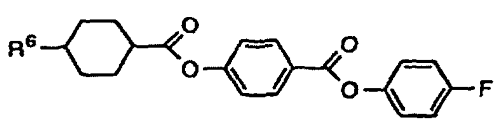

(7-25)

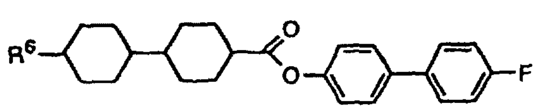

(7-26)

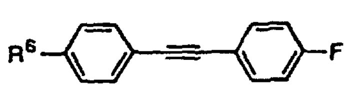

(7-27)

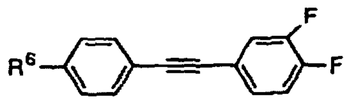

(7-28)

Compounds expressed by one of these general formulas (5-1) to (7-28) have a large positive dielectric anisotropy value and are used particularly for the purpose of lowering threshold voltage. They are used for the purpose of adjusting viscosity, adjusting optical anisotropy value, and widening temperature range of liquid crystal phase, and also for the purpose of improving the steepness.

Among the second component B, the compounds of one of the formulas (8-1) to (8-8) and (9-1) to (9-12) can be mentioned as preferable examples of the compounds included in the general formula (8) or (9), respectively.

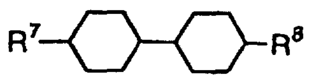

(8-1)

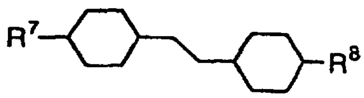

(8-2)

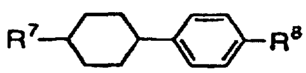

(8-3)

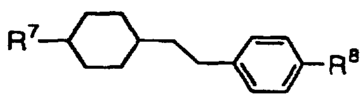

(8-4)

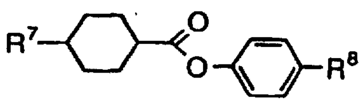

(8-5)

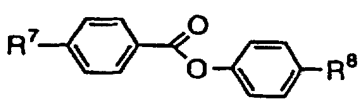

(8-6)

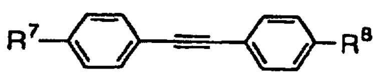

(8-7)

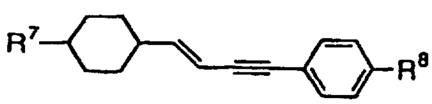

(8-8)

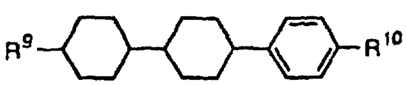

(9-1)

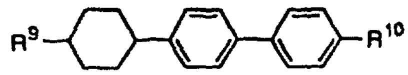

(9-2)

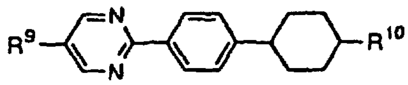

(9-3)

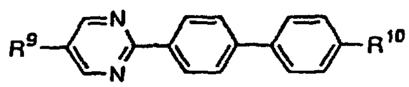

(9-4)

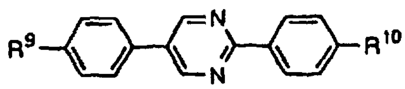

(9-5)

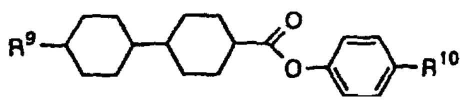

(9-6)

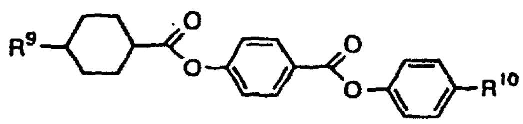

(9-7)

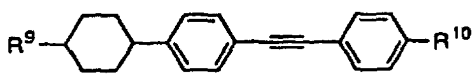

(9-8)

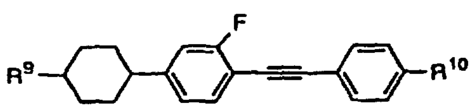

(9-9)

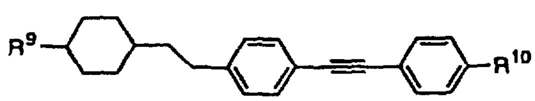

(9-10)

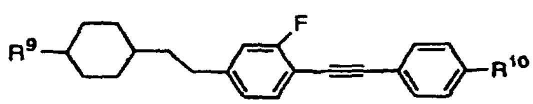

(9-11)

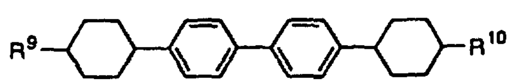

(9-11)

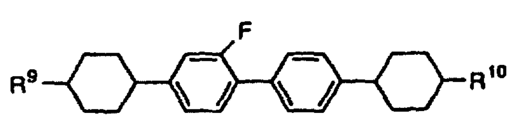

(9-12)

Amount of the compounds of the second component B described above to be used is suitably in the range of 1 to 99 % by weight, preferably 10 to 97 % by weight, and more desirably 40 to 95 % by weight based on the total

amount of liquid crystal composition.

Liquid crystal compositions used according to the present invention are produced by methods which are known or conventional by themselves. Generally, a method in which various components are dissolved with each other at a high temperature is adopted. Also, the liquid crystal materials of the present invention are improved and optimized depending on intended uses by a suitable additive. Such an additive is well known in the art and described in the literature in detail. For instance, a chiral dopant or the like can usually be added to induce a helical structure of liquid crystals thereby adjust a required twisting angle and avoid a reverse-twist.

Liquid crystal compositions used according to the present invention can be used as ones for guest-host (GH) mode by adding a dichroic dye such as merocyanine type, styryl type, azo type, azomethine type, azoxy type, quinophthalone type, anthraquinone type, or tetrazine type dye. Liquid crystal compositions of the present invention can also be used as liquid crystal compositions for NCAP which is prepared by the microencapsulation of a nematic liquid crystal, or for a polymer dispersed liquid crystal display device (PDLCD) represented by a polymer network liquid crystal display device (PNLCD) prepared by forming polymers of three-dimensional reticulated structure in a liquid crystal. In addition, the liquid crystal compositions of the present invention can be used as ones for electrically controlled birefringence (ECB) mode or dynamic scattering (DS) mode.

As nematic liquid crystal compositions comprising the compound of the present invention, the following composition examples (Composition Examples 1 through 21) can be demonstrated. In the composition examples, compounds are designated by the abbreviation according to the definitions shown in the following Table 1. That is, left hand side terminal groups are indicated by a, aO, aOb, Va, aVb, or aVbVd; bonding groups, 2, E, T, V, CF2O, CF2O2, or 1CF2O1; ring structure, B, B(F), B(2F), B(2F,3F), B(F,F), H, Py, D, or Ch; and right hand side terminal groups, F, CL, C, CF3, OCF3, OCF2H, w, Ow, EMe, wV, or wVz.

Table 1

| Left side terminal group | Symbol | Bonding group | Symbol |
|---|---|---|---|
| $C_aH_{2a+1}-$ | a- | $-CH_2CH_2-$ | 2 |
| $C_aH_{2a+1}O-$ | aO- | -COO- | E |
| $CaH_{2a+1}OC_bH_{2b}-$ | aOb- | $-C\equiv C-$ | T |
| $CH_2=CHC_aH_{2a}-$ | Va- | -CH=CH- | V |
| $C_aH_{2a+1}CH=CHC_bH_{2b}-$ | aVb- | $-CF_2O-$ | CF2O |
| $C_aH_{2a+1}CH=CHC_bH_{2b}CH=CHC_dH_{2d}-$ | aVbVd- | $-CF_2OC_2H_4-$ | CF2O2 |
| | | $-CH_2CF_2OCH_2-$ | 1CF2O1 |

| Ring structure | Symbol | Right side terminal group | Symbol |
|---|---|---|---|
| | | -F | -F |
| 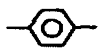 | B | -Cl | -CL |
| 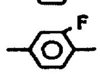 | B(F) | -CN | -C |
| 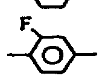 | B(2F) | $-CF_3$ | -CF3 |
| 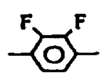 | B(2F,3F) | $-OCF_3$ | -OCF3 |
| 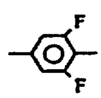 | B(F,F) | $-OCF_2H$ | -OCF2H |
| 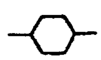 | H | $-C_wH_{2w+1}$ | -w |
| 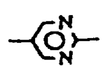 | Py | $-OC_wH_{2w+1}$ | -Ow |
| 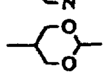 | D | $-COOCH_3$ | -EMe |
| 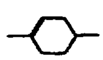 | Ch | $-C_wH_{2w}CH=CH_2$ | -wV |
| | | $-C_wH_{2w}CH=CHC_zH_{2z+1}$ | -wVz |

| Composition Example 1 | |
|---|---|
| 2-BCF2O2B(F,F)-F | 5.0 % |
| 3-BCF2O2B(F,F)-F | 5.0 % |

(continued)

| Composition Example 1 | |
|---|---|
| 3-B(F)CF2O2B(F,F)-F | 5.0 % |
| 5-B(F,F)CF2O2B(F,F)-F | 5.0 % |
| 1V-H1CF2O1B(F,F)-F | 10.0 % |
| 1V2-H1CF2O1B(F,F)-F | 10.0 % |
| 2-HB(F)CF2O2B(F,F)-F | 10.0 % |
| 3-HB(F,F)CF2O2B(F,F)-F | 10.0 % |
| 2-HBCF2O2B(F,F)-F | 6.0 % |
| 3-HBCF2O2B(F,F)-F | 7.0 % |
| 5-HBCF2O2B(F,F)-F | 7.0 % |
| V2-HBCF2O2B(F,F)-F | 10.0 % |
| 1V2-HBCF2O2B(F,F)-F | 10.0 % |

| Composition Example 2 | |
|---|---|
| 3-BCF2O2B(F)-F | 5.0 % |
| 5-B(F)CF2O2B(F)-F | 5.0 % |
| V-H1CF2O1B(F)-F | 10.0 % |
| 1V2-H1CF2O1B(F)-F | 10.0 % |
| 2-HB(F)CF202B(F)-F | 5.0 % |
| 3-HB(F)CF2O2B(F)-F | 8.0 % |
| 5-HB(F)CF2O2B(F)-F | 7.0 % |
| 3-HBCF2O2B(F)-F | 10.0 % |
| 5-HBCF2O2B(F)-F | 10.0 % |
| V2-HBCF2O2B(F)-F | 10.0 % |
| 1V2-HBCF2O2B(F)-F | 7.0 % |
| 1V-H1CF2O1H-4 | 5.0 % |
| 1V2-H1CF2O1H-5 | 5.0 % |
| V2-HB(F)CF2O2BH-3 | 3.0 % |

| Composition Example 3 | |
|---|---|
| 3-HBCF2O2B(F,F)-F | 5.0 % |
| 5-HBCF2O2B(F,F)-F | 10.0 % |
| 5-HBCF2O2B-CF3 | 5.0 % |
| 5-HBCF2O2B(F)-F | 5.0 % |
| 3-HBCF2O2B-OCF3 | 5.0 % |
| 7-HB(F,F)-F | 8.0 % |
| 3-HHB(F,F)-F | 6.0 % |
| 4-HHB(F,F)-F | 3.0 % |
| 3-H2HB(F,F)-F | 10.0 % |
| 4-H2HB(F,F)-F | 6.0 % |
| 5-H2HB(F,F)-F | 6.0 % |
| 3-HH2B(F,F)-F | 10.0 % |
| 5-HH2B (F, F)-F | 5.0 % |
| 3-HBB(F,F)-F | 5.0 % |
| 5-HBB(F,F)-F | 5.0 % |
| 3-HHBB(F,F)-F | 3.0 % |
| 3-HH2BB(F,F)-F | 3.0 % |

| Composition Example 4 | |
|---|---|
| 3-HBCF2O2B(F,F)-F | 5.0 % |
| 3-HBCF2O2B-OCF3 | 5.0 % |
| 3-HB-CL | 4.0 % |
| 5-HB-CL | 4.0 % |
| 7-HB-CL | 5.0 % |
| 2-HHB-CL | 6.0 % |
| 3-HHB-CL | 7.0 % |
| 5-HHB-CL | 6.0 % |
| 2-HBB(F)-F | 6.0 % |
| 3-HBB(F)-F | 6.0 % |
| 5-HBB(F)-F | 12.0 % |
| 3-HBB(F,F)-F | 13.0 % |
| 5-HBB(F,F)-F | 13.0 % |
| 3-H2HB(F)-CL | 3.0 % |
| 3-HB(F)TB-2 | 3.0 % |
| 3-HB(F)VB-2 | 2.0 % |

| Composition Example 5 | |
|---|---|
| 5-HBCF2O2B(F,F)-F | 10.0 % |
| 5-HBCF2O2B-CF3 | 5.0 % |
| 3-HBCF2O2B-OCF3 | 5.0 % |
| 2-HBB(F)-F | 3.0 % |
| 3-HBB(F)-F | 3.0 % |
| 5-HBB(F)-F | 6.0 % |
| 2-HBB-F | 3.0 % |
| 3-HBB-F | 3.0 % |
| 3-H2HB(F,F)-F | 6.0 % |
| 4-H2HB(F,F)-F | 6.0 % |
| 5-H2HB(F,F)-F | 6.0 % |
| 3-HHB(F,F)-F | 3.0 % |
| 4-HHB(F,F)-F | 3.0 % |
| 3-HH2B(F,F)-F | 12.0 % |
| 5-HH2B(F,F)-F | 6.0 % |
| 3-HBB(F,F)-F | 6.0 % |
| 5-HBB(F,F)-F | 6.0 % |
| 3-HHB-CL | 4.0 % |
| 5-HHB-CL | 4.0 % |

| Composition Example 6 | |
|---|---|
| 3-HBCF2O2B(F,F)-F | 8.0 % |
| 5-HBCF2O2B(F,F)-F | 3.0 % |
| 3-HBCF2O2B-OCF3 | 5.0 % |
| 2-HBB(F)-F | 8.0 % |
| 3-HBB(F)-F | 8.0 % |
| 5-HBB(F)-F | 16.0 % |
| 5-HB-F | 6.0 % |
| 7-HB-F | 6.0 % |

(continued)

| Composition Example 6 | |
|---|---|
| 5-HHB-OCF3 | 8.0 % |
| 3-H2HB-OCF3 | 8.0 % |
| 5-H2HB-OCF3 | 8.0 % |
| 3-HH2B-OCF3 | 8.0 % |
| 5-HH2B-OCF3 | 8.0 % |

| Composition Example 7 | |
|---|---|
| 3-HBCF2O2B-OCF3 | 5.0 % |
| 5-H2B(F)-F | 4.0 % |
| 7-HB(F)-F | 10.0 % |
| 2-HHB(F)-F | 12.0 % |
| 3-HHB(F)-F | 12.0 % |
| 5-HHB(F)-F | 12.0 % |
| 2-H2HB(F)-F | 12.0 % |
| 3-H2HB(F)-F | 6.0 % |
| 5-H2HB(F)-F | 12.0 % |
| 2-HBB(F)-F | 2.5 % |
| 3-HBB(F)-F | 2.5 % |
| 5-HBB(F)-F | 5.0 % |
| 3-HHB-F | 5.0 % |

| Composition Example 8 | |
|---|---|
| 3-HBCF2O2B(F,F)-F | 7.0 % |
| 1V2-HBCF2O2B(F)-F | 7.0 % |
| 7-HB(F)-F | 10.0 % |
| 3-HB-O2 | 10.0 % |
| 2-HHB-1 | 6.0 % |
| 3-HHB-1 | 8.0 % |
| 3-HHB-F | 3.0 % |
| 2-HHB(F)-F | 7.0 % |
| 3-HHB(F)-F | 7.0 % |
| 5-HHB(F)-F | 7.0 % |
| 2-HBB(F)-F | 7.0 % |
| 3-HBB(F)-F | 7.0 % |
| 5-HBB(F)-F | 14.0 % |

| Composition Example 9 | |
|---|---|
| 3-HBCF2O2B-OCF3 | 6.0 % |
| 3-HBCF2O2B(F,F)-F | 6.0 % |
| 3-HB(2F,3F)CF2O2BH-5 | 2.0 % |
| 5-HEB-F | 3.0 % |
| 7-HB(F)-F | 4.0 % |
| 2-HHB(F)-F | 13.0 % |
| 3-HHB(F)-F | 13.0 % |
| 5-HHB(F)-F | 13.0 % |

(continued)

| Composition Example 9 | |
|---|---|
| 2-H2HB(F)-F | 4.0 % |
| 3-H2HB(F)-F | 2.0 % |
| 5-H2HB(F)-F | 4.0 % |
| 3-HH2B(F)-F | 3.0 % |
| 5-HH2B(F)-F | 3.0 % |
| 3-HHB-1 | 5.0 % |
| 3-HHB-3 | 8.0 % |
| 3-HHEBB-F | 3.0 % |
| 5-HHEBB-F | 2.0 % |
| 3-HHEB-F | 3.0 % |
| 5-HHEB-F | 3.0 % |

| Composition Example 10 | |
|---|---|
| 3-HBCF2O2B-OCF3 | 5.0 % |
| 3-HBCF2O2B(F,F)-F | 7.0 % |
| 5-B(F,F)CF2O2B(F,F)-OCF2H | 7.0 % |
| V-HBCF2O2B-OCF3 | 7.0 % |
| 7-HB(F,F)-F | 3.0 % |
| 3-HHB(F,F)-F | 5.0 % |
| 3-H2HB(F,F)-F | 5.0 % |
| 3-HBB(F,F)-F | 10.0 % |
| 5-HBB(F,F)-F | 10.0 % |
| 3-H2BB(F,F)-F | 5.0 % |
| 4-H2BB(F,F)-F | 5.0 % |
| 5-H2BB(F,F)-F | 5.0 % |
| 3-HBEB(F,F)-F | 2.0 % |
| 4-HBEB(F,F)-F | 2.0 % |
| 5-HBEB(F,F)-F | 2.0 % |
| 3-HHEB(F,F)-F | 10.0 % |
| 4-HHEB(F,F)-F | 3.0 % |
| 5-HHEB(F,F)-F | 3.0 % |
| 2-HHHB(F,F)-F | 2.0 % |
| 3-HH2HB(F,F)-F | 2.0 % |

| Composition Example 11 | |
|---|---|
| 3-B(F)CF2O2B-1 | 8.0 % |
| 3-B(F,F)CF2O2B-5 | 8.0 % |
| 3-B(2F,3F)CF2O2B-5 | 6.0 % |
| V-H1CF2O1H-4 | 2.0 % |
| V2-H1CF2O1H-5 | 2.0 % |
| 3-HB(F)CF2O2B-2 | 6.0 % |
| 3-HB(F)CF2O2B-3 | 6.0 % |
| 3-HB(F)CF2O2B-4 | 6.0 % |
| 3-HB(2F,3F)CF2O2B-5 | 5.0 % |
| V2-HB-C | 12.0 % |
| 1V2-HB-C | 11.0 % |

(continued)

| Composition Example 11 | |
|---|---|
| 1V2-BEB(F,F)-C | 11.0 % |
| 3-HH-EMe | 2.0 % |
| 2-H2BTB-3 | 3.0 % |
| 2-H2BTB-4 | 3.0 % |
| 3-H2BTB-2 | 3.0 % |
| 3-H2BTB-3 | 3.0 % |
| 3-H2BTB-4 | 3.0 % |

| Composition Example 12 | |
|---|---|
| 2-B(F)CF2O2B-4 | 3.0 % |
| 3-B(F,F)CF2O2B-5 | 3.0 % |
| 3-B(2F,3F)CF2O2B-5 | 3.0 % |
| 3-HB(2F,3F)CF2O2B(2F)-3 | 5.0 % |
| 3-HB(2F,3F)CF2O2B(2F,3F)-4 | 5.0 % |
| 5-HB(F)CF2O2BH-3 | 2.0 % |
| 3O1-BEB(F)-C | 11.0 % |
| V2-HB-C | 11.0 % |
| 3-HB-O2 | 3.0 % |
| 2-BTB-O1 | 5.0 % |
| 3-BTB-O1 | 5.0 % |
| 4-BTB-O1 | 5.0 % |
| 4-BTB-O2 | 5.0 % |
| 5-BTB-O1 | 5.0 % |
| 3-HHB-O1 | 3.0 % |
| 3-H2BTB-2 | 2.0 % |
| 3-H2BTB-3 | 3.0 % |
| 3-H2BTB-4 | 3.0 % |
| 3-HB(F)TB-2 | 2.0 % |
| 3-HB(F)TB-3 | 2.0 % |
| 3-HB(F)TB-4 | 2.0 % |
| 2-PyBH-3 | 4.0 % |
| 3-PyBH-3 | 4.0 % |
| 3-PyBB-2 | 4.0 % |

| Composition Example 13 | |
|---|---|
| 3-HBCF2O2B-OCF3 | 8.0 % |
| 3-HBCF2O2B(F,F)-F | 8.0 % |
| 2-BB-C | 7.0 % |
| 1O1-HB-C | 8.0 % |
| 2O1-HB-C | 7.0 % |
| 2-BEB-C | 4.0 % |
| 5-PyB-F | 5.0 % |
| 3-PyBB-F | 5.0 % |
| 2-PyB-2 | 3.0 % |
| 3-PyB-2 | 3.0 % |
| 4-PyB-2 | 3.0 % |

(continued)

| Composition Example 13 | |
|---|---|
| 2-PyBH-3 | 5.0 % |
| 3-PyBH-3 | 5.0 % |
| 4-PyBH-3 | 5.0 % |
| 3-PyB-02 | 5.0 % |
| 2-HHB-1 | 4.0 % |
| 3-HHB-1 | 6.0 % |
| 3-HHB-3 | 9.0 % |

| Composition Example 14 | |
|---|---|
| 3-HBCF2O2B-OCF3 | 5.0 % |
| 5-HBCF2O2B-OCF3 | 5.0 % |
| 5-HBCF2O2B-CF3 | 5.0 % |
| 1V-H1CF2O1B-2 | 6.0 % |
| 3-PyB(F)-F | 6.0 % |
| 3-PyBB-F | 4.0 % |
| 4-PyBB-F | 4.0 % |
| 5-PyBB-F | 4.0 % |
| 2-PyB-2 | 6.0 % |
| 3-PyB-2 | 6.0 % |
| 4-PyB-2 | 6.0 % |
| 3-HEB-O4 | 4.0 % |
| 4-HEB-O2 | 4.0 % |
| 5-HEB-O1 | 4.0 % |
| 2-H2BTB-4 | 4.0 % |
| 3-H2BTB-4 | 4.0 % |
| 3-HHB-1 | 7.0 % |
| 3-HHB-3 | 7.0 % |
| 3-HHEBB-C | 3.0 % |
| 5-HHEBB-C | 3.0 % |
| 3-HBEBB-C | 3.0 % |

| Composition Example 15 | |
|---|---|
| 3-HBCF2O2B-CF3 | 9.0 % |
| 5-HBCF2O2B(F,F)-OCF3 | 9.0 % |
| 2-BEB-C | 5.0 % |
| 2-BB-C | 8.0 % |
| 30-BB-C | 3.0 % |
| 3-HB-C | 5.0 % |
| 10-BEB-2 | 10.0 % |
| 3-HEB-O4 | 7.0 % |
| 4-HEB-O2 | 7.0 % |
| 5-HEB-O1 | 7.0 % |
| 4-HEB-4 | 5.0 % |
| 5-HEB-1 | 4.0 % |
| 3-HBEB-F | 5.0 % |
| 3-HHEB-F | 5.0 % |

(continued)

| Composition Example 15 | |
|---|---|
| 5-HHEB-F | 5.0 % |
| 3-HEBEB-F | 3.0 % |
| 3-HEBEB-1 | 3.0 % |

| Composition Example 16 | |
|---|---|
| 5-HBCF2OB-CF3 | 5.0 % |
| 5-HBCF2OB(F,F)-CF3 | 10.0 % |
| 3-DB-C | 4.0 % |
| 4-DB-C | 4.0 % |
| 101-HB-C | 5.0 % |
| 2-PyB-2 | 9.0 % |
| 3-PyB-2 | 9.0 % |
| 4-PyB-2 | 9.0 % |
| 2-PyBH-3 | 7.0 % |
| 3-PyBH-3 | 6.0 % |
| 4-PyBH-3 | 6.0 % |
| 1O1-HH-5 | 8.0 % |
| 3-HB(F)TB-2 | 6.0 % |
| 3-HB(F)TB-3 | 6.0 % |
| 3-HB(F)TB-4 | 6.0 % |

| Composition Example 17 | |
|---|---|
| 3-HBCF2O2B(F,F)-OCF2H | 14.0 % |
| 5-HBCF2O2B(F,F)-F | 14.0 % |
| 5-HBCF2O2B(F)-OCF3 | 4.0 % |
| 1V2-BEB(F,F)-C | 12.0 % |
| 3O1-BEB(F)-C | 6.0 % |
| 5-BEB(F)-C | 5.0 % |
| 3-HB-C | 9.0 % |
| 3-HB(F)-C | 7.0 % |
| 3-HHB(F)-C | 6.0 % |
| 3-PyBB-F | 8.0 % |
| 4-PyBB-F | 8.0 % |
| 5-PyBB-F | 7.0 % |

| Composition Example 18 | |
|---|---|
| 3-HBCF2O2B-OCF3 | 10.0 % |
| 5-HBCF2O2B-OCF3 | 5.0 % |
| 2-HB(F)-C | 10.0 % |
| 3-HB(F)-C | 10.0 % |
| 3O1-BEB(F)-C | 7.0 % |
| 3-HHEB-F | 4.0 % |
| 5-HHEB-F | 4.0 % |
| 3-HHEB(F,F)-F | 15.0 % |
| 5-HHEB(F,F)-F | 10.0 % |

(continued)

| Composition Example 18 | |
|---|---|
| 3-HBEB(F,F)-F | 5.0 % |
| 5-HBEB(F,F)-F | 5.0 % |
| 3-HHB-1 | 8.0 % |
| 3-HHB-O1 | 4.0 % |
| 3-HHB-3 | 3.0 % |

| Composition Example 19 | |
|---|---|
| 3-HB(F,F)CF2O2B-CF3 | 5.0 % |
| 3-HB(F,F)CF2O2B(F,F)-F | 5.0 % |
| 5-HB(F)CF2O2B-OCF3 | 5.0 % |
| 1V-HB-C | 9.0 % |
| 1V2-HB-C | 9.0 % |
| 3-HB-C | 14.0 % |
| 2-HHB-C | 3.0 % |
| 3-HHB-C | 4.0 % |
| V2-HH-3 | 6.0 % |
| V-HH-5 | 4.0 % |
| 1O1-HH-5 | 8.0 % |
| 2-BTB-O1 | 11.0 % |
| V-HHB-1 | 8.0 % |
| V-HBB-2 | 5.0 % |
| 1V2-HBB-2 | 4.0 % |

| Composition Example 20 | |
|---|---|
| 2-HB(F)CF2O2B-1 | 6.0 % |
| 3-HB(2F,3F)CF2O2B-1 | 8.0 % |
| 3-HBCF2O2B-OCF3 | 8.0 % |
| 2-HB-C | 10.0 % |
| 3-HB-C | 10.0 % |
| 1O1-HB-C | 8.0 % |
| 2-BTB-1 | 3.0 % |
| 1-BTB-6 | 6.0 % |
| 4-BTB-4 | 3.0 % |
| 2-HHB-C | 5.0 % |
| 3-HHB-C | 4.0 % |
| 4-HHB-C | 4.0 % |
| 5-HHB-C | 5.0 % |
| 2-HHB(F)-C | 6.0 % |
| 3-HHB(F)-C | 6.0 % |
| 5-HBEB(F)-C | 4.0 % |
| 3-HB(F)EB(F)-C | 4.0 % |

| Composition Example 21 | |
|---|---|
| 3-B(F,F)CF2O2B-5 | 5.0 % |
| 3-HBCF2O2B-OCF3 | 8.0 % |

(continued)

| Composition Example 21 | |
|---|---|
| 1V2-BEB(F,F)-C | 11.0 % |
| 2O1-BEB(F)-C | 5.0 % |
| 3O1-BEB(F)-C | 9.0 % |
| 3-HB(F)-C | 15.0 % |
| 3-HH-4 | 5.0 % |
| 3-HH-5 | 4.0 % |
| 1O1-HH-3 | 3.0 % |
| 4-BTB-O2 | 4.0 % |
| 2-HHB(F)-C | 13.0 % |
| 3-HHB(F)-C | 14.0 % |
| 3-H2BTB-2 | 4.0 % |

[Methods for Producing Compounds]

Compounds of the present invention expressed by the general formula (1) can readily be produced by using ordinary procedures employed in synthetic organic chemistry without any limitation. For instance, the compounds can readily be synthesized by selecting proper procedures described in Organic Synthesis, Organic Reactions, or Jikken Kagaku Kouza (Course of Chemical Experiment) (published by Maruzen Co., Ltd.), and using the selected procedures in combination.

That is, alkyl halide derivatives (17) are reacted with butyl lithium to lithiate and then reacted with carbon disulfide to produce dithiocarboxylic acid derivatives (18). Subsequently, the compounds (18) are converted into thioncarboxylic acid chloride with thionyl chloride, and alcohol derivatives (19) are reacted thereto in the presence of a base such as pyridine to form thioncarboxylic acid-O-esters (20). Then, diethylaminosulfur trifluoride (hereinafter referred to as DAST) is reacted with the esters (20), or quaternary ammonium dihydrogentrifluoride is reacted with the esters (20) in the presence of N-bromosuccine imide according to a method described in Laid-open Japanese Patent Publication No. Hei 5-255165 to produce the compounds of the general formula (1).

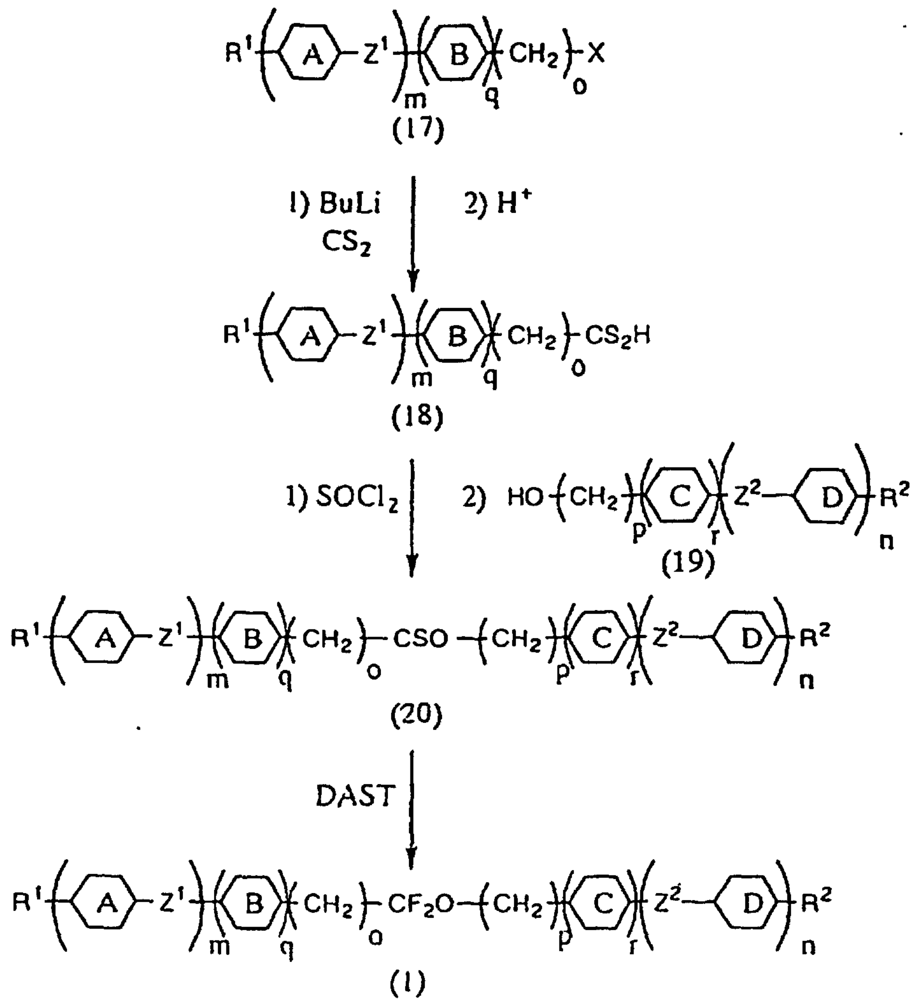


wherein $R^1$, $R^2$, rings A, B, C, and D, $Z^1$, $Z^2$, m, n, o, p, q and r have the same meaning as described above, and X represents chlorine, bromine, or iodine atom.

While the compounds expressed by the general formula (1) wherein $R^1$ is 1-alkenyl group can be produced by known procedures of organic synthesis, they can preferably be produced by the following methods. That is, ylides which are prepared by reacting the Wittig reagents (22) prepared in turn from an alkyl halide with a base such as a sodium alkoxide and alkyl lithium in tetrahydrofuran (hereinafter referred to as THF) are reacted with aldehyde derivatives (21) according to a method described in Organic Reactions, Vol. 14, Chapter 3 to obtain 1-alkenyl derivatives (17a). The 1-alkenyl derivatives (17a) are reacted with benzene sulfinic acid or p-toluene sulfinic acid to isomerize according to a method described in Japanese Patent Publication No. Hei 4-30382, or subjected to inversion of olefin to form derivatives in which alkenyl group is (E) isomer according to a method described in Japanese Patent Publication No. Hei 6-62462, when necessary. That is, according to the latter method, 1-alkenyl derivatives (17a) are reacted with m-chloroperbenzoic acid to lead to oxirane derivatives (23) and then reacted with dibromotriphenyl-phosphorane to produce 1,2-dibromo substituted isomers (24). (1E)-alkenyl derivatives (17b) can be produced by subjecting the isomers (24) to recrystallization to separate only erithro form isomer and then reducing it with zinc metal in acetic acid. Derivatives in which $R^1$ is 1-alkenyl group can be produced by using the compounds (17a) or (17b) instead of compounds (17) in the procedures for synthesizing the compounds of the general formula (1) described above.

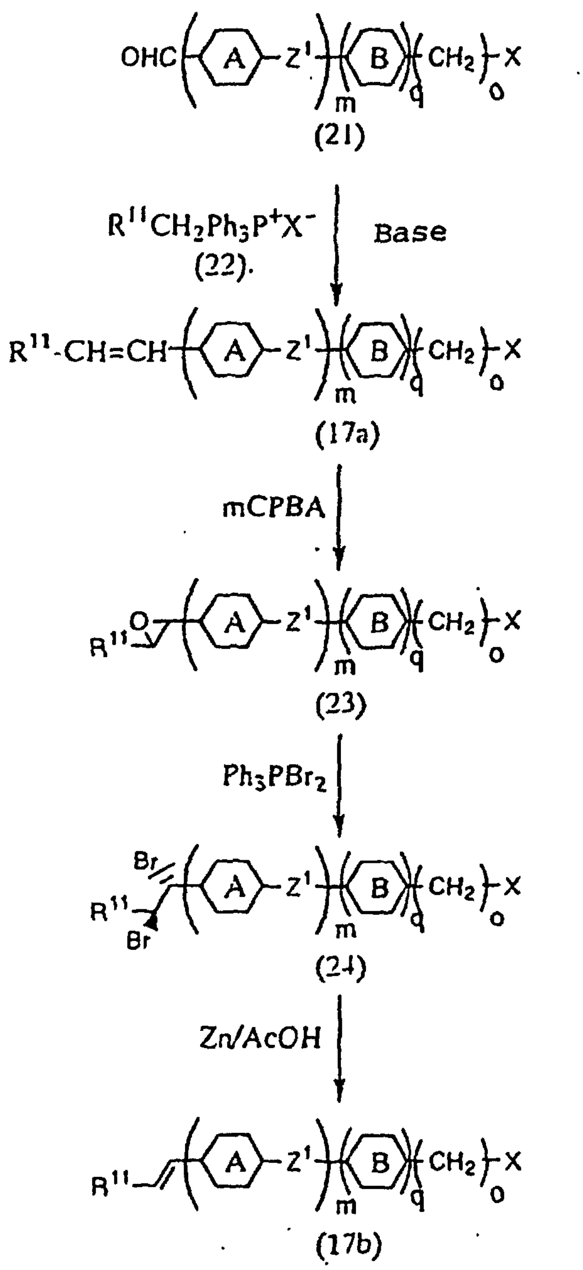


(21)

(17a)

(23)

(24)

(17b)

wherein rings A and B, $Z^1$, m, o and q have the same meaning as described above, X represents chlorine, bromine, or iodine atom, and $R^{11}$ represents an alkyl group having 1 to 13 carbon atoms.

Further, alkenyl derivatives other than 1-alkenyl can preferably be produced by the following methods. That is, ylides prepared by reacting methoxymethyltriphenylphosphonium chloride with a base such as a sodium alkoxide or alkyl lithium are reacted with aldehyde derivatives (21) to produce compounds (25). Subsequently, the compounds (25) can be treated with formic acid, acetic acid, hydrochloric acid, or sulfuric acid to produce aldehyde derivatives (21a) in which number of carbon atom is increased by 1. Aldehyde derivatives (21b) in which number of carbon atoms is increased by S can be produced by repeating s times the same procedure as that by which compounds (21) are led to compounds (21a). Alkenyl derivatives (17c) other than 1-alkenyl can be produced by reacting compounds (21a) with ylides prepared from the Wittig reagent (22) by the same procedures described above. Still further, the alkenyl derivatives (17c) can be subjected to the isomerization or inversion of olefin described above to produce trans (E)-alkenyl derivatives (17d) when necessary. Derivatives $R^1$ of which is an alkenyl group other than 1-alkenyl group can be produced by using the compounds (17c) or (17d) instead of compounds (17) in the procedures for synthesizing the compounds of the general formula (1) described above.

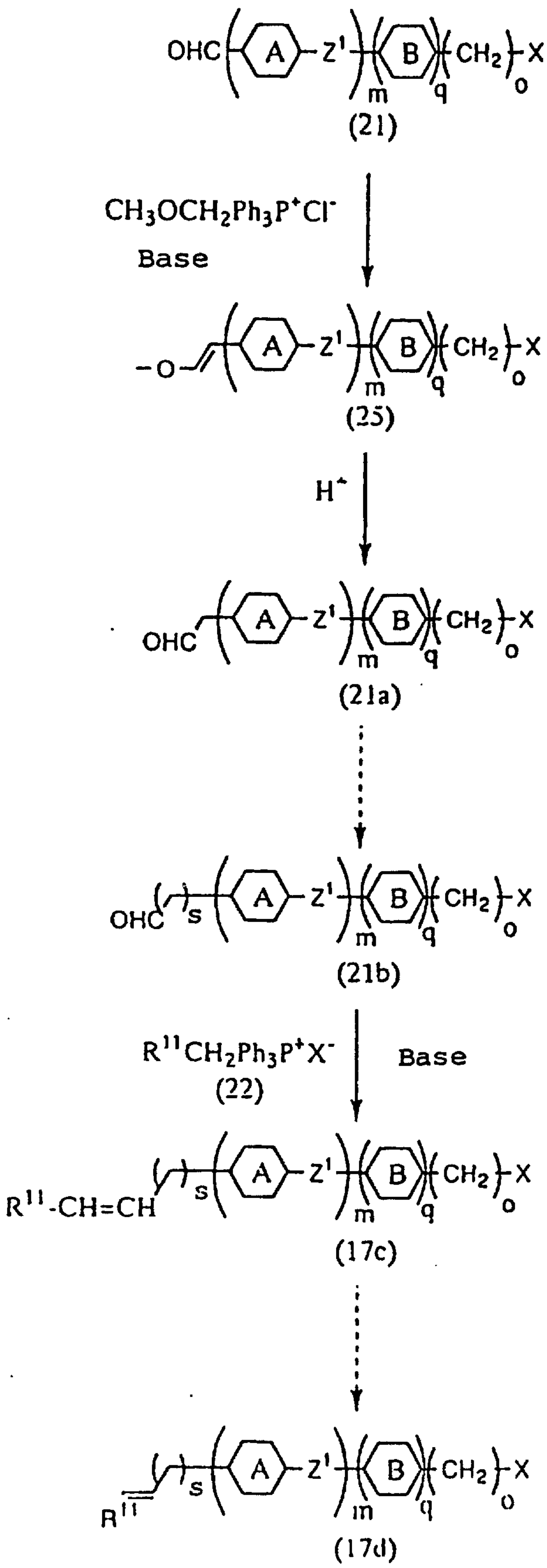
(21)
$CH_3OCH_2Ph_3P^+Cl^-$
Base
(25)
$H^+$
(21a)
(21b)
$R^{11}CH_2Ph_3P^+X^-$
(22)
Base
(17c)
(17d)

wherein rings A and B, $Z^{1,m,o}$ and q have the same meaning as described above, X represents chlorine, bromine, or iodine atom, $R^{11}$ represents an alkyl group having 1 to 13 carbon atoms, and S is an integer of 2 to 11.

Compounds having a partial structure of dicyclohexylethylene and expressed by one of the general formulas (1-34) to (1-37), (1-70) to (1-73), (1-90), (1-93), and (1-113) can preferably be produced by conducting the following procedures. That is, the Wittig reagents (27) prepared from alkyl halide derivatives (26) by a method described in Organic Reactions are reacted with a base such as a sodium alkoxide and alkyl lithium to prepare ylides, and then aldehyde derivatives (28) are reacted therewith to produce dicyclohexylethylene derivatives. The dicyclohexylethylene derivatives thus obtained can be subjected to the isomerization or inversion of olefin described above to produce trans isomers (17e). Further, compounds (30) can be produced by reacting compounds (29) instead of aldehyde derivatives (28), and alcohol derivatives (19a) can be produced by reducing the compounds (30) with lithium aluminum hydride. Compounds having a partial structure of dicyclohexylethylene described above can be produced by using the compounds (17e) or (19a) instead of compounds (17) or (19) in the procedures for synthesizing the compounds of the general formula (1) described above.

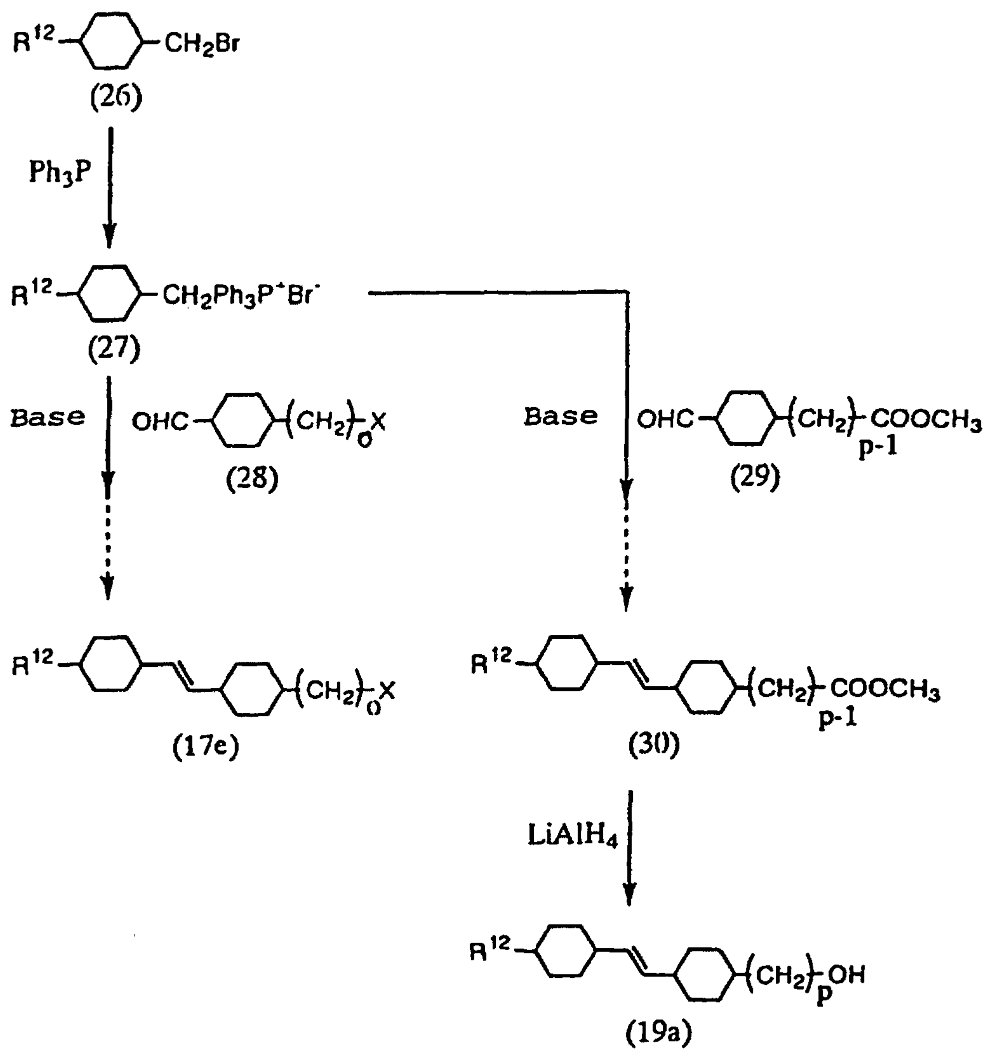


wherein o and p have the same meaning as described above, X represents chlorine, bromine, or iodine atom, and $R^{12}$ has the same meaning as the $R^1$ or R2 described above.

While the compounds having a partial structure of tolan and expressed by one of the general formulas (1-94) to (1-101), (1-103) to (1-110), and (1-112) can readily be synthesized by using an ordinary alkyne formation reaction, they can preferably be produced by using the following Castro reaction (J. Org. Chem., 28, 2163 (1963)). That is, compounds (17f) can be produced by reacting phenylacetylene derivatives (31) with halides (32) under a basic condition in the presence of a catalyst. In a similar way, compounds (34) can be produced by reacting phenylacetylene derivatives

(31) with compounds (33). Further, compounds (19b) can be produced by reducing the compounds (34) with lithium aluminum hydride. As the solvent in this reaction, a general amine compound such as diethylamine and triethylamine is preferable, and as the catalyst, a Pd- or Ni-containing compound is preferable. Compounds having the partial structure of tolan described above can be produced by using the compounds (17f) or (19b) instead of compounds (17) or (19) in the procedures for synthesizing the compounds of the general formula (1) described above.

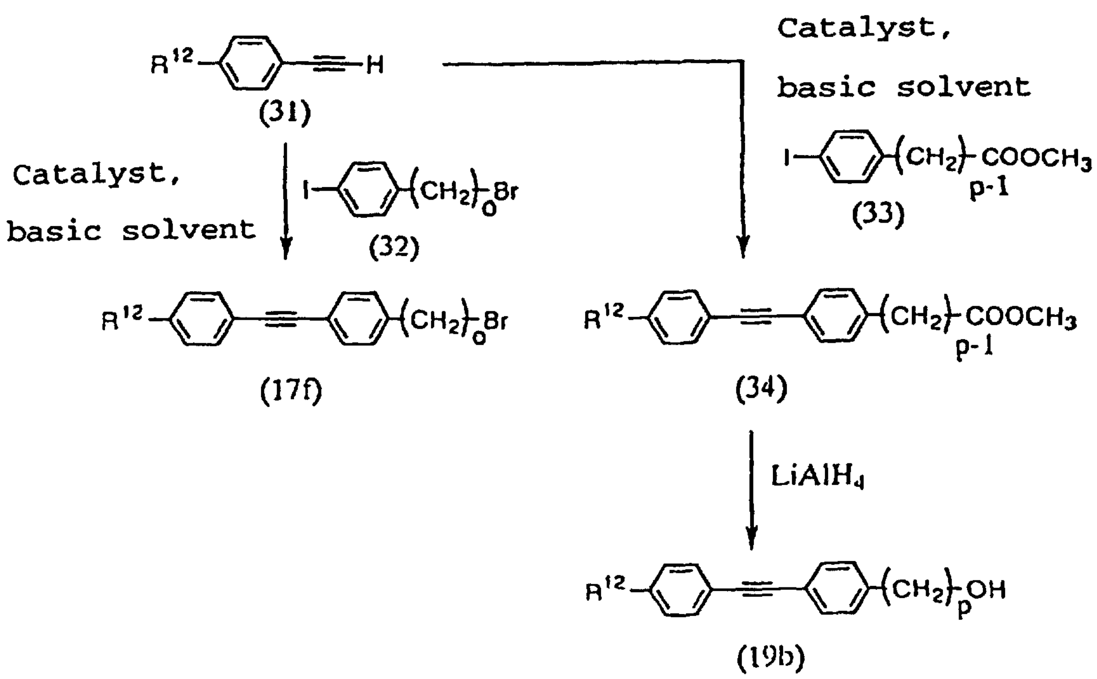


wherein o and p have the same meaning as described above, and $R^{12}$ have the same meaning as $R^1$ or $R^2$.

Phenylacetylene derivatives (31) can be produced from acetophenone derivatives (35). That is, the phenylacetylene derivatives (31) can be produced by chlorinating the carbonyl group of the acetophenone (35) and then carrying out dehydrogen chloride reaction in the presence of a strong base.

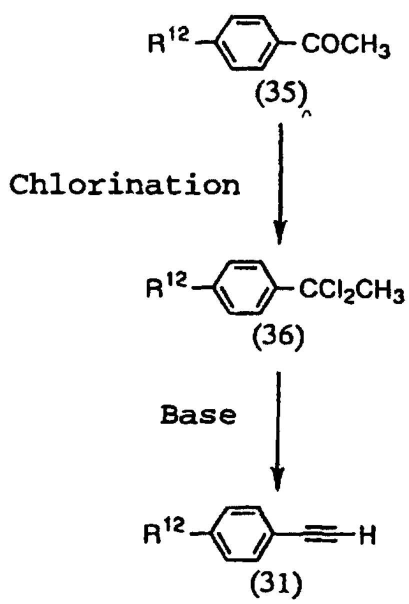

wherein $R^{12}$ have the same meaning as $R^1$ or $R^2$.

Also, a group of compounds other than those expressed by the sub-general formulas described above can readily be produced by selecting known reactions and using them in combination.

[Examples]

Now, the methods, for producing the compounds of the present invention and the methods for using them will be described in more detail with reference to Examples. However, it should be understood that the present invention is by no means restricted by such specific Examples. In the Examples, Cr indicates crystal; N, nematic phase; S, smectic phase; and Iso, isotropic liquid, and the unit of all phase transition temperatures is °C. In mass spectrum (GC-MS), M+ indicates molecular ion peak.

Example 1

Preparation of $\alpha,\alpha$-difluoro-4-propylbenzyloxy-2-(4-trifluoromethoxyphenyl)ethane (Compound expressed by the general formula (1) wherein q and r are 1, ring B and ring C are 1,4-phenylene group, m and n are 0, o is 0, p is 2, $R^1$ is n-$C_3H_7$, and $R^2$ is $OCF_3$ group; Compound No. 3)

First step

In a 500 ml, three-necked flask provided with a stirrer, thermometer, and nitrogen gas inlet tube, 3.9 g (158.2 mmol) of magnesium turnings were suspended in 100 ml of THF under nitrogen gas atmosphere while being stirred, and 200 ml of solution of 30 g (150.7 mmol) of 4-propylbromobenzene in THF was added dropwise thereto in 30 min so that the interior temperature did not exceed 50°C. The reaction solution was stirred while being heated at 50°C for 2 hours to age. Then, the interior temperature was lowered down to 5°C with an ice bath, and then 34.4 g (452.1 mmol) of carbon disulfide was added dropwise in 20 min so that the interior temperature did not exceed 10°C. The reaction solution was stirred while being maintained at a temperature lower than 10°C for 30 min, warmed up to room temperature, and stirred for 2 hours. The reaction solution was cooled again down to a temperature lower than 5°C, and 40 ml of 6N hydrochloric acid was added thereto to terminate the reaction. The reaction solution was extracted with 400 ml of diethyl ether, washed with 500 ml of water, and then dried over anhydrous magnesium sulfate. The diethyl ether was distilled off and the residue was concentrated to obtain 23.3 g of a deep purplish red (murex), oily product. This product was 4-propylphenyldithio-carboxylic acid.

Second step

In a 500 ml, eggplant type flask, 23.3 g of the 4-propylphenyldithiocarboxylic acid obtained by the procedures described above was dissolved in 300 ml of diethyl ether, 35.4 g (297.5 mmol) of thionyl chloride was added thereto at room temperature, and then the solution was heated to reflux on a hot water bath for 10 hours. The diethyl ether and unreacted thionyl chloride were distilled off under a reduced pressure produced with an aspirator, and the residue was concentrated to obtain 22.2 g of a deep murex, oily product. This product was 4-propylphenylthioncarboxylic acid chloride.

Next, in a 500 ml, three-necked flask provided with a stirrer, thermometer, and nitrogen gas inlet tube, 27.7 g (134.3 mmol) of 2-(4-trifluoromethoxyphenyl)ethanol (38) and 10.6 g (134.3 mmol) of pyridine were dissolved in 50 ml of toluene under nitrogen gas atmosphere, and then 80 ml of solution of 22.2 g of the 4-propylphenylthioncarboxylic acid chloride obtained by the procedures described above in toluene was added dropwise at room temperature while being stirred in 30 min. After finishing of the addition, the interior temperature was raised up to 60°C by heating the reaction solution on a hot water bath, and the solution was stirred for 3 hours to age. The reaction solution was cooled down to room temperature, 300 ml of water and 50 ml of 6N hydrochloric acid were added thereto, the toluene layer was separated, and the water layer was extracted with 300 ml of toluene. The extract layer was washed with 500 ml of water, 100 ml of 2N aqueous sodium hydroxide solution, and 500 ml of water in turn, and then dried over anhydrous magnesium sulfate. The toluene was distilled off under a reduced pressure, and the residue was concentrated to obtain 38.3 g of a deep murex, paste like product. The reaction product was purified by column chromatography by using a silica gel as filler and heptane as eluent, and then recrystallized from heptane to obtain 26.1 g of light yellow, needle-shaped crystals. These crystals were thioncarboxylic acid-O-ester derivative (37).

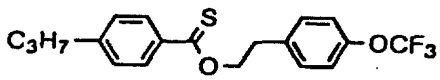

(37)

Third step

In a 300 ml, eggplant type flask, 10 g (27.1 mmol) of the thioncarboxylic acid-O-ester derivative (37) obtained by the procedures described above was dissolved in 70 ml of dichloromethane under nitrogen gas stream, 13.1 g (81.4 mmol) of DAST was added thereto at room temperature, and the solution was stirred at room temperature for 25 hours. The reaction solution was added to 200 ml of an ice water to terminate the reaction, the dichloromethane layer was separated, and then the water layer was extracted with 100 ml of dichloromethane. The extract layer was washed with 200 ml of water, 50 ml of 2N aqueous sodium hydroxide solution, and 200 ml of water in turn, and then dried over anhydrous magnesium sulfate. The dichloromethane was distilled off and the residue was concentrated to obtain 9.0 g of a light yellow, crystalline mixture. The reaction product was purified by column chromatography using a silica gel as filler and heptane as eluent, and then recrystallized from heptane to obtain 3.9 g of colorless, needle-shaped crystals. These crystals were objective α,α-difluoro-4-propylbenzyloxy-2-(4-trifluoromethoxyphenyl)ethane. Results of determination of various kind of spectra strongly supported its structure.
GC-MS : M+ 374

The 2-(4-trifluoromethoxyphenyl)ethanol used in the procedures described above was synthesized by the following method:

In a 1000 ml, three-necked flask provided with a stirrer, thermometer, and nitrogen gas inlet tube, 7.4 g (304.9 mmol) of magnesium turnings were suspended while being stirred in 200 ml of THF under nitrogen gas atmosphere, and 300 ml of solution of 70.0 g (290.4 mmol) of 4-trifluoromethoxybromobenzene in THF was added dropwise thereto in 45 min so that the internal temperature did not exceed 50°C. The reaction solution was stirred while being heated at 50°C with a hot water bath for 2 hours to age. Subsequently the internal temperature was lowered down to 5°C with an ice bath, 25.6 g (580.9 mmol) of ethylene oxide was added thereto in one breath, and then the solution was stirred at room temperature for 5 hours. The reaction solution was cooled again down to a temperature lower than 5°C, and then 80 ml of 6N hydrochloric acid was added to terminate the reaction. The reaction solution was extracted with 600 ml of diethyl ether, washed with 600 ml of water, and dried over anhydrous magnesium sulfate. The diethyl ether was distilled off and the residue was concentrated to obtain 45.5 g of a colorless, oily product. The reaction product thus obtained was purified by column chromatography using a silica gel as filler and heptane-ethyl acetate as eluent, and then the fraction of bp. 91~93°C/666,5Pa (91~93°C/5 Torr) was separated by distillation under a reduced pressure to obtain 30.9 g of a colorless oily product. This product was 2-(4-trifluoromethoxyphenyl)ethanol (38).

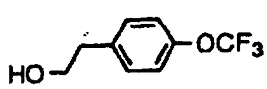

(38)

Following compounds can be prepared according to the preparation method described above with the exception that a 4-alkylbromobenzene having a chain length of alkyl group different from propyl is used in place of 4-propylbromobenzene.

(Compound No. 1)
α, α-difluoro-4-methylbenzyloxy-2-(4-trifluoromethoxyphenyl)ethane
(Compound No. 2)
α,α-difluoro-4-ethylbenzyloxy-2-(4-trifluoromethoxyphenyl)ethane
(Compound No. 4)
α, α-difluoro-4-butylbenzyloxy-2- (4-trifluoromethoxyphenyl) ethane
(Compound No. 5)
α,α-difluoro-4-pentylbenzyloxy-2-(4-trifluoromethoxyphenyl)ethane
(Compound No. 6)
α,α-difluoro-4-hexylbenzyloxy-2-(4-trifluoromethoxyphenyl)ethane
(Compound No. 7)
α,α-difluoro-4-heptylbenzyloxy-2-(4-trifluoromethoxyphenyl)ethane
(Compound No. 8)
α,α-difluoro-4-octylbenzyloxy-2-(4-trifluoromethoxyphenyl)ethane

(Compound No. 9)
α,α-difluoro-4-nonylbenzyloxy-2-(4-trifluoromethoxyphenyl)ethane
(Compound No. 10)
α,α-difluoro-4-decylbenzyloxy-2-(4-trifluoromethoxyphenyl)ethane

Following compounds can be prepared according to the preparation method described above with the exception that one of various known ethanol derivatives is used.

(Compound No. 11)
α,α-difluoro-4-ethylbenzyloxy-2-(4-fluorophenyl)ethane
(Compound No. 12)
α,α-difluoro-4-propylbenzyloxy-2-(4-fluorophenyl)ethane
(Compound No. 13)
α,α-difluoro-4-pentylbenzyloxy-2-(4-fluorophenyl)ethane
(Compound No. 14)
α,α-difluoro-4-ethylbenzyloxy-2-(3,4-difluorophenyl)ethane
(Compound No. 15)
α,α-difluoro-4-propylbenzyloxy-2-(3,4-difluorophenyl)ethane
(Compound No. 16)
α,α-difluoro-4-pentylbenzyloxy-2-(3,4-difluorophenyl)ethane
(Compound No. 17)
α,α-difluoro-4-ethylbenzyloxy-2-(3,4,5-trifluorophenyl)ethane
(Compound No. 18)
α,α-difluoro-4-propylbenzyloxy-2-(3,4,5-trifluorophenyl)ethane
(Compound No. 19)
α,α-difluoro-4-pentylbenzyloxy-2-(3,4,5-trifluorophenyl)ethane
(Compound No. 20)
α,α-difluoro-4-ethylbenzyloxy-2-(4-trifluoromethylphenyl)ethane
(Compound No. 21)
α,α-difluoro-4-propylbenzyloxy-2-(4-trifluoromethylphenyl)ethane
(Compound No. 22)
α,α-difluoro-4-pentylbenzyloxy-2-(4-trifluoromethylphenyl)ethane
(Compound No. 23)
α,α-difluoro-4-ethylbenzyloxy-2-(3-fluoro-4-trifluoromethylphenyl)ethane
(Compound No. 24)
α,α-difluoro-4-propylbenzyloxy-2-(3-fluoro-4-trifluoromethylphenyl)ethane
(Compound No. 25)
α,α-difluoro-4-pentylbenzyloxy-2-(3-fluoro-4-trifluoromethylphenyl)ethane
(Compound No. 26)
α,α-difluoro-4-ethylbenzyloxy-2-(3,5-difluoro-4-trifluoromethylphenyl)ethane
(Compound No. 27)
α,α-difluoro-4-propylbenzyloxy-2-(3,5-difluoro-4-trifluoromethylphenyl)ethane
(Compound No. 28)
α,α-difluoro-4-pentylbenzyloxy-2-(3,5-difluoro-4-trifluoromethylphenyl)ethane
(Compound No. 29)
α,α-difluoro-4-ethylbenzyloxy-2-(3-fluoro-4-trifluoromethoxyphenyl)ethane
(Compound No. 30)
α,α-difluoro-4-propylbenzyloxy-2-(3-fluoro-4-trifluoromethoxyphenyl)ethane
(Compound No. 31)
α,α-difluoro-4-pentylbenzyloxy-2-(3-fluoro-4-trifluoromethoxyphenyl)ethane
(Compound No. 32)
α,α-difluoro-4-ethylbenzyloxy-2-(3,5-difluoro-4-trifluoromethoxyphenyl)ethane
(Compound No. 33)
α,α-difluoro-4-propylbenzyloxy-2-(3,5-difluoro-4-trifluoromethoxyphenyl)ethane
(Compound No. 34)
α,α-difluoro-4-pentylbenzyloxy-2-(3,5-difluoro-4-trifluoromethoxyphenyl)ethane
(Compound No. 35)
α,α-difluoro-4-ethylbenzyloxy-2-(4-difluoromethoxyphenyl)ethane

(Compound No. 36)
α,α-difluoro-4-propylbenzyloxy-2-(4-difluoromethoxyphenyl)ethane
(Compound No. 37)
α,α-difluoro-4-pentylbenzyloxy-2-(4-difluoromethoxyphenyl)ethane
(Compound No. 38)
α,α-difluoro-4-ethylbenzyloxy-2-(3-fluoro-4-difluoromethoxyphenyl)ethane
(Compound No. 39)
α,α-difluoro-4-propylbenzyloxy-2-(3-fluoro-4-difluoromethoxyphenyl)ethane
(Compound No. 40)
α,α-difluoro-4-pentylbenzyloxy-2-(3-fluoro-4-difluoromethoxyphenyl)ethane
(Compound No. 41)
α,α-difluoro-4-ethylbenzyloxy-2-(3,5-difluoro-4-difluoromethoxyphenyl)ethane
(Compound No. 42)
α,α-difluoro-4-propylbenzyloxy-2-(3,5-difluoro-4-difluoromethoxyphenyl)ethane
(Compound No. 43)
α,α-difluoro-4-pentylbenzyloxy-2-(3,5-difluoro-4-difluoromethoxyphenyl)ethane
(Compound No. 44)
α,α-difluoro-4-ethylbenzyloxy-2-(4-cyanophenyl)ethane
(Compound No. 45)
α,α-difluoro-4-propylbenzyloxy-2-(4-cyanophenyl)ethane
(Compound No. 46)
α,α-difluoro-4-pentylbenzyloxy-2-(4-cyanophenyl)ethane
(Compound No. 47)
α,α-difluoro-4-ethylbenzyloxy-2-(3-fluoro-4-cyanophenyl)ethane
(Compound No. 48)
α,α-difluoro-4-propylbenzyloxy-2-(3-fluoro-4-cyanophenyl)ethane
(Compound No. 49)
α,α-difluoro-4-pentylbenzyloxy-2-(3-fluoro-4-cyanophenyl) ethane
(Compound No. 50)
α,α-difluoro-4-ethylbenzyloxy-2-(3,5-difluoro-4-cyanophenyl)ethane
(Compound No. 51)
α,α-difluoro-4-propylbenzyloxy-2-(3,5-difluoro-4-cyanophenyl)ethane
(Compound No. 52)
α,α-difluoro-4-pentylbenzyloxy-2-(3,5-difluoro-4-cyanophenyl)ethane
(Compound No. 53)
α,α-difluoro-4-ethylbenzyloxy-2-(4-propylphenyl) ethane
(Compound No. 54)
α,α-difluoro-4-propylbenzyloxy-2-(4-propylphenyl)ethane
(Compound No. 55)
α,α-difluoro-4-pentylbenzyloxy-2-(4-propylphenyl)ethane
(Compound No. 56)
α,α-difluoro-4-ethylbenzyloxy-2-(4-pentylphenyl)ethane
(Compound No. 57)
α,α-difluoro-4-propylbenzyloxy-2-(4-pentylphenyl)ethane
(Compound No. 58)
α,α-difluoro-4-pentylbenzyloxy-2-(4-pentylphenyl)ethane
(Compound No. 59)
α,α-difluoro-2-fluoro-4-propylbenzyloxy-2-(4-trifluoromethoxyphenyl)ethane
(Compound No. 60)
α,α-difluoro-2-fluoro-4-pentylbenzyloxy-2-(4-trifluoromethoxyphenyl)ethane
(Compound No. 61)
α,α-difluoro-2,6-difluoro-4-propylbenzyloxy-2-(4-trifluoromethoxyphenyl)ethane
(Compound No. 62)
α,α-difluoro-2-fluoro-4-propylbenzyloxy-2-(4-propylphenyl) ethane
(Compound No. 63)
α,α-difluoro-2-fluoro-4-propylbenzyloxy-2-(4-pentylphenyl) ethane
(Compound No. 64)
α,α-difluoro-2,6-difluoro-4-propylbenzyloxy-2-(4-pentylphenyl) ethane

(Compound No. 65)
α,α-difluoro-2,3-difluoro-4-propylbenzyloxy-2-(4-pentylphenyl)ethane

Example 2

Preparation of α,α-difluoro-4-(trans-4-propylcyclohexyl)-benzyloxy-2-(4-trifluoromethoxyphenyl)ethane (Compound expressed by the general formula (1) wherein m is 1, n is 0, q and r are 1, ring A is trans-1,4-cyclohexylene group, ring B and ring C are 1,4-phenylene group, o is 0, p is 2, $R^1$ is n-$C_3H_7$, and $R_2$ is $OCF_3$ group; Compound No. 68)

Objective α,α-difluoro-4-(trans-4-propylcyclohexyl)-benzyloxy-2-(4-trifluoromethoxyphenyl)ethane was obtained by conducting the same procedures as in Example 1 with the exception that 4-(trans-4-propylcyclohexyl)bromobenzene was used in place of 4-propylbromomobenzene. Results of determination of various kind of spectra supported its structure.
GC-MS : M+ 456

Following compounds can be prepared according to the preparation method described above with the exception that a 4-(trans-4-alkylcyclohexyl)bromobenzene having a chain length of alkyl group different from propyl is used in place of 4-(trans-4-propylcyclohexyl)- bromobenzene.

(Compound No. 66)
α,α-difluoro-4-(trans-4-methylcyclohexyl)benzyloxy-2-(4-trifluoromethoxyphenyl)ethane
(Compound No. 67)
α,α-difluoro-4-(trans-4-ethylcyclohexyl)benzyloxy-2-(4-trifluoromethoxyphenyl)ethane
(Compound No. 69)
α,α-difluoro-4-(trans-4-butylcyclohexyl)benzyloxy-2-(4-trifluoromethoxyphenyl)ethane
(Compound No. 70)
α,α-difluoro-4-(trans-4-pentylcyclohexyl)benzyloxy-2-(4-trifluoromethoxyphenyl)ethane
(Compound No. 71)
α,α-difluoro-4-(trans-4-hexylcyclohexyl)benzyloxy-2-(4-trifluoromethoxyphenyl)ethane
(Compound No. 72)
α,α-difluoro-4-(trans-4-heptylcyclohexyl)benzyloxy-2-(4-trifluoromethoxyphenyl)ethane
(Compound No. 73)
α,α-difluoro-4-(trans-4-octylcyclohexyl)benzyloxy-2-(4-trifluoromethoxyphenyl)ethane
(Compound No. 74)
α,α-difluoro-4-(trans-4-nonylcyclohexyl)benzyloxy-2-(4-trifluoromethoxyphenyl)ethane
(Compound No. 75)
α,α-difluoro-4-(trans-4-decylcyclohexyl)benzyloxy-2-(4-trifluoromethoxyphenyl)ethane

Following compounds can be prepared according to the method described above and the method shown in Example 1 with the exception that one of known ethanol derivatives is used in place of 2-(4-trifluoromethoxy-phenyl) ethanol.

(Compound No. 76)
α,α-difluoro-4-(trans-4-propylcyclohexyl)benzyloxy-2-(3-fluoro-4-trifluoromethoxyphenyl)ethane
(Compound No. 77)
α,α-difluoro-4-(trans-4-pentylcyclohexyl)benzyloxy-2-(3-fluoro-4-trifluoromethoxyphenyl)ethane
(Compound No. 78)
α,α-difluoro-4-(trans-4-propylcyclohexyl)benzyloxy-2-(3,5-difluoro-4-trifluoromethoxyphenyl)ethane
(Compound No. 79)
α,α-difluoro-4-(trans-4-pentylcyclohexyl)benzyloxy-2-(3,5-difluoro-4-trifluoromethoxyphenyl)ethane
(Compound No. 80)
α,α-difluoro-4-(trans-4-propylcyclohexyl)benzyloxy-2-(3,4-difluorophenyl)ethane
(Compound No. 81)
α,α-difluoro-4-(trans-4-pentylcyclohexyl)benzyloxy-2-(3,4-difluorophenyl)ethane
(Compound No. 82)
α,α-difluoro-4-(trans-4-propylcyclohexyl)benzyloxy-2-(3,4,5-trifluorophenyl)ethane
(Compound No. 83)
α,α-difluoro-4-(trans-4-pentylcyclohexyl)benzyloxy-2-(3,4,5-trifluorophenyl)ethane
(Compound No. 84)
α,α-difluoro-4-(trans-4-propylcyclohexyl)benzyloxy-2-(4-difluoromethoxyphenyl)ethane

(Compound No. 85)
α,α-difluoro-4-(trans-4-pentylcyclohexyl)benzyloxy-2-(4-difluoromethoxyphenyl)ethane
(Compound No. 86)
α,α-difluoro-4-(trans-4-propylcyclohexyl)benzyloxy-2-(3-fluoro-4-difluoromethoxyphenyl)ethane
(Compound No. 87)
α,α-difluoro-4-(trans-4-pentylcyclohexyl)benzyloxy-2-(3-fluoro-4-difluoromethoxyphenyl)ethane
(Compound No. 88)
α,α-difluoro-4-(trans-4-propylcyclohexyl)benzyloxy-2-(3,5-difluoro-4-difluoromethoxyphenyl)ethane
(Compound No. 89)
α,α-difluoro-4-(trans-4-pentylcyclohexyl)benzyloxy-2-(3,5-difluoro-4-difluoromethoxyphenyl)ethane
(Compound No. 90)
α,α-difluoro-4-(trans-4-propylcyclohexyl)benzyloxy-2-(4-cyanophenyl)ethane
(Compound No. 91)
α,α-difluoro-4-(trans-4-pentylcyclohexyl)benzyloxy-2-(4-cyanophenyl)ethane
(Compound No. 92)
α,α-difluoro-4-(trans-4-propylcyclohexyl)benzyloxy-2 -(3-fluoro-4-cyanophenyl)ethane
(Compound No. 93)
α,α-difluoro-4-(trans-4-pentylcyclohexyl)benzyloxy-2-(3-fluoro-4-cyanophenyl)ethane
(Compound No. 94)
α,α-difluoro-4-(trans-4-propylcyclohexyl)benzyloxy-2-(3,5-difluoro-4-cyanophenyl)ethane
(Compound No. 95)
α,α-difluoro-4-(trans-4-pentylcyclohexyl)benzyloxy-2-(3,5-difluoro-4-cyanophenyl)ethane
(Compound No. 96)
α,α-difluoro-4-(trans-4-propylcyclohexyl)benzyloxy-2-(4-propylphenyl)ethane
(Compound No. 97)
α,α-difluoro-4-(trans-4-pentylcyclohexyl)benzyloxy-2-(4-propylphenyl)ethane
(Compound No. 98)
α,α-difluoro-4-(trans-4-propylcyclohexyl)benzyloxy-2-(4-pentylphenyl) ethane
(Compound No. 99)
α,α-difluoro-4-(trans-4-pentylcyclohexyl)benzyloxy-2-(4-pentylphenyl)ethane
(Compound No. 100)
α,α-difluoro-4-(trans-4-propylcyclohexyl)benzyloxy-2-(3-fluoro-4-propylphenyl)ethane
(Compound No. 101)
α,α-difluoro-4-(trans-4-pentylcyclohexyl)benzyloxy-2-(3-fluoro-4-propylphenyl)ethane
(Compound No. 102)
α,α-difluoro-4-(trans-4-propylcyclohexyl)benzyloxy-2-(3-fluoro-4-pentylphenyl) ethane
(Compound No. 103)
α,α-difluoro-4-(trans-4-pentylcyclohexyl)benzyloxy-2-(3-fluoro-4-pentylphenyl)ethane
(Compound No. 104)
α,α-difluoro-4-(trans-4-propylcyclohexyl)benzyloxy-2-(2-fluoro-4-propylphenyl)ethane
(Compound No. 105)
α,α-difluoro-4-(trans-4-pentylcyclohexyl)benzyloxy-2-(2-fluoro-4-propylphenyl)ethane
(Compound No. 106)
α,α-difluoro-4-(trans-4-propylcyclohexyl)benzyloxy-2-(2-fluoro-4-pentylphenyl)ethane
(Compound No. 107)
α,α-difluoro-4-(trans-4-pentylcyclohexyl)benzyloxy-2-(2-fluoro-4-pentylphenyl)ethane
(Compound No. 108)
α,α-difluoro-2-fluoro-4-(trans-4-propylcyclohexyl)benzyloxy-2-(4-propylphenyl)ethane
(Compound No. 109)
α,α-difluoro-2-fluoro-4-(trans-4-pentylcyclohexyl)benzyloxy-2-(4-propylphenyl)ethane
(Compound No. 110)
α,α-difluoro-2-fluoro-4-(trans-4-propylcyclohexyl)benzyloxy-2-(4-pentylphenyl)ethane
(Compound No. 111)
α,α-difluoro-2-fluoro-4-(trans-4-pentylcyclohexyl)benzyloxy-2-(4-pentylphenyl)ethane
(Compound No. 112)
α,α-difluoro-2-fluoro-4-(trans-4-propylcyclohexyl)benzyloxy-2-(2-fluoro-4-propylphenyl)ethane
(Compound No. 113)
α,α-difluoro-2-fluoro-4-(trans-4-pentylcyclohexyl)benzyloxy-2-(2-fluoro-4-propylphenyl)ethane

(Compound No. 114)
α,α-difluoro-2-fluoro-4-(trans-4-propylcyclohexyl)benzyloxy-2-(2-fluoro-4-pentylphenyl)ethane
(Compound No. 115)
α,α-difluoro-2-fluoro-4-(trans-4-pentylcyclohexyl)benzyloxy-2-(2-fluoro-4-pentylphenyl)ethane
(Compound No. 116)
α,α-difluoro-2-fluoro-4-(trans-4-propylcyclohexyl)benzyloxy-2-(4-trifluoromethoxyphenyl)ethane
(Compound No. 117)
α,α-difluoro-2-fluoro-4-(trans-4-pentylcyclohexyl)benzyloxy-2-(4-trifluoromethoxyphenyl)ethane
(Compound No. 118)
α,α-difluoro-2-fluoro-4-(trans-4-propylcyclohexyl)benzyloxy-2-(3-fluoro-4-trifluoromethoxyphenyl)ethane
(Compound No. 119)
α,α-difluoro-2-fluoro-4-(trans-4-pentylcyclohexyl)benzyloxy-2-(3-fluoro-4-trifluoromethoxyphenyl)ethane
(Compound No. 120)
α,α-difluoro-2-fluoro-4-(trans-4-propylcyclohexyl)benzyloxy-2-(3,5-difluoro-4-trifluoromethoxyphenyl)ethane
(Compound No. 121)
α,α-difluoro-2-fluoro-4-(trans-4-pentylcyclohexyl)benzyloxy-2-(3,5-difluoro-4-trifluoromethoxyphenyl)ethane
(Compound No. 122)
α,α-difluoro-2-fluoro-4-(trans-4-propylcyclohexyl)benzyloxy-2-(3,4-difluorophenyl)ethane
(Compound No. 123)
α,α-difluoro-2-fluoro-4-(trans-4-pentylcyclohexyl)benzyloxy-2-(3,4-difluorophenyl)ethane
(Compound No. 124)
α,α-difluoro-2-fluoro-4-(trans-4-propylcyclohexyl)benzyloxy-2-(3,4,5-trifluorophenyl)ethane
(Compound No. 125)
α,α-difluoro-2-fluoro-4-(trans-4-pentylcyclohexyl)benzyloxy-2-(3,4,5-trifluorophenyl)ethane
(Compound No. 126)
α,α-difluoro-2-fluoro-4-(trans-4-propylcyclohexyl)benzyloxy-2-(4-cyanophenyl)ethane
(Compound No. 127)
α,α-difluoro-2-fluoro-4-(trans-4-pentylcyclohexyl)benzyloxy-2-(4-cyanophenyl)ethane
(Compound No. 128)
α,α-difluoro-2-fluoro-4-(trans-4-propylcyclohexyl)benzyloxy-2-(3-fluoro-4-cyanophenyl)ethane
(Compound No. 129)
α,α-difluoro-2-fluoro-4-(trans-4-pentylcyclohexyl)benzyloxy-2-(3-fluoro-4-cyanophenyl)ethane
(Compound No. 130)
α,α-difluoro-2-fluoro-4-(trans-4-propylcyclohexyl)benzyloxy-2-(3,5-difluoro-4-cyanophenyl)ethane
(Compound No. 131)
α,α-difluoro-2-fluoro-4-(trans-4-pentylcyclohexyl)benzyloxy-2-(3,5-difluoro-4-cyanophenyl)ethane
(Compound No. 132)
α,α-difluoro-2,3-difluoro-4-(trans-4-propylcyclohexyl)-benzyloxy-2-(4-propylphenyl)ethane
(Compound No. 133)
α,α-difluoro-2,3-difluoro-4-(trans-4-pentylcyclohexyl)-benzyloxy-2-(4-propylphenyl)ethane
(Compound No. 134)
α,α-difluoro-2,3-difluoro-4-(trans-4-propylcyclohexyl)-benzyloxy-2-(4-pentylphenyl)ethane
(Compound No. 135)
α,α-difluoro-2,3-difluoro-4-(trans-4-pentylcyclohexyl)-benzyloxy-2-(4-pentylphenyl)ethane
(Compound No. 136)
α,α-difluoro-2,3-difluoro-4-(trans-4-propylcyclohexyl)-benzyloxy-2-(2-fluoro-4-propylphenyl)ethane
(Compound No. 137)
α,α-difluoro-2,3-difluoro-4-(trans-4-pentylcyclohexyl)-benzyloxy-2-(2-fluoro-4-propylphenyl)ethane
(Compound No. 138)
α,α-difluoro-2,3-difluoro-4-(trans-4-propylcyclohexyl)-benzyloxy-2-(2-fluoro-4-pentylphenyl)ethane
(Compound No. 139)
α,α-difluoro-2,3-difluoro-4-(trans-4-pentylcyclohexyl)-benzyloxy-2-(2-fluoro-4-pentylphenyl)ethane
(Compound No. 140)
α,α-difluoro-2,3-difluoro-4-(trans-4-propylcyclohexyl)-benzyloxy-2-(3-fluoro-4-propylphenyl)ethane
(Compound No. 141)
α,α-difluoro-2,3-difluoro-4-(trans-4-pentylcyclohexyl)-benzyloxy-2-(3-fluoro-4-propylphenyl)ethane
(Compound No. 142)
α,α-difluoro-2,3-difluoro-4-(trans-4-propylcyclohexyl)-benzyloxy-2-(3-fluoro-4-pentylphenyl)ethane

(Compound No. 143)
α,α-difluoro-2,3-difluoro-4-(trans-4-pentylcyclohexyl)-benzyloxy-2-(3-fluoro-4-pentylphenyl)ethane
(Compound No. 144)
α,α-difluoro-2,3-difluoro-4-(trans-4-propylcyclohexyl)-benzyloxy-2-(2,3-difluoro-4-propylphenyl)ethane
(Compound No. 145)
α,α-difluoro-2,3-difluoro-4-(trans-4-pentylcyclohexyl)-benzyloxy-2-(2,3-difluoro-4-propylphenyl)ethane
(Compound No. 146)
α,α-difluoro-2,3-difluoro-4-(trans-4-propylcyclohexyl)-benzyloxy-2-(2,3-difluoro-4-ethoxyphenyl)ethane
(Compound No. 147)
α,α-difluoro-2,3-difluoro-4-(trans-4-pentylcyclohexyl)-benzyloxy-2-(2,3-difluoro-4-ethoxyphenyl)ethane
(Compound No. 148)
α,α-difluoro-2,6-difluoro-4-(trans-4-propylcyclohexyl)-benzyloxy-2-(4-trifluoromethoxyphenyl)ethane
(Compound No. 149)
α,α-difluoro-2,6-difluoro-4-(trans-4-pentylcyclohexyl)-benzyloxy-2-(4-trifluoromethoxyphenyl)ethane
(Compound No. 150)
α,α-difluoro-2,6-difluoro-4-(trans-4-propylcyclohexyl)-benzyloxy-2-(3-fluoro-4-trifluoromethoxyphenyl)ethane
(Compound No. 151)
α,α-difluoro-2,6-difluoro-4-(trans-4-pentylcyclohexyl)-benzyloxy-2-(3-fluoro-4-trifluoromethoxyphenyl)ethane
(Compound No. 152)
α,α-difluoro-2,6-difluoro-4-(trans-4-propylcyclohexyl)-benzyloxy-2-(3,5-difluoro-4-trifluoromethoxyphenyl)ethane
(Compound No. 153)
α,α-difluoro-2,6-difluoro-4-(trans-4-pentylcyclohexyl)-benzyloxy-2-(3,5-difluoro-4-trifluoromethoxyphenyl)ethane
(Compound No. 154)
α,α-difluoro-2,6-difluoro-4-(trans-4-propylcyclohexyl)-benzyloxy-2-(3,4-difluorophenyl)ethane
(Compound No. 155)
α,α-difluoro-2,6-difluoro-4-(trans-4-pentylcyclohexyl)-benzyloxy-2-(3,4-difluorophenyl)ethane
(Compound No. 156)
α,α-difluoro-2,6-difluoro-4-(trans-4-propylcyclohexyl)-benzyloxy-2-(3,4,5-trifluorophenyl)ethane
(Compound No. 157)
α,α-difluoro-2,6-difluoro-4-(trans-4-pentylcyclohexyl)-benzyloxy-2-(3,4,5-trifluorophenyl)ethane
(Compound No. 158)
α,α-difluoro-2,6-difluoro-4-(trans-4-propylcyclohexyl)-benzyloxy-2-(4-cyanophenyl)ethane
(Compound No. 159)
α,α-difluoro-2,6-difluoro-4-(trans-4-pentylcyclohexyl)-benzyloxy-2-(4-cyanophenyl)ethane
(Compound No. 160)
α,α-difluoro-2,6-difluoro-4-(trans-4-propylcyclohexyl)-benzyloxy-2-(3-fluoro-4-cyanophenyl)ethane
(Compound No. 161)
α,α-difluoro-2,6-difluoro-4-(trans-4-pentylcyclohexyl)-benzyloxy-2-(3-fluoro-4-cyanophenyl)ethane
(Compound No. 162)
α,α-difluoro-2,6-difluoro-4-(trans-4-propylcyclohexyl)-benzyloxy-2-(3,5-difluoro-4-cyanophenyl)ethane
(Compound No. 163)
α,α-difluoro-2,6-difluoro-4-(trans-4-pentylcyclohexyl)-benzyloxy-2-(3,5-difluoro-4-cyanophenyl)ethane
(Compound No. 164)
α,α-difluoro-4-(trans-4-methylcyclohexyl)benzyloxy-2-(4-(trans-4-methylcyclohexyl)phenyl)ethane
(Compound No. 165)
α,α-difluoro-4-(trans-4-methylcyclohexyl)benzyloxy-2-(4-(trans-4-ethylcyclohexyl)phenyl)ethane
(Compound No. 166)
α,α-difluoro-4-(trans-4-methylcyclohexyl)benzyloxy-2-(4-(trans-4-propylcyclohexyl)phenyl)ethane
(Compound No. 167)
α,α-difluoro-4-(trans-4-methylcyclohexyl)benzyloxy-2-(4-(trans-4-pentylcyclohexyl)phenyl)ethane
(Compound No. 168)
α,α-difluoro-4-(trans-4-methylcyclohexyl)benzyloxy-2-(4-(trans-4-hexylcyclohexyl)phenyl)ethane
(Compound No. 169)
α,α-difluoro-4-(trans-4-ethylcyclohexyl) benzyloxy-2- (4- (trans-4-methylcyclohexyl)phenyl)ethane
(Compound No. 170)
α,α-difluoro-4-(trans-4-ethylcyclohexyl)benzyloxy-2-(4-(trans-4-ethylcyclohexyl)phenyl)ethane
(Compound No. 171)
α,α-difluoro-4-(trans-4-ethylcyclohexyl)benzyloxy-2-(4-(trans-4-propylcyclohexyl)phenyl)ethane

(Compound No. 172)
α,α-difluoro-4-(trans-4-ethylcyclohexyl)benzyloxy-2-(4-(trans-4-pentylcyclohexyl)phenyl)ethane
(Compound No. 173)
α,α-difluoro-4-(trans-4-propylcyclohexyl)benzyloxy-2-(4- (trans-4-methylcyclohexyl)phenyl)ethane
(Compound No. 174)
α,α-difluoro-4-(trans-4-propylcyclohexyl)benzyloxy-2-(4-(trans-4-ethylcyclohexyl)phenyl)ethane
(Compound No. 175)
α,α-difluoro-4-(trans-4-propylcyclohexyl)benzyloxy-2-(4-(trans-4-propylcyclohexyl)phenyl)ethane
(Compound No. 176)
α,α-difluoro-4-(trans-4-propylcyclohexyl)benzyloxy-2-(4-(trans-4-pentylcyclohexyl)phenyl)ethane
(Compound No. 177)
α,α-difluoro-4-(trans-4-pentylcyclohexyl)benzyloxy-2-(4-(trans-4-methylcyclohexyl)phenyl)ethane
(Compound No. 178)
α,α-difluoro-4-(trans-4-pentylcyclohexyl)benzyloxy-2-(4-(trans-4-ethylcyclohexyl)phenyl)ethane
(Compound No. 179)
α,α-difluoro-4-(trans-4-pentylcyclohexyl)benzyloxy-2-(4-(trans-4-propylcyclohexyl)phenyl)ethane
(Compound No. 180)
α,α-difluoro-4-(trans-4-pentylcyclohexyl)benzyloxy-2-(4-(trans-4-pentylcyclohexyl)phenyl)ethane
(Compound No. 181)
α,α-difluoro-2-fluoro-4-(trans-4-methylcyclohexyl)benzyloxy-2-(4-(trans-4-methylcyclohexyl)phenyl)ethane
(Compound No. 182)
α,α-difluoro-2-fluoro-4-(trans-4-methylcyclohexyl)benzyloxy-2-(4-(trans-4-ethylcyclohexyl)phenyl)ethane
(Compound No. 183)
α,α-difluoro-2-fluoro-4-(trans-4-methylcyclohexyl)benzyloxy-2-(4-(trans-4-propylcyclohexyl)phenyl)ethane
(Compound No. 184)
α,α-difluoro-2-fluoro-4-(trans-4-methylcyclohexyl)benzyloxy-2-(4-(trans-4-pentylcyclohexyl)phenyl)ethane
(Compound No. 185)
α,α-difluoro-2-fluoro-4-(trans-4-ethylcyclohexyl)benzyloxy-2-(4-(trans-4-methylcyclohexyl)phenyl)ethane
(Compound No. 186)
α,α-difluoro-2-fluoro-4-(trans-4-ethylcyclohexyl)benzyloxy-2-(4-(trans-4-ethylcyclohexyl)phenyl)ethane
(Compound No. 187)
α,α-difluoro-2-fluoro-4-(trans-4-ethylcyclohexyl)benzyloxy-2-(4-(trans-4-propylcyclohexyl)phenyl)ethane
(Compound No. 188)
α,α-difluoro-2-fluoro-4-(trans-4-ethylcyclohexyl)benzyloxy-2-(4-(trans-4-pentylcyclohexyl)phenyl)ethane
(Compound No. 189)
α,α-difluoro-2-fluoro-4-(trans-4-propylcyclohexyl)benzyloxy-2-(4-(trans-4-methylcyclohexyl)phenyl)ethane
(Compound No. 190)
α,α-difluoro-2-fluoro-4-(trans-4-propylcyclohexyl)benzyloxy-2-(4-(trans-4-ethylcyclohexyl)phenyl)ethane
(Compound No. 191)
α,α-difluoro-2-fluoro-4-(trans-4-propylcyclohexyl)benzyloxy-2-(4-(trans-4-propylcyclohexyl)phenyl)ethane
(Compound No. 192)
α,α-difluoro-2-fluoro-4-(trans-4-propylcyclohexyl)benzyloxy-2-(4-(trans-4-pentylcyclohexyl)phenyl)ethane
(Compound No. 193)
α,α-difluoro-2-fluoro-4-(trans-4-pentylcyclohexyl)benzyloxy-2-(4-(trans-4-methylcyclohexyl)phenyl)ethane
(Compound No. 194)
α,α-difluoro-2-fluoro-4-(trans-4-pentylcyclohexyl)benzyloxy-2-(4-(trans-4-ethylcyclohexyl)phenyl)ethane
(Compound No. 195)
α,α-difluoro-2-fluoro-4-(trans-4-pentylcyclohexyl)benzyloxy-2-(4-(trans-4-propylcyclohexyl)phenyl)ethane
(Compound No. 196)
α,α-difluoro-2-fluoro-4-(trans-4-pentylcyclohexyl)benzyloxy-2-(4-(trans-4-pentylcyclohexyl)phenyl)ethane
(Compound No. 197)
α,α-difluoro-2,3-difluoro-4-(trans-4-methylcyclohexyl)-benzyloxy-2-(4-(trans-4-methylcyclohexyl)phenyl)ethane
(Compound No. 198)
α,α-difluoro-2,3-difluoro-4-(trans-4-methylcyclohexyl)-benzyloxy-2-(4-(trans-4-ethylcyclohexyl)phenyl)ethane
(Compound No. 199)
α,α-difluoro-2,3-difluoro-4-(trans-4-methylcyclohexyl)-benzyloxy-2-(4-(trans-4-propylcyclohexyl)phenyl)ethane
(Compound No. 200)
α,α-difluoro-2,3-difluoro-4-(trans-4-methylcyclohexyl)-benzyloxy-2-(4-(trans-4-pentylcyclohexyl)phenyl)ethane

(Compound No. 201)
α,α-difluoro-2,3-difluoro-4-(trans-4-ethylcyclohexyl)-benzyloxy-2-(4-(trans-4-methylcyclohexyl)phenyl)ethane
(Compound No. 202)
α,α-difluoro-2,3-difluoro-4-(trans-4-ethylcyclohexyl)-benzyloxy-2-(4-(trans-4-ethylcyclohexyl)phenyl)ethane
(Compound No. 203)
α,α-difluoro-2,3-difluoro-4-(trans-4-ethylcyclohexyl)-benzyloxy-2-(4-(trans-4-propylcyclohexyl)phenyl)ethane
(Compound No. 204)
α,α-difluoro-2,3-difluoro-4-(trans-4-ethylcyclohexyl)-benzyloxy-2-(4-(trans-4-pentylcyclohexyl)phenyl)ethane
(Compound No. 205)
α,α-difluoro-2,3-difluoro-4-(trans-4-propylcyclohexyl)-benzyloxy-2-(4-(trans-4-methylcyclohexyl)phenyl)ethane
(Compound No. 206)
α,α-difluoro-2,3-difluoro-4-(trans-4-propylcyclohexyl)-benzyloxy-2-(4-(trans-4-ethylcyclohexyl)phenyl)ethane
(Compound No. 207)
α,α-difluoro-2,3-difluoro-4-(trans-4-propylcyclohexyl)-benzyloxy-2-(4-(trans-4-propylcyclohexyl)phenyl)ethane
(Compound No. 208)
α,α-difluoro-2,3-difluoro-4-(trans-4-propylcyclohexyl)-benzyloxy-2-(4-(trans-4-pentylcyclohexyl)phenyl)ethane
(Compound No. 209)
α,α-difluoro-2,3-difluoro-4-(trans-4-pentylcyclohexyl)-benzyloxy-2-(4-(trans-4-methylcyclohexyl)phenyl)ethane
(Compound No. 210)
α,α-difluoro-2,3-difluoro-4-(trans-4-pentylcyclohexyl)-benzyloxy-2-(4-(trans-4-ethylcyclohexyl)phenyl)ethane
(Compound No. 211)
α,α-difluoro-2,3-difluoro-4-(trans-4-pentylcyclohexyl)-benzyloxy-2-(4-(trans-4-propylcyclohexyl)phenyl)ethane
(Compound No. 211)
α,α-difluoro-2,3-difluoro-4-(trans-4-pentylcyclohexyl)-benzyloxy-2-(4-(trans-4-pentylcyclohexyl)phenyl)ethane

Example 3

Preparation of (trans-4-propylcyclohexyl)methyleneoxy-1,1-difluoro-2-(trans-4-ethenylcyclohexyl)ethane (Compound expressed by the general formula (1) wherein m and n are 0, q and r are 1, ring B and ring C are 1,4-cyclohexylene group, o and p are 1, $R^1$ is ethenyl group, and $R^2$ is n-$C_3H_7$; Compound No. 215)

Preparation process is broadly divided into two stages of

1) preparation of 4-ethenylcyclohexylmethylenebormide (39) and
2) preparation of (trans-4-propylcyclohexyl)methyleneoxy-1,1-difluoro-2-(trans-4-ethenylcyclohexyl)ethane.

The preparation process is described in detail with the process divided into steps.

1) Synthesis of 4-ethenylcyclohexylmethylene bromide (39)

First step

In a 1000 ml, three-necked flask provided with a stirrer, thermometer, and nitrogen gas inlet tube, 158.0 g (461.0 mmol) of methoxymethyltriphenylphosphonium chloride was suspended in 400 ml of THF under nitrogen gas atmosphere and cooled down to a temperature lower than -50°C in a dry ice-acetone bath, 54.3 g (484.1 mmol) of potassium-t-butoxide was added thereto, and the suspension was stirred while being maintained at the same temperature lower than -50°C for 2 hours to prepare an ylide. Then, 150 ml of solution of 60.0 g(384.2 mmol) of 1,4-cyclohexanedionemonoethyleneketal in THF was added dropwise at the same temperature in 25 min, stirred at the same temperature for 1 hour, warmed up to room temperature, and then further stirred at room temperature for 4 hours. Water in an amount of 400 ml was added to the reaction solution to terminate the reaction, the THF layer was separated, and the water layer was extracted with 400 ml of toluene. The extract layer was washed with 600 ml of water and dried over anhydrous magnesium sulfate. The solvent was distilled off under a reduced pressure, and the residue was concentrated. The concentrated residue was purified by column chromatography using a silica gel as filler and toluene as eluent to obtain 64.4 g of a yellowish brown reaction product. Next, the reaction product was dissolved in 200 ml of toluene in a 1000 ml, eggplant type flask, 81.3 g (1748.0 mmol) of 99 % formic acid was added thereto, and the solution was heated to reflux for 2 hours. The reaction solution was concentrated as it was under a reduced pressure to obtain 44.1 g of a concentrated product. The concentrated residue was subjected to distillation under a reduced pressure to separate a fraction of bp. 141°C/42,6 hPa (141°C/32 Torr) thereby obtain 30. g of a colorless oily product. This product was 4-formylcyclohexanone.

Second step

In a 500 ml, three-necked flask provided with a stirrer and thermometer, 30.4 g (241.2 mmol) of the 4-formylcyclohexanone obtained by the procedures described above was dissolved in 250 ml of ethanol, 7.3 g (192.9 mmol) of sodium boron hydride was added thereto at room temperature while being stirred, and stirred at room temperature for 5 hours. The reaction solution was cooled down to a temperature lower than 10°C under cooling with ice, and 80 ml of 3N aqueous hydrochloric acid solution was added thereto to terminate the reaction. From the reaction solution, ethanol was distilled off under a reduced pressure, and then the solution was extracted with 400 ml of diethyl ether. The extract layer was washed with 300 ml of water and dried over anhydrous magnesium sulfate, and the solvent was distilled off to obtain 30.3 g of alcohol derivative (40).

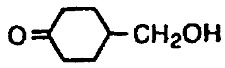

(40)

Third step

In a 500 ml, three-necked flask provided with a stirrer, cooling tube, and thermometer, 30.3 g of alcohol derivative (40) was dissolved in 40 ml of xylene, 122.0 g (709.2 mmol) of 47 % aqueous hydrobromic acid solution was added thereto, and the solution was heated to reflux for 8 hours. The solution was cooled down to room temperature, 300 ml of water was added to the reaction solution, and the solution was extracted with 400 ml of ethyl acetate. The extract layer was washed with 400 ml of water, 100 ml of saturated aqueous sodium bicarbonate solution, and 400 ml of water in turn, and dried over anhydrous magnesium sulfate. The solvent was distilled off under a reduced pressure to obtain 43.5 g of a concentrated product. The concentrated product was purified by column chromatography using a silica gel as filler and toluene as eluent to obtain 37.9 g of a colorless oily product. This product was bromine substituted compound (41).

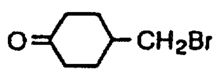

(41)

Fourth step

In a 500 ml, three-necked flask provided with a stirrer, thermometer, and nitrogen gas inlet tube, 81.7 g (238.3 mmol) of methoxymethyltriphenylphosphonium chloride was suspended in 400 ml of THF under nitrogen gas atmosphere and cooled down to a temperature lower than -50°C in a dry ice-acetone bath, 28.1 g (250.2 mmol) of potassium-t-butoxide was added thereto, and the mixture was stirred while being maintained at the same temperature lower than -50°C for 2 hours to prepare an ylide. Subsequently, 100 ml of solution of 37.9 g (198.6 mmol) of the bromine substituted compound obtained by the procedures described above in THF was added dropwise thereto at the same temperature in 20 min, stirred at the same temperature for 1 hour, warmed up to room temperature, and further stirred at room temperature for 4 hours. Water in an amount of 200 ml was added to the reaction solution to terminate the reaction, the THF layer was separated, and the water layer was extracted with 200 ml of toluene. The extract layer was washed with 400 ml of water and dried over anhydrous magnesium sulfate. The solvent was distilled off under a reduced pressure and the residue was concentrated. The concentrated residue was purified by column chromatography using a silica gel as filler and toluene as eluent to obtain 40.9 g of a yellowish brown oily reaction product. Then, the reaction product was dissolved in 200 ml of toluene in a 500 ml, eggplant type flask, 21.7 g (466.8 mmol) of 99 % formic acid was added thereto, and the solution was heated to reflux for 2 hours. The reaction solution was concentrated as it was under a reduced pressure to obtain 31.8 g of a concentrated product. The concentrated residue was purified by column chromatography using a silica gel as filler and toluene as eluent to obtain 27.9 g of a colorless oily reaction product. This product was aldehyde derivative (42).

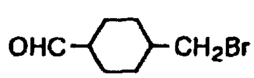

(42)

Fifth step

In a 500 ml, three-necked flask provided with a stirrer, thermometer, and nitrogen gas inlet tube, 58.4 g (163.6 mmol) of methyltriphenylphosphonium bromide was suspended in 200 ml of THF under nitrogen gas atmosphere and cooled down to a temperature lower than -50°C in a dry ice-acetone bath, 19.3 g (171.8 mmol) of potassium-t-butoxide was added thereto, and the mixture was stirred while being maintained at the same temperature lower than -50°C for 2 hours to prepare an ylide. Then, 80 ml of solution of 27.9 g (136.4 mmol) of the aldehyde derivative (42) obtained by the procedures described above in THF was added dropwise thereto at the same temperature in 15 min, stirred at the same temperature for 1 hour, warmed up to room temperature, and then further stirred for 4 hours. Water in an amount of 200 ml was added to the reaction solution to terminate the reaction, the THF layer was separated, and the water layer was extracted with 200 ml of toluene. The extract layer was washed with 400 ml of water and dried over anhydrous magnesium sulfate. The solvent was distilled off under a reduced pressure and the residue was concentrated. The concentrated residue was purified by column chromatography using a silica gel as filler and a mixed solvent of heptane-toluene as eluent to obtain 24.7 g of a colorless oily reaction product. This product was 4-ethenylcyclohexylmethylene bromide (39).

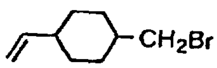

(39)

2) Preparation of (trans-4-propylcyclohexyl)methyleneoxy-1,1-difluoro-2-(trans-4-ethenylcyclohexyl)ethane

Sixth step

In a 500 ml, three-necked flask provided with a stirrer, thermometer, and nitrogen gas inlet tube, 24.7 g (121.4 mmol) of the 4-ethenylcyclohexylmethylene bromide (39) obtained by the procedures described above was dissolved in 80 ml of THF and cooled down to a temperature lower than -50°C with a dry ice-acetone bath, and 113.8 ml of 1.6 M n-butyl lithium solution was added dropwise thereto while being maintained at a temperature lower than -50°C in 50 min. After finishing of the dropping, the solution was further stirred at the same temperature for 2 hours, and 27.7 g (364.2 mmol) of carbon disulfide was added dropwise at the same temperature lower than -50°C in 15 min. After finishing of the dropping, the solution was stirred for 2 hours, gradually warded up to room temperature in 20 min, and further stirred for 2 hours. Water in an amount of 100 ml and 50 ml of 3N aqueous hydrochloric acid were added to the reaction solution to terminate the reaction, and then reaction product was extracted with 300 ml of diethyl ether. The extract layer was washed with 400 ml of water and dried over anhydrous magnesium sulfate. The solvent was distilled off to obtain 24.1 g of a murex oily product. This product was dithiocarboxylic acid derivative (43).

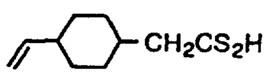

(43)

Seventh step

From 24.1 g of the dithiocarboxylic acid derivative (43) described above and 28.0 g (182.1 mmol) of trans-4-propylcyclohexanecarbinol (44), 3.9 of the objective product, (trans-4-propylcyclohexyl)methyleneoxy-1,1-difluoro-2-(trans-4-ethenylcyclohexyl)ethane was obtained according to the preparation method shown in Example 1. Results of determination of various kind of spectra well supported its structure. GC-MS: M+ 328

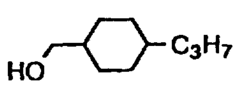

(44)

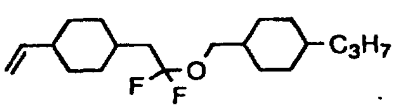

(Compound No. 215)

Following compounds can be prepared according to the preparation method described above with the exception that a trans-4-alkylcyclohexanecarbinol or trans-4-(trans-4-alkylcyclohexyl)cyclohexanecarbinol having an alkyl chain different from propyl is used in place of trans-4-propylcyclohexanecarbinol (44) in the sixth step.

(Compound No. 213)
(trans-4-methylcyclohexyl)methyleneoxy-1,1-difluoro-2-(trans-4-ethenylcyclohexyl)ethane
(Compound No. 214)
(trans-4-ethylcyclohexyl)methyleneoxy-1,1-difluoro-2-(trans-4-ethenylcyclohexyl)ethane
(Compound No. 216)
(trans-4-butylcyclohexyl)methyleneoxy-1,1-difluoro-2-(trans-4-ethenylcyclohexyl)ethane
(Compound No. 217)
(trans-4-pentylcyclohexyl)methyleneoxy-1,1-difluoro-2-(trans-4-ethenylcyclohexyl)ethane
(Compound No. 218)
(trans-4-hexylcyclohexyl)methyleneoxy-1,1-difluoro-2-(trans-4-ethenylcyclohexyl)ethane
(Compound No. 219)
(trans-4-heptylcyclohexyl)methyleneoxy-1,1-difluoro-2-(trans-4-ethenylcyclohexyl)ethane
(Compound No. 220)
(trans-4-octylcyclohexyl)methyleneoxy-1,1-difluoro-2-(trans-4-ethenylcyclohexyl)ethane
(Compound No. 221)
(trans-4-nonylcyclohexyl)methyleneoxy-1,1-difluoro-2-(trans-4-ethenylcyclohexyl)ethane
(Compound No. 222)
(trans-4-decylcyclohexyl)methyleneoxy-1,1-difluoro-2-(trans-4-ethenylcyclohexyl)ethane
(Compound No. 223)
trans-4-(trans-4-methylcyclohexyl)cyclohexylmethyleneoxy-1,1-difluoro-2-(trans-4-ethenylcyclohexyl)ethane
(Compound No. 224)
trans-4-(trans-4-ethylcyclohexyl)cyclohexylmethyleneoxy-1,1-difluoro-2-(trans-4-ethenylcyclohexyl)ethane
(Compound No. 225)
trans-4-(trans-4-propylcyclohexyl)cyclohexylmethyleneoxy-1,1-difluoro-2-(trans-4-ethenylcyclohexyl)ethane
(Compound No. 226)
trans-4-(trans-4-pentylcyclohexyl)cyclohexylmethyleneoxy-1,1-difluoro-2-(trans-4-ethenylcyclohexyl)ethane

Following compounds can be prepared according to the preparation method described above with the exception that ethyltriphenylphosphonium iodide is used in place of methyltriphenylphosphonium bromide in the fifth step, and the product is reacted with benzenesulfinic acid or p-toluenesulfinic acid to isomerize according to the method described in Japanese Patent Publication No. Hei 4-30382, or subjected to steric inversion of olefin according to a method described in Japanese Patent Publication No. Hei 6-62462.

(Compound No. 227)
(trans-4-methylcyclohexyl)methyleneoxy-1,1-difluoro-2-(trans-4-((E)-1-propenyl)cyclohexyl)ethane
(Compound No. 228)
(trans-4-ethylcyclohexyl)methyleneoxy-1,1-difluoro-2-(trans-4-((E)-1-propenyl)cyclohexyl)ethane
(Compound No. 229)
(trans-4-propylcyclohexyl)methyleneoxy-1,1-difluoro-2-(trans-4-((E)-1-propenyl)cyclohexyl)ethane
(Compound No. 230)
(trans-4-pentylcyclohexyl)methyleneoxy-1,1-difluoro-2-(trans-4-((E)-1-propenyl)cyclohexyl)ethane
(Compound No. 231)
trans-4-(trans-4-methylcyclohexyl)cyclohexylmethyleneoxy-1,1-difluoro-2-(trans-4-((E)-1-propenyl)cyclohexyl)ethane
(Compound No. 232)
trans-4-(trans-4-ethylcyclohexyl)cyclohexylmethyleneoxy-1,1-difluoro-2-(trans-4-((E)-1-propenyl)cyclohexyl)ethane
(Compound No. 233)
trans-4-(trans-4-propylcyclohexyl)cyclohexylmethyleneoxy-1,1-difluoro-2-(trans-4-((E)-1-propenyl)cyclohexyl)ethane
(Compound No. 234)
trans-4-(trans-4-pentylcyclohexyl)cyclohexylmethyleneoxy-1,1-difluoro-2-(trans-4-((E)-1-propenyl)cyclohexyl)ethane

Following compounds can be prepared according to the preparation method described above with the exception that 4,4'-dicyclohexanedionemonoethyleneketal (45) is used in place of 1,4-cyclohexanedionemonoethyleneketal in the first step.

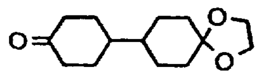

(45)

(Compound No. 235)
(trans-4-methylcyclohexyl)methyleneoxy-1,1-difluoro-2-(trans-4-(trans-4-ethenylcyclohexyl)cyclohexyl)ethane
(Compound No. 236)
(trans-4-ethylcyclohexyl)methyleneoxy-1,1-difluoro-2-(trans-4-(trans-4-ethenylcyclohexyl)cyclohexyl)ethane
(Compound No. 237)
(trans-4-propylcyclohexyl)methyleneoxy-1,2-difluoro-2-(trans-4-(trans-4-ethenylcyclohexyl)cyclohexyl)ethane
(Compound No. 238)
(trans-4-pentylcyclohexyl)methyleneoxy-2,1-difluoro-2-(trans-4-(trans-4-ethenylcyclohexyl)cyclohexyl)ethane
(Compound No. 239)
(trans-4-methylcyclohexyl)methyleneoxy-1,1-difluoro-2-(trans-4-(trans-4-((E)-1-propenyl)cyclohexyl)cyclohexyl) ethane
(Compound No. 240)
(trans-4-ethylcyclohexyl)methyleneoxy-1,1-difluoro-2-(trans-4-(trans-4-((E)-1-propenyl)cyclohexyl)cyclohexyl) ethane
(Compound No. 241)
(trans-4-propylcyclohexyl)methyleneoxy-1,1-difluoro-2-(trans-4-(trans-4-((E)-1-propenyl)cyclohexyl)cyclohexyl) ethane
(Compound No. 242)
(trans-4-pentylcyclohexyl)methyleneoxy-1,1-difluoro-2-(trans-4-(trans-4-((E)-1-propenyl)cyclohexyl)cyclohexyl) ethane

Following compounds can be prepared according to the preparation method described above with the exception that one of various benzyl alcohol derivatives is used in place of trans-4-propylcyclohexanecarbinol in the seventh step.

(Compound No. 243)
4-methylbenzyloxy-1,1-difluoro-2-(trans-4-ethenylcyclohexyl)ethane
(Compound No. 244)
4-ethylbenzyloxy-1,1-difluoro-2-(trans-4-ethenylcyclohexyl)ethane
(Compound No. 245)
4-propylbenzyloxy-1,1-difluoro-2-(trans-4-ethenylcyclohexyl)ethane
(Compound No. 246)
4-pentylbenzyloxy-1,1-difluoro-2-(trans-4-ethenylcyclohexyl)ethane
(Compound No. 247)
4-methylbenzyloxy-1,1-difluoro-2-(trans-4-((E)-1-propenyl)cyclohexyl)ethane
(Compound No. 248)
4-ethylbenzyloxy-1,1-difluoro-2-(trans-4-((E)-1-propenyl)cyclohexyl)ethane
(Compound No. 249)
4-propylbenzyloxy-1,1-difluoro-2-(trans-4-((E)-1-propenyl)cyclohexyl)ethane
(Compound No. 250)
4-pentylbenzyloxy-1,1-difluoro-2-(trans-4-((E)-1-propenyl)cyclohexyl)ethane
(Compound No. 251)
4-cyanobenzyloxy-1,1-difluoro-2-(trans-4-ethenylcyclohexyl)ethane
(Compound No. 252)
3-fluoro-4-cyanobenzyloxy-1,1-difluoro-2-(trans-4-ethenylcyclohexyl)ethane
(Compound No. 253)
3,5-difluoro-4-cyanobenzyloxy-1,1-difluoro-2-(trans-4-ethenylcyclohexyl)ethane
(Compound No. 254)
3,4-difluorobenzyloxy-1,1-difluoro-2-(trans-4-ethenylcyclohexyl)ethane

(Compound No. 255)
3,4,5-trifluorobenzyloxy-1,1-difluoro-2-(trans-4-ethenylcyclohexyl)ethane
(Compound No. 256)
4-trifluoromethoxybenzyloxy-1,1-difluoro-2-(trans-4-ethenylcycloheyxl)ethane
(Compound No. 257)
3-fluoro-4-trifluoromethoxybenzyloxy-1,1-difluoro-2-(trans-4-ethenylcyclohexyl)ethane
(Compound No. 258)
3,5-difluoro-4-trifluoromethoxybenzyloxy-1,1-difluoro-2-(trans-4-ethenylcyclohexyl)ethane
(Compound No. 259)
4-difluoromethoxybenzyloxy-1,1-difluoro-2-(trans-4-ethenylcyclohexyl)ethane
(Compound No. 260)
3-fluoro-4-difluoromethoxybenzyloxy-1,1-difluoro-2-(trans-4-ethenylcyclohexyl)ethane
(Compound No. 261)
3,5-difluoro-4-difluoromethoxybenzyloxy-1,1-difluoro-2-(trans-4-ethenylcyclohexyl)ethane
(Compound No. 262)
4-trifluoromethylbenzyloxy-1,1-difluoro-2-(trans-4-ethenylcyclohexyl)ethane
(Compound No. 263)
3-fluoro-4-trifluoromethylbenzyloxy-2,1-difluoro-2-(trans-4-ethenylcyclohexyl)ethane
(Compound No. 264)
3,5-difluoro-4-trifluoromethylbenzyloxy-1,1-difluoro-2-(trans-4-ethenylcyclohexyl)ethane
(Compound No. 265)
4-cyanobenzyloxy-1,1-difluoro-2-(trans-4-((E)-1-propenyl)cyclohexyl)ethane
(Compound No. 266)
3-fluoro-4-cyanobenzyloxy-1,1-difluoro-2-(trans-4-((E)-1-propenyl)cyclohexyl)ethane
(Compound No. 267)
3,5-difluoro-4-cyanobenzyloxy-1,1-difluoro-2-(trans-4-((E)-1-propenyl)cyclohexyl)ethane
(Compound No. 268)
3,4-difluorobenzyloxy-1,1-difluoro-2-(trans-4-((E)-1-propenyl)cyclohexyl)ethane
(Compound No. 269)
3,4,5-trifluorobenzyloxy-1,1-difluoro-2-(trans-4-((E)-1-propenyl)cyclohexyl)ethane
(Compound No. 270)
4-trifluoromethoxybenzyloxy-1,1-difluoro-2-(trans-4-((E)-1-propenyl)cyclohexyl)ethane
(Compound No. 271)
3-fluoro-4-trifluoromethoxybenzyloxy-1,1-difluoro-2-(trans-4-((E)-1-propenyl)cyclohexyl)ethane
(Compound No. 272)
3,5-difluoro-4-trifluoromethoxybenzyloxy-1,1-difluoro-2-(trans-4-((E)-1-propenyl)cyclohexyl)ethane
(Compound No. 273)
4-difluoromethoxybenzyloxy-1,1-difluoro-2-(trans-4-((E)-1-propenyl)cyclohexyl)ethane
(Compound No. 274)
3-fluoro-4-difluoromethoxybenzyloxy-1,1-difluoro-2-(trans-4-((E)-1-propenyl)cyclohexyl)ethane
(Compound No. 275)
3,5-difluoro-4-difluoromethoxybenzyloxy-1,1-difluoro-2-(trans-4-((E)-1-propenyl)cyclehexyl)ethane
(Compound No. 276)
4-trifluoromethylbenzyloxy-1,1-difluoro-2-(trans-4-ethenylcyclohexyl)ethane
(Compound No. 277)
3-fluoro-4-trifluoromethylbenzyloxy-1,1-difluoro-2-(trans-4-((E)-1-propenyl)cyclohexyl)ethane
(Compound No. 278)
3,5-difluoro-4-trifluoromethylbenzyloxy-1,1-difluoro-2-(trans-4-((E)-1-propenyl)cyclohexyl)ethane
(Compound No. 279)
4-(trans-4-methylcyclohexyl)benzyloxy-1,1-difluoro-2-(trans-4-ethenylcyclohexyl)ethane
(Compound No. 280)
4-(trans-4-ethylcyclohexyl)benzyloxy-1,1-difluoro-2-(trans-4-ethenylcyclohexyl)ethane
(Compound No. 281)
4-(trans-4-propylcyclohexyl)benzyloxy-1,1-difluoro-2-(trans-4-ethenylcyclohexyl)ethane
(Compound No. 282)
4-(trans-4-pentylcyclohexyl)benzyloxy-1,1-difluoro-2-(trans-4-ethenylcyclohexyl)ethane
(Compound No. 283)
4-(trans-4-methylcyclohexyl)benzyloxy-1,1-difluoro-2-(trans-4-((E)-1-propenyl)cyclohexyl)ethane

(Compound No. 284)
4-(trans-4-ethylcyclohexyl)benzyloxy-1,1-difluoro-2-(trans-4-((E)-1-propenyl)cyclohexyl)ethane
(Compound No. 285)
4-(trans-4-propylcyclohexyl)benzyloxy-1,1-difluoro-2-(trans-4-((E)-1-propenyl)cyclohexyl)ethane
(Compound No. 286)
4-(trans-4-pentylcyclohexyl)benzyloxy-1,1-difluoro-2-(trans-4-((E)-1-propenyl)cyclohexyl)ethane
(Compound No. 287)
4-(trans-4-ethenylcyclohexyl)benzyloxy-1,1-difluoro-2-(trans-4-ethenylcyclohexyl)ethane
(Compound No. 288)
4-(trans-4-ethenylcyclohexyl)benzyloxy-1,1-difluoro-2-(trans-4-((E)-1-propenyl)cyclohexyl)ethane
(Compound No. 289)
4-(trans-4-((E)-1-propenyl)cyclohexyl)benzyloxy-1,1-difluoro-2-(trans-4-ethenylcyclohexyl)ethane
(Compound No. 289)
4-(trans-4-((E)-1-propenyl)cyclohexyl)benzyloxy-1,1-difluoro-2-(trans-4-((E)-1-propenyl)cyclohexyl)ethane

Example 4

Preparation of (trans-4-methylcyclohexyl)methyleneoxy-1,1-difluoro-2-(trans-4-(3-butenyl)cyclohexyl)ethane (Compound expressed by the general formula (1) wherein m and n are 0, q and r are 1, ring B and ring C are 1,4-cyclohexylene group, o and p are 1, $R^1$ is 3-butenyl group, and $R^2$ is n-$C_3H_7$; Compound No. 291)

First step

In a 1000 ml, three-necked flask provided with a stirrer, thermometer, and nitrogen gas inlet tube, 69.6 g (156.9 mmol) of 2-(1,3-dioxane-2-yl)ethyltriphenylphosphonium bromide was suspended in 300 ml of THF under nitrogen gas atmosphere and cooled down to a temperature lower than -50°C in a dry ice-acetone bath, 18.5 g (164.8 mmol) of potassium-t-butoxide was added thereto, and the suspension was stirred while being maintained at the same temperature lower than -50°C for 2 hours to prepare an ylide. Then, 70 ml of solution of 25.0 g (130.8 mmol) of the compound (41) preparation method of which was shown in Example 3, step 3 in THF was added dropwise thereto at the same temperature in 20 min, stirred at the same temperature for 1 hour, warmed up to room temperature, and then further stirred for 4 hours. Water in an amount of 200 ml was added to the reaction solution to terminate the reaction, the THF layer was separated, and the water layer was extracted with 200 ml of toluene. The extract layer was washed with 400 ml of water and dried over anhydrous magnesium sulfate. The solvent was distilled off under a reduced pressure and the residue was concentrated. The concentrated residue was purified by column chromatography using a silica gel as filler and toluene as eluent to obtain 32.4 g of a colorless oily reaction product. This product was exo-methylene derivative (46).

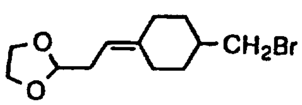

(46)

Second step

In a 500 ml, eggplant type flask, 32.4 g (117.7 mmol) of the exo-methylene derivative (46) obtained by the procedures described above was dissolved in 300 ml of mixed solvent of toluene/ethanol (1/1), 1.6 g of 5 % palladium carbon catalyst was added thereto, and the solution was stirred at room temperature under a condition of hydrogen gas pressure of 1-2 kg/cm2 for 8 hours. The catalyst was separated from the reaction solution by filtration and the reaction solution was concentrated under a reduced pressure to obtain 30.0 g of a reaction product. The reaction product was dissolved in 100 ml of toluene in a 500 ml, eggplant type flask, 12.6 g (270.7 mmol) of 99 % formic acid was added thereto, and the solution was heated to reflux for 8 hours. Water in an amount of 100 ml was added to the reaction solution and then extracted with 300 ml of toluene. The extract layer was washed with 300 ml of water, 200 ml of saturated aqueous sodium bicarbonate solution, and 300 ml of water in turn, and dried over anhydrous magnesium sulfate. The solvent was distilled off under a reduced pressure and then the residue was concentrated. The concentrated residue was purified by column chromatography using a silica gel as filler and toluene as eluent to obtain 21.2 g of a colorless oily reaction product. This product was aldehyde derivative (47).

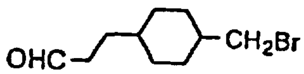

(47)

Third step

In a 500 ml, three-necked flask provided with a stirrer, thermometer, and nitrogen gas inlet tube, 38.9 g (109.2 mmol) of methyltriphenylphosphonium bromide was suspended in 100 ml of THF under nitrogen gas atmosphere and cooled down to a temperature lower than -50°C in a dry ice-acetone bath, 12.9 g (114.7 mmol) of potassium-t-butoxide was added to thereto, and the suspension was stirred while being maintained at the same temperature of lower than -50°C for 2 hours to prepare an ylide. Then, 60 ml of solution of 21.2 g (90.9 mmol) of the aldehyde derivative (47) obtained by the procedures described above in THF was added dropwise thereto at the same temperature in 10 min, stirred at the same temperature for 1 hour, warmed up to room temperature, and then further stirred at room temperature for 4 hours. Water in an amount of 100 ml was added to the reaction solution to terminate the reaction, the THF layer was separated, and the water layer was extracted with 200 ml of toluene. The extract layer was washed with 300 ml of water and dried over anhydrous magnesium sulfate. The solvent was distilled off under a reduced pressure and the residue was concentrated. The concentrated residue was purified by column chromatography using a silica gel as filler and a mixed solvent of toluene-heptane to obtain 16.5 g of a colorless oily reaction product. This product was 4-(3-butenyl)cyclohexylmethylene bromide (48).

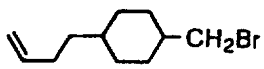

(48)

Fourth step

From 4-(3-butenyl)cyclohexylmethylene bromide (48) and trans-4-methylcyclohexanecarbinol, 2.5 g of the objective (trans-4-methylcyclohexyl)methyleneoxy-1,1-difluoro-2-(trans-4-(3-butenyl)cyclohexyl)ethane was obtained according to the method described in Example 3. The results of determination of various kind of spectra well supported its structure.
GC-MS: $M^+$ 356

Following compounds can be prepared according to the preparation method described above with the exception that a trans-4-alkyl-cyclohexanecarbinol having an alkyl chain different from methyl is used in place of trans-4-methylcyclohexanecarbinol.

(Compound No. 292)
(trans-4-ethylcyclohexyl)methyleneoxy-1,1-difluoro-2-(trans-4-(3-butenyl)cyclohexyl)ethane
(Compound No. 293)
(trans-4-propylcyclohexyl)methyleneoxy-1,1-difluoro-2-(trans-4-(3-butenyl)cyclohexyl)ethane
(Compound No. 294)
(trans-4-butylcyclohexyl)methyleneoxy-1,1-difluoro-2-(trans-4-(3-butenyl)cyclohexyl)ethane
(Compound No. 295)
(trans-4-pentylcyclohexyl)methyleneoxy-1,1-difluoro-2-(trans-4-(3-butenyl)cyclohexyl)ethane
(Compound No. 296)
(trans-4-hexylcyclohexyl)methyleneoxy-1,1-difluoro-2-(trans-4-(3-butenyl)cyclohexyl)ethane
(Compound No. 297)
(trans-4-heptylcyclohexyl)methyleneoxy-1,1-difluoro-2-(trans-4-(3-butenyl)cyclohexyl)ethane

Following compounds can be prepared according to the preparation method described above with the exception that ethyltriphenylphosphonium iodide is used in place of methyltriphenylphosphonium iodide used in the third step, and the product is isomerized by reacting with benzenesulfinic acid or p-toluenesulfinic acid according to a method described in Japanese Patent Publication No. Hei 4-30382 or the product is subjected to steric inversion of olefin according to a method described in Japanese Patent Publication No. Hei 6-62462.

(Compound No. 298)
(trans-4-methylcyclohexyl)methyleneoxy-1,1-difluoro-2-(trans-4-((E)-3-pentenyl)cyclohexyl)ethane

(Compound No. 299)
(trans-4-ethylcyclohexyl)methyleneoxy-1,1-difluoro-2-(trans-4-((E)-3-pentenyl)cyclohexyl)ethane
(Compound No. 300)
(trans-4-propylcyclohexyl)methyleneoxy-1,1-difluoro-2-(trans-4-((E)-3-pentenyl)cyclohexyl)ethane
(Compound No. 301)
(trans-4-butylcyclohexyl)methyleneoxy-1,1-difluoro-2-(trans-4-((E)-3-pentenyl)cyclohexyl)ethane
(Compound No. 302)
(trans-4-pentylcyclohexyl)methyleneoxy-1,1-difluoro-2-(trans-4-((E)-3-pentenyl)cyclohexyl)ethane
(Compound No. 303)
(trans-4-heptylcyclohexyl)methyleneoxy-1,1-difluoro-2-(trans-9-((E)-3-pentenyl)cyclohexyl)ethane

Following compounds can be prepared according to the preparation method described above with the exception that one of other various benzyl alcohol derivatives is used in place of trans-4-methylcyclohexane carbinol used in the fourth step.

(Compound No. 304)
4-methylbenzyloxy-1,1-difluoro-2-(trans-4-(3-butenyl)cyclohexyl)ethane
(Compound No. 305)
4-ethylbenzyloxy-1,1-difluoro-2-(trans-4-(3-butenyl)cyclohexyl)ethane
(Compound No. 306)
4-propylbenzyloxy-1,1-difluoro-2-(trans-4-(3-butenyl)cyclohexyl)ethane
(Compound No. 307)
4-pentylbenzyloxy-1,1-difluoro-2-(trans-4-(3-butenyl)cyclohexyl)ethane
(Compound No. 308)
4-heptylbenzyloxy-1,1-difluoro-2-(trans-4-(3-butenyl)cyclohexyl)ethane
(Compound No. 309)
4-methylbenzyloxy-1,1-difluoro-2-(trans-4-((E)-3-pentenyl)cyclohexyl)ethane
(Compound No. 310)
4-ethylbenzyloxy-1,1-difluoro-2-(trans-4-((E)-3-pentenyl)cyclohexyl)ethane
(Compound No. 311)
4-propylbenzyloxy-1,1-difluoro-2-(trans-4-((E)-3-pentenyl)cyclohexyl)ethane
(Compound No. 312)
4-pentylbenzyloxy-1,1-difluoro-2-(trans-4-((E)-3-pentenyl)cyclohexyl)ethane
(Compound No. 313)
4-heptylbenzyloxy-1,1-difluoro-2-(trans-4-((E)-3-pentenyl)cyclohexyl)ethane
(Compound No. 314)
4-vinylbenzyloxy-1,1-difluoro-2-(trans-4-(3-butenyl)cyclohexyl)ethane
(Compound No. 315)
4-vinylbenzyloxy-1,1-difluoro-2-(trans-4-((E)-3-pentenyl)cyclohexyl)ethane
(Compound No. 316)
4-((E)-1-propenyl)benzyloxy-1,1-difluoro-2-(trans-4-(3-butenyl)cyclohexyl)ethane
(Compound No. 317)
4-((E)-1-propenyl)benzyloxy-1,1-difluoro-2-(trans-4-(((E)-3-pentenyl)cyclohexyl)ethane
(Compound No. 318)
4-(3-butenyl)benzyloxy-1,1-difluoro-2-(trans-4-(3-butenyl)cyclohexyl)ethane
(Compound No. 319)
4-(3-butenyl)benzyloxy-1,1-difluoro-2-(trans-4-((E)-3-pentenyl)cyclohexyl)ethane
(Compound No. 320)
4-((E)-3-pentenyl)benzyloxy-1,1-difluoro-Z-(trans-4-(3-butenyl)cyclohexyl) ethane
(Compound No. 321)
4-((E)-3-pentenyl)benzyloxy-1,1-difluoro-2-(trans-4-((E)-3-pentenyl)cyclohexyl)ethane
(Compound No. 322)
4-cyanobenzyloxy-1,1-difluoro-2-(trans-4-(3-butenyl)cyclohexyl)ethane
(Compound No. 323)
4-cyanobenzyloxy-1,1-difluoro-2-(trans-4-((E)-3-pentenyl)cyclohexyl)ethane
(Compound No. 324)
3-fluoro-4-cyanobenzyloxy-1,1-difluoro-2-(trans-4-(3-butenyl)cyclohexyl)ethane
(Compound No. 324)

3-fluoro-4-cyanobenzyloxy-1,1-difluoro-2-(trans-4-(3-butenyl)cyclohexyl)ethane
(Compound No. 325)
3-fluoro-4-cyanobenzyloxy-1,1-difluoro-2-(trans-4-((E)-3-pentenyl)cyclohexyl)ethane
(Compound No. 326)
3,5-difluoro-4-cyanobenzyloxy-1,1-difluoro-2-(trans-4-(3-butenyl)cyclohexyl)ethane
(Compound No. 327)
3,5-difluoro-4-cyanobenzyloxy-1,1-difluoro-2-(trans-4-((E)-3-pentenyl)cyclohexyl)ethane
(Compound No. 328)
4-fluorobenzyloxy-1,1-difluoro-2-(trans-4-(3-butenyl)cyclohexyl)ethane
(Compound No. 329)
4-fluorobenzyloxy-1,1-difluoro-2-(trans-4-((E)-3-pentenyl)cyclohexyl)ethane
(Compound No. 330)
3,4-difluorobenzyloxy-1,1-difluoro-2-(trans-4-(3-butenyl)cyclohexyl)ethane
(Compound No. 331)
3,4-difluorobenzyloxy-1,2-difluoro-2-(trans-4-((E)-3-pentenyl)cyclohexyl)ethane
(Compound No. 332)
3,4,5-trifluorobenzyloxy-1,1-difluoro-2-(trans-4-(3-butenyl)cyclohexyl)ethane
(Compound No. 333)
3,4,5-trifluorobenzyloxy-1,1-difluoro-2-(trans-4-((E)-3-pentenyl)cyclohexyl)ethane
(Compound No. 334)
4-trifluoromethoxybenzyloxy-1,1-difluoro-2-(trans-4-(3-butenyl)cyclohexyl)ethane
(Compound No. 335)
4-trifluoromethoxybenzyloxy-1,1-difluoro-2-(trans-4-((E)-3-pentenyl)cyclohexyl)ethane
(Compound No. 336)
3-fluoro-4-trifluoromethoxybenzyloxy-1,1-difluoro-2-(trans-4-(3-butenyl)cyclohexyl)ethane
(Compound No. 337)
3-fluoro-4-trifluoromethoxybenzyloxy-1,1-difluoro-2-(trans-4-((E)-3-pentenyl)cyclohexyl)ethane
(Compound No. 338)
3,5-difluoro-4-trifluoromethoxybenzyloxy-1,1-difluoro-2-(trans-4-(3-butenyl)cyclohexyl)ethane
(Compound No. 339)
3,5-difluoro-4-trifluoromethoxybenzyloxy-1,1-difluoro-2-(trans-4-((E)-3-pentenyl)cyclohexyl)ethane
(Compound No. 340)
4-difluoromethoxybenzyloxy-1,1-difluoro-2-(trans-4-(3-butenyl)cyclohexyl)ethane
(Compound No. 341)
4-difluoromethoxybenzyloxy-1,2-difluoro-2-(trans-4-((E)-3-pentenyl)cyclohexyl)ethane
(Compound No. 342)
3-fluoro-4-difluoromethoxybenzyloxy-1,1-difluoro-2-(trans-4-(3-butenyl)cyclohexyl)ethane
(Compound No. 343)
3-fluoro-4-difluoromethoxybenzyloxy-1,1-difluoro-2-(trans-4-((E)-3-pentenyl)cyclohexyl)ethane
(Compound No. 344)
3,5-difluoro-4-difluoromethoxybenzyloxy-1,1-difluoro-2-(trans-4-(3-butenyl)cyclohexyl)ethane
(Compound No. 345)
3,5-difluoro-4-difluoromethoxybenzyloxy-1,1-difluoro-2-(trans-4-((E)-3-pentenyl)cyclohexyl)ethane
(Compound No. 346)
4-trifluoromethylbenzyloxy-1,1-difluoro-2-(trans-4-(3-butenyl)cyclohexyl)ethane
(Compound No. 347)
4-trifluoromethylbenzyloxy-1,1-difluoro-2-(trans-4-((E)-3-pentenyl)cyclohexyl)ethane
(Compound No. 348)
3-fluoro-4-trifluoromethylbenzyloxy-1,1-difluoro-2-(trans-4-(3-butenyl)cyclohexyl)ethane
(Compound No. 349)
3-fluoro-4-trifluoromethylbenzyloxy-1,1-difluoro-2-(trans-4-((E)-3-pentenyl)cyclohexyl)ethane
(Compound No. 350)
3,5-difluoro-4-trifluoromethylbenzyloxy-1,1-difluoro-2-(trans-4-(3-butenyl)cyclohexyl)ethane
(Compound No. 351)
3,5-difluoro-4-trifluoromethylbenzyloxy-1,1-difluoro-2-(trans-4-((E)-3-pentenyl)cyclohexyl)ethane

Example 5

Preparation of α,α-difluoro-4-(trans-4-ethenylcyclohexyl)-benzyloxy-2-(4-(trans-4-propylcyclohexyl)phenyl) ethane (Compounds expressed by the general formula (1) wherein m, n, q, and r are 1, ring B and ring C are 1,4-phenylene group, ring A and ring D are trans-1,4-cyclohexylene group, o is 0, p is 2, $R^1$ is ethenyl group, and $R^2$ is n-$C_3H_7$; Compound No. 354)

First step

In a 1000 ml, three-necked flask provided with a stirrer, thermometer, dropping funnel, and nitrogen gas inlet tube, 30.0 g (172.2 mmol) of 4-phenylcyclohexanone was dissolved in 240 ml of chloroform while being stirred under nitrogen gas atmosphere and cooled down to a temperature lower than -20°C in a dry ice-acetone bath, and then 35.8 g (223.8 mmol) of bromine was added dropwise while being maintained at the same temperature lower than -20°C in 20 min. Then, the solution was stirred at the same temperature for 30 min, warmed up to room temperature, and then further stirred at room temperature for 4 hours. The reaction solution was cooled again down to a temperature lower than -20°C, and then 200 ml of water was added thereto to terminate the reaction. The chloroform layer was separated from the reaction solution and then the water layer was further extracted with 200 ml of chloroform. The extract.layer was washed with 400 ml of water, 100 ml of saturated aqueous sodium bicarbonate solution, and 400 ml of water in turn, and then dried over anhydrous magnesium sulfate. The reaction solution was concentrated under a reduced pressure, and the concentrated residue was purified by column chromatography using a silica gel as filler and a mixed solvent of toluene-heptane as eluent to obtain 37.0 g of a colorless crystal product. This product was 4-(4-bromophenyl) cyclohexanone.

Second step

In a 1000 ml, three-necked flask provided with a stirrer, thermometer, dropping funnel, and nitrogen gas inlet tube, 60.2 g (175.7 mmol) of methoxymethyltriphenylphosphonium chloride was suspended in 200 ml of THF under nitrogen gas atmosphere and cooled down to a temperature lower than -50°C in a dry ice-acetone bath, 20.7 g (184.5 mmol) of potassium-t-butoxide was added thereto, and then the suspension was stirred while being maintained at the same temperature lower than -50°C for 2 hours to prepare an ylide. Then, 80 ml of solution of 37.0 g (131.8 mmol) of the 4-(4-bromophenyl)cyclohexanone obtained by the procedures described above in THF was added dropwise thereto at the same temperature in 15 min, stirred at the same temperature for 1 hour, warmed up to room temperature, and then further stirred at room temperature for 4 hours. Water in an amount of 300 ml was added to the reaction solution to terminate the reaction, the THF layer was separated, and the water layer was extracted with 200 ml of toluene. The extract layer was washed with 500 ml of water and then dried over anhydrous magnesium sulfate. The solvent was distilled off under a reduced pressure and the residue was concentrated. The concentrated residue was purified by column chromatography using a silica gel as filler and a mixed solvent of toluene-heptane as eluent to obtain 37.0 g of a colorless crystalline reaction product. Then, the reaction product was dissolved in 100 ml of toluene in a 500 ml, eggplant type flask, 15.3 g (329.4 mmol) of 99 % formic acid was added thereto, and the solution was heated to reflux for 2 hours. Water in an amount of 100 ml was added to the reaction solution, the toluene layer was separated, and then the water layer was extracted with 200 ml of toluene. The extract layer was washed with 300 ml of water, 100 ml of saturated aqueous sodium bicarbonate solution, and 300 ml of water in turn, and then dried over anhydrous magnesium sulfate. The solvent was distilled off under a reduced pressure and the residue was concentrated. The concentrated residue was purified by column chromatography using a silica gel as filler and toluene as eluent to obtain 31.9 g of a colorless crystalline reaction product. This product was 4-(4-bromophenyl)cyclohexane-carbaldehyde.

Third step

In a 500 ml, three-necked flask provided with a stirrer, thermometer, dropping funnel, and nitrogen gas inlet tube, 51.4 g (143.9 mmol) of methyltriphenylphosphonium bromide was suspended in 200 ml of THF under nitrogen gas atmosphere and cooled down to a temperature lower than -50°C in a dry ice-acetone bath, 16.9 g (151.1 mmol) of potassium-t-butoxide was added thereto, and then the suspension was stirred while being maintained at the same temperature lower than -50°C for 2 hours to prepare an ylide. Then, 80 ml of solution of 31.9 g (119.9 mmol) of the 4-(4-bromophenyl)cyclohexanecarbaldehyde obtained by the procedures described above in THF was added dropwise thereto at the same temperature in 10 min, stirred at the same temperature for 1 hour, warmed up to room temperature, and then further stirred at room temperature for 4 hours. Water in an amount of 200 ml was added to the reaction solution to terminate the reaction, the THF layer was separated, and the water layer was extracted with 200 ml of toluene. The extract layer was washed with 400 ml of water and dried over anhydrous magnesium sulfate. The solvent

was distilled off under a reduced pressure and the residue was concentrated. The concentrated residue was purified by column chromatography using a silica gel as filler and heptane as eluent, and recrystallized from a mixed solvent of toluene-heptane to obtain 19.2 g of a colorless crystal product. This product was 4-(trans-4-ethenylcyclohexyl)-bromobenzene (49).

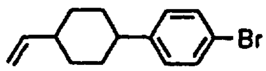

(49)

Fourth step

From the 4-(trans-4-ethenylcyclohexyl)bromobenzene (49) obtained by the procedures described above and 2-(4-(trans-4-propylcyclohexyl)phenyl)ethanol, 2.5 g of the objective α,α-difluoro-4-(trans-4-ethenylcyclohexyl)benzyloxy-2-(4-(trans-4-propylcyclohexyl)phenyl)ethane was obtained according to the preparation method shown in Example 1. Results of determination of various kind of spectra well supported its structure.
GC-MS: $M^+$ 480

Following compounds can be prepared according to the preparation method described above with the exception that a 2-(4-(trans-4-alkylcyclohexyl)phenyl)ethanol having an alkyl chain different from propyl is used in place of 2-(4-(trans-4-propylcyclohexyl)phenyl)ethanol.

(Compound No. 352)
α,α-difluoro-4-(trans-4-ethenylcyclohexyl)benzyloxy-2-(4-(trans-4-methylcyclohexyl)phenyl)ethane
(Compound No. 353)
α,α-difluoro-4-(trans-4-ethenylcyclohexyl)benzyloxy-2-(4-(trans-4-ethylcyclohexyl)phenyl)ethane
(Compound No. 355)
α,α-difluoro-4-(trans-4-ethenylcyclohexyl)benzyloxy-2-(4-(trans-4-butylcyclohexyl)phenyl)ethane
(Compound No. 356)
α,α-difluoro-4-(trans-4-ethenylcyclohexyl)benzyloxy-2-(4-(trans-4-pentylcyclohexyl)phenyl)ethane
(Compound No. 357)
α,α-difluoro-4-(trans-4-ethenylcyclohexyl)benzyloxy-2-(4-(trans-4-hexylcyclohexyl)phenyl)ethane
(Compound No. 358)
α,α-difluoro-4-(trans-4-ethenylcyclohexyl)benzyloxy-2-(4-(trans-4-heptylcyclohexyl)phenyl)ethane

Following compounds can be prepared according to the preparation method described above with the exception that ethyltriphenylphosphonium iodide is used in place of methyltriphenylphosphonium used in the third step, and the product is isomerized by reacting with benzenesulfinic acid or p-toluenesulfinic acid according to a method described in Japanese Patent Publication No. Hei 4-30382 or the product is subjected to steric inversion of olefin according to a method described in Japanese Patent Publication No. Hei 6-62462.

(Compound No. 359)
α,α-difluoro-4-(trans-4((E)-1-propenyl)cyclohexyl)benzyloxy-2-(4-(trans-4-methylcyclohexyl)phenyl)ethane
(Compound No. 360)
α,α-difluoro-4-(trans-4((E)-1-propenyl)cyclohexyl)benzyloxy-2-(4-(trans-4-ethylcyclohexyl)phenyl)ethane
(Compound No. 361)
α,α-difluoro-4-(trans-4((E)-1-propenyl)cyclohexyl)benzyloxy-2-(4-(trans-4-propylcyclohexyl)phenyl)ethane
(Compound No. 362)
α,α-difluoro-4-(trans-4((E)-1-propenyl)cyclohexyl)benzyloxy-2-(4-(trans-4-pentylcyclohexyl)phenyl)ethane
(Compound No. 363)
α,α-difluoro-4-(trans-4((E)-1-propenyl)cyclohexyl)benzyloxy-2-(4-(trans-4-heptylcyclohexyl)phenyl)ethane

Following compounds can be prepared according to the preparation method described above and the method described in Example 4 with the exception that 2-(1,3-dioxane-2-yl)ethyltriphenylphosphonium bromide is used in place of methoxymethyltriphenylphosphonium chloride used in the second step.

(Compound No. 364)
α,α-difluoro-4-(trans-4(3-butenyl)cyclohexyl)benzyloxy-2-(4-(trans-4-methylcyclohexyl)phenyl)ethane
(Compound No. 365)

α,α-difluoro-4-(trans-4(3-butenyl)cyclohexyl)benzyloxy-2-(4-(trans-4-ethylcyclohexyl)phenyl)ethane
(Compound No. 366)
α,α-difluoro-4-(trans-4(3-butenyl)cyclohexyl)benzyloxy-2-(4-(trans-4-propylcyclohexyl)phenyl)ethane
(Compound No. 367)
α,α-difluoro-4-(trans-4(3-butenyl)cyclohexyl)benzyloxy-2-(4-(trans-4-pentylcyclohexyl)phenyl)ethane
(Compound No. 368)
α,α-difluoro-4-(trans-4(3-butenyl)cyclohexyl)benzyloxy-2-(4-(trans-4-heptylcyclohexyl)phenyl)ethane
(Compound No. 369)
α,α-difluoro-4-(trans-4((E)-3-pentenyl)cyclohexyl)benzyloxy-2-(4-(trans-4-methylcyclohexyl)phenyl)ethane
(Compound No. 370)
α,α-difluoro-4-(trans-4((E)-3-pentenyl)cyclohexyl)benzyloxy-2-(4-(trans-4-ethylcyclohexyl)phenyl)ethane
(Compound No. 371)
α,α-difluoro-4-(trans-4((E)-3-pentenyl)cyclohexyl)benzyloxy-2-(4-(trans-4-propylcyclohexyl)phenyl)ethane
(Compound No. 372)
α,α-difluoro-4-(trans-4((E)-3-pentenyl)cyclohexyl)benzyloxy-2-(4-(trans-4-pentylcyclohexyl)phenyl)ethane
(Compound No. 373)
α,α-difluoro-4-(trans-4((E)-3-pentenyl)cyclohexyl)benzyloxy-2-(4-(trans-4-heptylcyclohexyl)phenyl)ethane

Following compounds can be prepared according to the preparation method described above with the exception that one of known ethanol derivatives is used.

(Compound No. 374)
α,α-difluoro-4-(trans-4-ethenylcyclohexyl)benzyloxy-2-(4-trifluoromethoxyphenyl)ethane
(Compound No. 375)
α,α-difluoro-4-(trans-4-((E)-1-propenyl)cyclohexyl)benzyloxy-2-(4-trifluoromethoxyphenyl)ethane
(Compound No. 376)
α,α-difluoro-4-(trans-4-(3-butenyl)cyclohexyl)benzyloxy-2-(4-trifluoromethoxyphenyl)ethane
(Compound No. 377)
α,α-difluoro-4-(trans-4-((E)-3-pentenyl)cyclohexyl)benzyloxy-2-(4-trifluoromethoxyphenyl)ethane
(Compound No. 378)
α,α-difluoro-4-(trans-4-ethenylcyclohexyl)benzyloxy-2-(3-fluoro-4-trifluoromethoxyphenyl)ethane
(Compound No. 379)
α,α-difluoro-4-(trans-4-((E)-1-propenyl)cyclohexyl)benzyloxy-2-(3-fluoro-4-trifluoromethoxyphenyl)ethane
(Compound No. 380)
α,α-difluoro-4-(trans-4-(3-butenyl)cyclohexyl)benzyloxy-2-(3-fluoro-4-trifluoromethoxyphenyl)ethane
(Compound No. 381)
α,α-difluoro-4-(trans-4-((E)-3-pentenyl)cyclohexyl)benzyloxy-2-(3-fluoro-4-trifluoromethoxyphenyl)ethane
(Compound No. 382)
α,α-difluoro-4-(trans-4-(ethenylcyclohexyl)benzyloxy-2-(3,5-difluoro-4-trifluoromethoxyphenyl)ethane
(Compound No. 383)
α,α-difluoro-4-(trans-4-((E)-1-propenyl)cyclohexyl)benzyloxy-2-(3,5-difluoro-4-trifluoromethoxyphenyl)ethane
(Compound No. 384)
α,α-difluoro-4-(trans-4-(3-butenyl)cyclohexyl)benzyloxy-2-(3,5-difluoro-4-trifluoromethoxyphenyl)ethane
(Compound No. 385)
α,α-difluoro-4-(trans-4-((E)-3-pentenyl)cyclohexyl)benzyloxy-2-(3,5-difluoro-4-trifluoromethoxyphenyl)ethane
(Compound No. 386)
α,α-difluoro-4-(trans-4-ethenylcyclohexyl)benzyloxy-2-(4-cyanophenyl)ethane
(Compound No. 387)
α,α-difluoro-4-(trans-4-((E)-1-propenyl)cyclohexyl)benzyloxy-2-(4-cyanophenyl)ethane
(Compound No. 388)
α,α-difluoro-4-(trans-4-(3-butenyl)cyclohexyl)benzyloxy-2-(4-cyanophenyl)ethane
(Compound No. 389)
α,α-difluoro-4-(trans-4-((E)-3-pentenyl)cyclohexyl)benzyloxy-2-(4-cyanophenyl)ethane
(Compound No. 390)
α,α-difluoro-4-(trans-4-ethenylcyclohexyl)benzyloxy-2-(3-fluoro-4-cyanophenyl)ethane
(Compound No. 391)
α,α-difluoro-4-(trans-4-((E)-1-propenyl)cyclohexyl)benzyloxy-2-(3-fluoro-4-cyanophenyl)ethane
(Compound No. 392)

α,α-difluoro-4-(trans-4-(3-butenyl)cyclohexyl)benzyloxy-2-(3-fluoro-4-cyanophenyl)ethane (Compound No. 393)
α,α-difluoro-4-(trans-4-((E)-3-pentenyl)cyclohexyl)benzyloxy-2-(3-fluoro-4-cyanophenyl)ethane (Compound No. 394)
α,α-difluoro-4-(trans-4-ethenylcyclohexyl)benzyloxy-2-(3,5-difluoro-4-cyanophenyl)ethane (Compound No. 395)
α,α-difluoro-4-(trans-4-((E)-1-propenyl)cyclohexyl)benzyloxy-2-(3,5-difluoro-4-cyanophenyl)ethane (Compound No. 396)
α,α-difluoro-4-(trans-4-(3-butenyl)cyclohexyl)benzyloxy-2-(3,5-difluoro-4-cyanophenyl)ethane (Compound No. 397)
α,α-difluoro-4-(trans-4-((E)-3-pentenyl)cyclohexyl)benzyloxy-2-(3,5-difluoro-4-cyanophenyl)ethane (Compound No. 398)
α,α-difluoro-4-(trans-4-ethenylcyclohexyl)benzyloxy-2-(4-trifluoromethylphenyl)ethane (Compound No. 399)
α,α-difluoro-4-(trans-4-((E)-1-propenyl)cyclohexyl)benzyloxy-2-(4-trifluoromethylphenyl)ethane (Compound No. 400)
α,α-difluoro-4-(trans-4-(3-butenyl)cyclohexyl)benzyloxy-2-(4-trifluoromethylphenyl)ethane (Compound No. 401)
α,α-difluoro-4-(trans-4-((E)-3-pentenyl)cyclohexyl)benzyloxy-2-(4-trifluoromethylphenyl)ethane (Compound No. 402)
α,α-difluoro-4-(trans-4-ethenylcyclohexyl)benzyloxy-2-(3-fluoro-4-trifluoromethylphenyl)ethane (Compound No. 403)
α,α-difluoro-4-(trans-4-((E)-1-propenyl)cyclohexyl)benzyloxy-2-(3-fluoro-4-trifluoromethylphenyl)ethane (Compound No. 404)
α,α-difluoro-4-(trans-4-(3-butenyl)cyclohexyl)benzyloxy-2-(3-fluoro-4-trifluoromethylphenyl)ethane (Compound No. 405)
α,α-difluoro-4-(trans-4-((E)-3-pentenyl)cyclohexyl)benzyloxy-2-(3-fluoro-4-trifluoromethylphenyl)ethane (Compound No. 406)
α,α-difluoro-4-(trans-4-ethenylcyclohexyl)benzyloxy-2-(3,5-difluoro-4-trifluoromethylphenyl)ethane (Compound No. 407)
α,α-difluoro-4-(trans-4-((E)-1-propenyl)cyclohexyl)benzyloxy-2-(3,5-difluoro-4-trifluoromethylphenyl)ethane (Compound No. 408)
α,α-difluoro-4-(trans-4-(3-butenyl)cyclohexyl)benzyloxy-2-(3,5-difluoro-4-trifluoromethylphenyl)ethane (Compound No. 409)
α,α-difluoro-4-(trans-4-((E)-3-pentenyl)cyclohexyl)benzyloxy-2-(3,5-difluoro-4-trifluoromethylphenyl)ethane (Compound No. 410)
α,α-difluoro-4-(trans-4-ethenylcyclohexyl)benzyloxy-2-(4-(3-butenyl)phenyl)ethane (Compound No. 411)
α,α-difluoro-4-(trans-4-ethenylcyclohexyl)benzyloxy-2-(4-((E)-3-pentenyl)phenyl)ethane (Compound No. 412)
α,α-difluoro-4-(trans-4-((E)-1-propenyl)cyclohexyl)benzyloxy-2-(4-(3-butenyl)phenyl)ethane (Compound No. 413)
α,α-difluoro-4-(trans-4-((E)-1-propenyl)cyclohexyl)benzyloxy-2-(4-((E)-3-pentenyl)phenyl)ethane (Compound No. 414)
α,α-difluoro-4-(trans-4-(3-butenyl)cyclohexyl)benzyloxy-2-(4-(3-butenyl)phenyl)ethane (Compound No. 415)
α,α-difluoro-4-(trans-4-(3-butenyl)cyclohexyl)benzyloxy-2-(4-((E)-3-pentenyl)phenyl)ethane (Compound No. 416)
α,α-difluoro-4-(trans-4-((E)-3-pentenyl)cyclohexyl)benzyloxy-2-(4-(3-butenyl)phenyl)ethane (Compound No. 417)
α,α-difluoro-4-(trans-4-((E)-3-pentenyl)cyclohexyl)benzyloxy-2-(4-((E)-3-pentenyl)phenyl)ethane (Compound No. 418)
α,α-difluoro-2-fluoro-4-(trans-4-ethenylcyclohexyl)benzyloxy-2-(4-(3-butenyl)phenyl)ethane (Compound No. 419)
α,α-difluoro-2-fluoro-4-(trans-4-ethenylcyclohexyl)benzyloxy-2-(4-((E)-3-pentenyl)phenyl)ethane (Compound No. 420)
α,α-difluoro-2-fluoro-4-(trans-4-((E)-1-propenyl)cyclohexyl)benzyloxy-2-(4-(3-butenyl)phenyl)ethane (Compound No. 421)

α,α-difluoro-2-fluoro-4-(trans-4-((E)-1-propenyl)cyclohexyl)benzyloxy-2-(4-((E)-3-pentenyl)phenyl)ethane
(Compound No. 422)
α,α-difluoro-2-fluoro-4-(trans-4-(3-butenyl)cyclohexyl)benzyloxy-2-(4-(3-butenyl)phenyl)ethane
(Compound No. 423)
α,α-difluoro-2-fluoro-4-(trans-4-(3-butenyl)cyclohexyl)benzyloxy-2-(4-((E)-3-pentenyl)phenyl)ethane
(Compound No. 424)
α,α-difluoro-2-fluoro-4-(trans-4-((E)-3-pentenyl)cyclohexyl)benzyloxy-2-(4-(3-butenyl)phenyl)ethane
(Compound No. 425)
α,α-difluoro-2-fluoro-4-(trans-4-((E)-3-pentenyl)cyclohexyl)benzyloxy-2-(4-((E)-3-pentenyl)phenyl)ethane
(Compound No. 426)
α,α-difluoro-2,6-difluoro-4-(trans-4-ethenylcyclohexyl)benzyloxy-2-(4-(3-butenyl)phenyl)ethane
(Compound No. 427)
α,α-difluoro-2,6-difluoro-4-(trans-4-ethenylcyclohexyl)benzyloxy-2-(4-((E)-3-pentenyl)phenyl)ethane
(Compound No. 428)
α,α-difluoro-2,6-difluoro-4-(trans-4-((E)-1-propenyl)cyclohexyl)benzyloxy-2-(4-(3-butenyl)phenyl)ethane
(Compound No. 429)
α,α-difluoro-2,6-difluoro-4-(trans-4-((E)-1-propenyl)cyclohexyl)benzyloxy-2-(4-((E)-3-pentenyl)phenyl) ethane
(Compound No. 430)
α,α-difluoro-2,6-difluoro-4-(trans-4-(3-butenyl)cyclohexyl)benzyloxy-2-(4-(3-butenyl)phenyl)ethane
(Compound No. 431)
α,α-difluoro-2,6-difluoro-4-(trans-4-(3-butenyl)cyclohexyl)benzyloxy-2-(4-((E)-3-pentenyl)phenyl)ethane
(Compound No. 432)
α,α-difluoro-2,6-difluoro-4-(trans-4-((E)-3-pentenyl)cyclohexyl)benzyloxy-2-(4-(3-butenyl)phenyl)ethane
(Compound No. 433)
α,α-difluoro-2,6-difluoro-4-(trans-4-((E)-3-pentenyl)cyclohexyl)benzyloxy-2-(4-((E)-3-pentenyl)phenyl)ethane
(Compound No. 434)
α,α-difluoro-4-(trans-4-ethenylcyclohexyl)benzyloxy-2-(4-(trans-4-ethenylcyclohexyl)phenyl)ethane
(Compound No. 435)
α,α-difluoro-4-(trans-4-ethenylcyclohexyl)benzyloxy-2-(4-(trans-4-((E)-1-propenyl)cyclohexyl)phenyl)ethane
(Compound No. 436)
α,α-difluoro-4-(trans-4-ethenylcyclohexyl)benzyloxy-2-(4-(trans-4-(3-butenyl)cyclohexyl)phenyl)ethane
(Compound No. 437)
α,α-difluoro-4-(trans-4-ethenylcyclohexyl)benzyloxy-2-(4-(trans-4-((E)-3-pentenyl)cyclohexyl)phenyl)ethane
(Compound No. 438)
α,α-difluoro-4-(trans-4-((E)-1-propenyl)cyclohexyl)benzyloxy-2-(4-(trans-4-ethenylcyclohexyl)phenyl)ethane
(Compound No. 439)
α,α-difluoro-4-(trans-4-((E)-1-propenyl)cyclohexyl)benzyloxy-2-(4-(trans-4-((E)-1-propenyl)cyclohexyl)phenyl) ethane
(Compound No. 440)
α,α-difluoro-4-(trans-4-((E)-1-propenyl)cyclohexyl)benzyloxy-2-(4-(trans-4-(3-butenyl)cyclohexyl)phenyl)ethane
(Compound No. 441)
α,α-difluoro-4-(trans-4-((E)-1-propenyl)cyclohexyl)benzyloxy-2-(4-(trans-4-((E)-3-pentenyl)cyclohexyl)phenyl) ethane
(Compound No. 442)
α,α-difluoro-4-(trans-4-(3-butenyl)cyclohexyl)benzyloxy-2-(4-(trans-4-ethenylcyclohexyl)phenyl)ethane
(Compound No. 443)
α,α-difluoro-4-(trans-4-(3-butenyl)cyclohexyl)benzyloxy-2-(4-(trans-4-((E)-1-propenyl)cyclohexyl)phenyl)ethane
(Compound No. 444)
α,α-difluoro-4-(trans-4-(3-butenyl)cyclohexyl)benzyloxy-2-(4-(trans-4-(3-butenyl)cyclohexyl)phenyl)ethane
(Compound No. 445)
α,α-difluoro-4-(trans-4-(3-butenyl)cyclohexyl)benzyloxy-2-(4-(trans-4-((E)-3-pentenyl)cyclohexyl)phenyl)ethane
(Compound No. 446)
α,α-difluoro-4-(trans-4-((E)-3-pentenyl)cyclohexyl)benzyloxy-2-(4-(trans-4-ethenylcyclohexyl)phenyl)ethane
(Compound No. 447)
α,α-difluoro-4-(trans-4-((E)-3-pentenyl)cyclohexyl)benzyloxy-2-(4-(trans-4-((E)-1-propenyl)cyclohexyl)phenyl) ethane
(Compound No. 448)
α,α-difluoro-4-(trans-4-((E)-3-pentenyl)cyclohexyl)benzyloxy-2-(4-(trans-4-(3-butenyl)cyclohexyl)phenyl)ethane

(Compound No. 449)
α,α-difluoro-4-(trans-4-((E)-3-pentenyl)cyclohexyl)benzyloxy-2-(4-(trans-4-((E)-3-pentenyl)cyclohexyl)phenyl) ethane
(Compound No. 450)
α,α-difluoro-2-fluoro-4-(trans-4-ethenylcycloheyxl)benzyloxy-2-(4-(trans-4-ethenylcyclohexyl)phenyl)ethane
(Compound No. 451)
α,α-difluoro-2-fluoro-4-(trans-4-ethenylcycloheyxl)benzyloxy-2-(4-(trans-4-((E)-1-propenyl)cyclohexyl)phenyl) ethane
(Compound No. 452)
α,α-difluoro-2-fluoro-4-(trans-4-ethenylcycloheyxl)benzyloxy-2-(4-(trans-4-(3-butenyl)cyclohexyl)phenyl)ethane
(Compound No. 453)
α,α-difluoro-2-fluoro-4-(trans-4-ethenylcycloheyxl)benzyloxy-2-(4-(trans-4-((E)-(3-pentenyl)cyclohexyl)phenyl) ethane
(Compound No. 454)
α,α-difluoro-2-fluoro-4-(trans-4-((E)-1-propenyl)cyclohexyl)benzyloxy-2-(4-(trans-4-ethenylcyclohexyl)phenyl) ethane
(Compound No. 455)
α,α-difluoro-2-fluoro-4-(trans-4-((E)-1-propenyl)cyclohexyl)benzyloxy-2-(4-(trans-4-((E)-1-propenyl)cyclohexyl) phenyl)ethane
(Compound No. 456)
α,α-difluoro-2-fluoro-4-(trans-4-((E)-1-propenyl)cyclohexyl)benzyloxy-2-(4-(trans-4-(3-butenyl)cyclohexyl)phenyl)ethane
(Compound No. 457)
α,α-difluoro-2-fluoro-4-(trans-4-((E)-1-propenyl)cyclohexyl)benzyloxy-2-(4-(trans-4-((E)-3-pentenyl)cyclohexyl) phenyl)ethane
(Compound No. 458)
α,α-difluoro-2-fluoro-4-(trans-4-(3-butenyl)cyclohexyl)benzyloxy-2-(4-(trans-4-ethenylcyclohexyl)phenyl)ethane
(Compound No. 459)
α,α-difluoro-2-fluoro-4-(trans-4-(3-butenyl)cyclohexyl)benzyloxy-2-(4-(trans-4-((E)-1-propenyl)cyclohexyl)phenyl)ethane
(Compound No. 460)
α,α-difluoro-2-fluoro-4-(trans-4-(3-butenyl)cyclohexyl)benzyloxy-2-(4-(trans-4-(3-butenyl)cyclohexyl)phenyl) ethane
(Compound No. 461)
α,α-difluoro-2-fluoro-4-(trans-4-(3-butenyl)cyclohexyl)benzyloxy-2-(4-(trans-4-((E)-3-pentenyl)cyclohexyl)phenyl)ethane
(Compound No. 462)
α,α-difluoro-2-fluoro-4-(trans-4-((E)-3-pentenyl)cyclohexyl)benzyloxy-2-(4-(trans-4-ethenylcyclohexyl)phenyl) ethane
(Compound No. 463)
α,α-difluoro-2-fluoro-4-(trans-4-((E)-3-pentenyl)cyclohexyl)benzyloxy-2-(4-(trans-4-((E)-1-propenyl)cyclohexyl) phenyl)ethane
(Compound No. 464)
α,α-difluoro-2-fluoro-4-(trans-4-((E)-3-pentenyl)cyclohexyl)benzyloxy-2-(4-(trans-4-(3-butenyl)cyclohexyl)phenyl)ethane
(Compound No. 465)
α,α-difluoro-2-fluoro-4-(trans-4-((E)-3-pentenyl)cyclohexyl)benzyloxy-2-(4-(trans-4-((E)-3-pentenyl)cyclohexyl) phenyl)ethane
(Compound No. 466)
α,α-difluoro-2-fluoro-4-(trans-4-ethenylcyclohexyl)benzyloxy-2-(4-(trans-4-methylcyclohexyl)phenyl)ethane
(Compound No. 467)
α,α-difluoro-2-fluoro-4-(trans-4-ethenylcyclohexyl)benzyloxy-2-(4-(trans-4-ethylcyclohexyl)phenyl)ethane
(Compound No. 468)
α,α-difluoro-2-fluoro-4-(trans-4-ethenylcyclohexyl)benzyloxy-2-(4-(trans-4-butylcyclohexyl)phenyl)ethane
(Compound No. 469)
α,α-difluoro-2-fluoro-4-(trans-4-ethenylcyclohexyl)benzyloxy-2-(4-(trans-4-pentylcyclohexyl)phenyl)ethane
(Compound No. 470)
α,α-difluoro-2-fluoro-4-(trans-4-ethenylcyclohexyl)benzyloxy-2-(4-(trans-4-hexylcyclohexyl)phenyl)ethane

(Compound No. 471)
α,α-difluoro-2-fluoro-4-(trans-4-ethenylcyclohexyl)benzyloxy-2-(4-(trans-4-heptylcyclohexyl)phenyl)ethane
(Compound No. 472)
α,α-difluoro-2-fluoro-4-(trans-4-((E)-1-propenyl)cyclohexyl)benzyloxy-2-(4-(trans-4-methylcyclohexyl)phenyl) ethane
(Compound No. 473)
α,α-difluoro-2-fluoro-4-(trans-4-((E)-1-propenyl)cyclohexyl)benzyloxy-2-(4-(trans-4-ethylcyclohexyl)phenyl) ethane
(Compound No. 474)
α,α-difluoro-2-fluoro-4-(trans-4-((E)-1-propenyl)cyclohexyl)benzyloxy-2-(4-(trans-4-propylcyclohexyl)phenyl) ethane
(Compound No. 475)
α,α-difluoro-2-fluoro-4-(trans-4-((E)-1-propenyl)cyclohexyl)benzyloxy-2-(4-(trans-4-pentylcyclohexyl)phenyl) ethane
(Compound No. 476)
α,α-difluoro-2-fluoro-4-(trans-4-((E)-1-propenyl)cyclohexyl)benzyloxy-2-(4-(trans-4-heptylcyclohexyl)phenyl) ethane
(Compound No. 477)
α,α-difluoro-2-fluoro-4-(trans-4-(3-butenyl)cyclohexyl)benzyloxy-2-(4-(trans-4-methylcyclohexyl)phenyl)ethane
(Compound No. 478)
α,α-difluoro-2-fluoro-4-(trans-4-(3-butenyl)cyclohexyl)benzyloxy-2-(4-(trans-4-ethylcyclohexyl)phenyl)ethane
(Compound No. 479)
α,α-difluoro-2-fluoro-4-(trans-4-(3-butenyl)cyclohexyl)benzyloxy-2-(4-(trans-4-propylcyclohexyl)phenyl)ethane
(Compound No. 480)
α,α-difluoro-2-fluoro-4-(trans-4-(3-butenyl)cyclohexyl)benzyloxy-2-(4-(trans-4-pentylcyclohexyl)phenyl)ethane
(Compound No. 481)
α,α-difluoro-2-fluoro-4-(trans-4-(3-butenyl)cyclohexyl)benzyloxy-2-(4-(trans-4-heptylcyclohexyl)phenyl)ethane
(Compound No. 482)
α,α-difluoro-2-fluoro-4-(trans-4-((E)-3-pentenyl)cyclohexyl)benzyloxy-2-(4-(trans-4-methylcyclohexyl)phenyl) ethane
(Compound No. 483)
α,α-difluoro-2-fluoro-4-(trans-4-((E)-3-pentenyl)cyclohexyl)benzyloxy-2-(4-(trans-4-ethylcyclohexyl)phenyl) ethane
(Compound No. 484)
α,α-difluoro-2-fluoro-4-(trans-4-((E)-3-pentenyl)cyclohexyl)benzyloxy-2-(4-(trans-4-propylcyclohexyl)phenyl) ethane
(Compound No. 485)
α,α-difluoro-2-fluoro-4-(trans-4-((E)-3-pentenyl)cyclohexyl)benzyloxy-2-(4-(trans-4-pentylcyclohexyl)phenyl) ethane
(Compound No. 486)
α,α-difluoro-2-fluoro-4-(trans-4-((E)-3-pentenyl)cyclohexyl)benzyloxy-2-(4-(trans-4-heptylcyclohexyl)phenyl) ethane

Example 6 (Use Example 1)

Nematic liquid crystal of the liquid crystal composition comprising the following compounds in the amount each shown below

| | |
|---|---|
| 4-(trans-4-propylcyclohexyl)benzonitrile | 24 % (% by weight, same rule applies to the followings) |
| 4-(trans-4-pentylcyclohexyl)benzonitrile | 36 % |
| 4-(trans-4-heptylcyclohexyl)benzonitrile | 25 % |
| 4-(4-propylphenyl)benzonitrile | 15 % |

had a clearing point (Cp) of 72.4°C. When this liquid crystal composition was filled in a TN cell (twisted nematic cell) having a cell thickness of 9 μm, it exhibited a driving threshold voltage ($V_{th}$) of 1.78 V, dielectric anisotropy value (Δε) of +11.0, optical anisotropy value (Δn) of 0.137, and viscosity at 20°C ($\eta_{20}$) of 27.0 mPa.s. Next, 85 parts of this liquid crystal composition was used as mother liquid crystal (hereinafter referred to as mother liquid crystal A), and it was

mixed with 15 parts of α,α-difluoro-4-(trans-4-propylcyclohexyl)benzyloxy-2-(3,4,5-trifluorophenyl)ethane (Compound No. 82) shown in Example 2, and then its physical properties were determined. As the result, it was found out that Cp was 65.1°C, $V_{th}$: 1.43 V, Δε : 11.6, Δn: 0.121, and $\eta_{20}$: 26.9 mPa·s. While this composition was left in a freezer at -20°C for 25 days, separation of crystals or development of smectic phase was not observed.

Example 7 (Use Example 2)

Mother liquid crystal A in an amount of 85 parts was mixed with 15 parts of α,α-difluoro-4-(trans-4-propylcyclohexyl)benzyloxy-2-(4-(trans-4-pentylcyclohexyl)phenyl)ethane (Compound No. 176) shown in Example 2 and its physical properties were determined. As the result, it was found out that Cp was 96.0°C, $V_{th}$: 1.91 V, Δε: 9.9, Δn: 0.129, $\eta_{20}$: 28.5 mPa·s. While this composition was left in a freezer at -20°C for 25 days, separation of crystals or development of smectic phase was not observed.

Comparative Example

As comparative compounds to those of the present invention, compound (11a) ($R=C_3H_7$) described in U.S. Patent No. 5,032,313, compound (14a) described in Laid-open Japanese Patent Publication No. Hei 5-310605, and four ring compound (50) described in Japanese Patent Publication No. Sho 62-46527 each described in the section of BACKGROUND ART were actually synthesized according to a preparation method described in each of the patent publications.

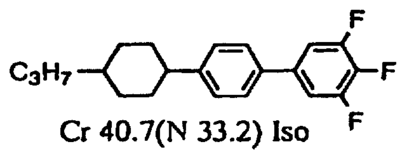

(11a)

Cr 40.7(N 33.2) Iso

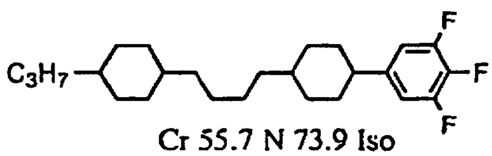

(14a)

Cr 55.7 N 73.9 Iso

Japanese Patent Publication No. Sho 62-46527

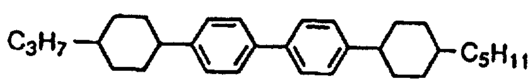

(50)

Cr 61.6 Sm 231.1 N 311.0 Iso

Using the compounds described above as comparative compound, 85 parts of the mother liquid crystal A described above was mixed with 15 parts of each comparative compound to prepare liquid crystal compositions anew and their physical properties were determined. Liquid crystal compositions prepared for the purpose of judging miscibility were left as they were in freezers at -20°C, and the number of days elapsed from the day when the liquid crystal compositions were put in the freezers to the day when crystals separated or smectic phase developed in liquid crystal compositions was counted. The physical properties thus obtained are shown in Table 2 together with the results of Examples 6 and 7 (numerals in the parentheses are extrapolated values from mother liquid crystal).

Table 2

| | Cp (°C) | Δε | η20 | Vth (V) | Mutual solubility |
|---|---|---|---|---|---|
| Mother liquid crystal A | 72.4 | 11.0 | 27.0 | 1.78 | >25 |
| 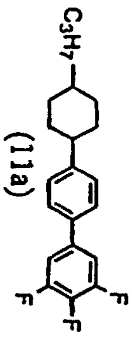 (11a) | 68.7 | 11.1 (11.7) | 28.8 (39.0) | 1.51 | 14 |
| 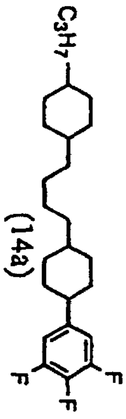 (14a) | 71.0 | 10.5 (7.5) | 27.8 (32.4) | 1.61 | >25 |
| 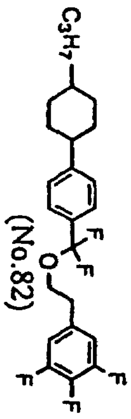 (No.82) | 65.1 | 11.6 (15.0) | 26.9 (26.7) | 1.43 | >25 |
| 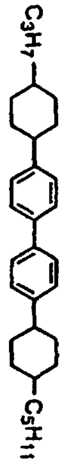 (50) | 100.8 | 9.5 (1.0) | 31.8 (59.0) | 2.02 | 12 |
| 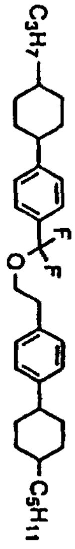 (No.176) | 96.0 | 9.9 (3.7) | 28.5 (36.9) | 1.91 | >25 |

From Table 2, it will be understood that the compound of the present invention (Compound No. 82) exhibits a large dielectric anisotropy value compared with compounds (11a) and (14a). It was more surprising to find out the

fact that whereas comparative compounds (11a) and (14a) caused the increase of viscosity of mother liquid crystal A when the formers were added to the latter, the compound of the present invention (Compound No. 82) did not raise the viscosity of the mother liquid crystal A, but more reduced only threshold voltage by 0.1-0.2 V than the comparative compounds.

On the other hand, it can be seen that in the comparison between the compound (Compound No. 176) of the present invention and comparative compound (50) having no central bonding group, the compound (50) has a considerably high clearing point and raised clearing point of mother liquid crystal A by nearly 30°C by mixing it in a ratio of 15 %. However, in the aspect of viscosity, it can be seen that the compound (50) raised viscosity even about 17.7 % compared with mother liquid crystal. In contrast to such facts, it can be seen that the compound (Compound No. 176) of the present invention raised only 5 % in viscosity compared with mother liquid crystal and thus the present compound is remarkably low in viscosity, whereas the present compound is slightly inferior in the capability of raising clearing point (about 24°C) to the comparative compound. Further, in the aspect of miscibility, it was confirmed that the compounds (Compound No. 82) and (Compound No. 176) of the present invention did not separate crystals or develop smectic phase over more than 3 weeks and thus had remarkably excellent miscibility at low temperatures, whereas separation of crystals was noticed in liquid crystal compositions in 2 weeks with comparative compounds (11a) and (50).

As demonstrated in Examples and Comparative Example, since the compounds of the present invention

1) exhibit a low threshold voltage,
2) are low in viscosity, and
3) have excellent characteristics in miscibility with other known liquid crystalline compounds, particularly in the miscibility at low temperatures,

liquid crystal compositions and liquid crystal display devices which can be driven at a low voltage and can respond at a high speed can be provided by using the compounds of the present invention as component of liquid crystal compositions.

INDUSTRIAL APPLICABILITY

Compounds of the present invention expressed by the general formula (1) are very stable against outside environments, are considerably low in viscosity, and have a high dielectric anisotropy. Besides, the present compounds are excellent in miscibility with other liquid crystal compounds, particularly in the solubility at low temperatures. Further, as the embodiments of the compounds of the present invention, the compounds expressed by the general formula (1) wherein an alkyl group is selected for $R^1$, and a halogen atom or groups other than CN group is selected for $R^2$ exhibit a high voltage holding ratio, and have preferable characteristics for liquid crystal materials used for TFT mode display devices, particularly for low voltage driving and high speed response. On the other hand, the compounds of the present invention wherein an alkenyl group or alkyl group is selected for $R^1$, and an alkenyl group or CN group is selected for $R^2$ are remarkably low in viscosity or have a high clearing point, and thus are very useful as viscosity reducing agent for STN display devices. By using the compounds of the present invention, liquid crystal compositions and liquid crystal display devices which are stable against outside environment, are capable of being driven at a low voltage, and are capable of responding at a high speed can be provided.

**Claims**

**1.** A liquid crystalline compound having a fluorine substituted bonding group and expressed by the general formula (1)

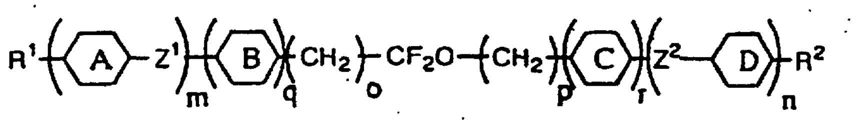

(1)

wherein $R^1$ and $R^2$ independently represent a straight chain or branched alkyl group having 1 to 15 carbon atoms or an alkenyl group having 2 to 15 carbon atoms, a halogen atom, CN group, $CF_3$ group, $OCF_3$ group, or $OCHF_2$ group, one or more hydrogen atoms in the alkyl or alkenyl group may be replaced by a halogen atom, and one or not-adjacent two or more $CH_2$ groups in the alkyl or alkenyl group may be replaced by oxygen atom, ring A, ring B, ring C, and ring D independently represent 1,4-cyclohexylene group or 1,4-phenylene group one or more hydrogen atoms on the ring may be replaced by a Fluorine atom; $Z^1$ and $Z^2$ independently represent $-(CH_2)_2-$,

-CH=CH-, -C≡C-, or single bond; m and n are independently an integer of 0 or 1; q and r are each 1; o and p are independently an integer of 0 to 2 provided that o + p = 2; and an element which constitutes the compound may be replaced by its isotope.

**2.** The compound according to claim 1 wherein o is 2 and p is 0 in the general formula (1).

**3.** The compound according to claim 1 wherein o is 1 and p is 1 in the general formula (1).

**4.** The compound according to claim 1 wherein o is 0 and p is 2 in the general formula (1).

**5.** The compound according to claim 1 wherein both ring B and ring C are 1,4-phenylene group.

**6.** The compound according to claim 4 wherein ring B is 1,4-phenylene group in the general formula (1).

**7.** A liquid crystal composition comprising at least two components and comprising at least one liquid crystalline compound defined in any one of the claims 1 to 6.

**8.** A liquid crystal composition according to claim 7, further comprising, as a second component, at least one compound selected from the group consisting of the compounds expressed by any one of the general formulas (2), (3), and (4)

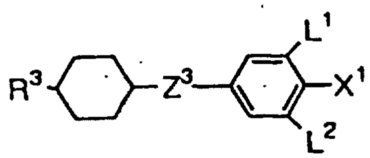

(2)

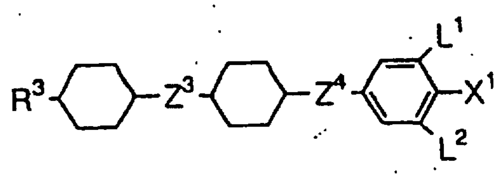

(3)

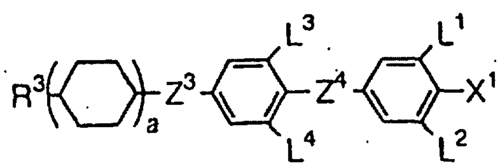

(4)

wherein $R^3$ represents an alkyl group having 1 to 10 carbon atoms; $X^1$ represents F, Cl, $OCF_3$, $OCF_2H$, $CF_3$, $CF_2H$, or $CFH_2$; $L^1$, $L^2$, $L^3$, and $L^4$ independently represent H or F; $Z^3$ and $Z^4$ independently represent $-(CH_2)_2-$, -CH=CH-, or single bond; and a is 1 or 2.

**9.** A liquid crystal composition according to claim 7, further comprising, as a second compound, at least one compound selected from the group consisting of the compounds expressed by any one of the general formulas (5), (6), (7), (8), and (9)

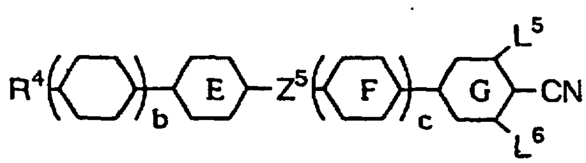

(5)

wherein $R^4$ represents F, an alkyl group having 1 to 10 carbon atoms, or an alkenyl group having 2 to 10 carbon atoms any methylene group ($-CH_2-$) in the alkyl or alkenyl group may be replaced by oxygen atom (-O-), but in no case two or more methylene groups are continuously replaced by oxygen atom; ring E represents trans-1,4-cyclohexylene group, 2,4-phenylene group, pyrimidine-2,5-diyl group, or 1,3-dioxane-2,5-diyl group; ring F represents trans-1,4-cyclohexylene group, 1,4-phenylene group, or pyrimidine-2,5-diyl group; ring G represents trans-1,4-cyclohexylene group or 1,4-phenylene group; $Z^5$ represents - $(CH_2)_2$-, -COO-, or single bond; $L^5$ and $L^6$ independently represent H or F; and b and c are independently 0 or 1,

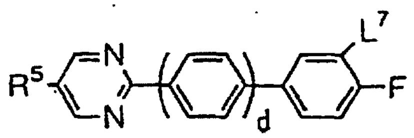

(6)

wherein $R^5$ represents an alkyl group having 1 to 10 carbon atoms, $L^7$ represents H or F; and d is 0 or 1,

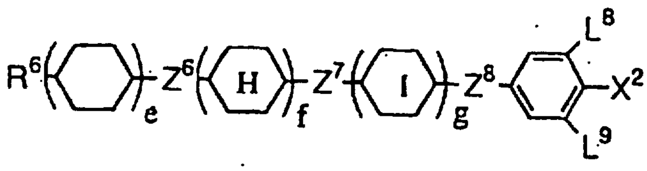

(7)

wherein $R^6$ represents an alkyl group having 1 to 10 carbon atoms; ring H and ring I independently represent trans-1,4-cyclohexylene group or 1,4-phenylene group; $Z^6$ and $Z^7$ independently represent -COO- or single bond; $Z^8$ represents - COO- or -C=C-; $L^8$ and $L^9$ independently represent H or F; $X^2$ represents F, $OCF_3$, $OCF_2H$, $CF_3$, $CF_2H$, or $CFH_2$ provided that when $X^2$ represents $OCF_3$, $OCF_2H$, $CF_3$, $CF_2H$, or $CFH_2$, then both $L^8$ and $L^9$ represent H; and e, f, and g are independently 0 or 1,

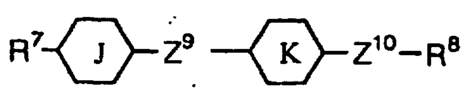

(8)

wherein $R^7$ and $R^8$ independently represent an alkyl group having 1 to 10 carbon atoms or an alkenyl group having 2 to 10 carbon atoms any methylene group ($-CH_2-$) in the alkyl or alkenyl group may be replaced by oxygen atom (-O-), but in no case two or more methylene groups are continuously replaced by oxygen atom; ring J represents trans-1,4-cyclohexylene group, 1,4-phenylene group, or pyrimidine-2,5-diyl group; ring K represents trans-1,4-cyclohexylene group or 1,4-phenylene group; $Z^9$ represents $-C\equiv C-$, -COO-, $-(CH_2)_2-$, $-CH=CH-C\equiv C-$, or single bond; and $Z^{10}$ represents -COO- or single bond;

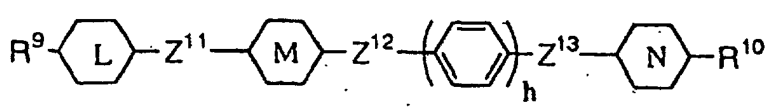

(9)

wherein $R^9$ and $R^{10}$ independently represent an alkyl group having 1 to 10 carbon atoms or an alkenyl group having 2 to 10 carbon atoms any methylene group ($-CH_2-$) in the alkyl or alkenyl group may be replaced by oxygen atom (-O-), but in no case two or more methylene groups are continuously replaced by oxygen atom; ring L represents trans-1,4-cyclohexylene group, 1,4-phenylene group, or pyrimidine-2,5-diyl group, ring M represents trans-1,4-cyclohexylene group, 1,4-phenylene group one or more hydrogen atoms on the ring may be replaced by F, or pyrimidine-2,5-diyl group; ring N represents trans-1,4-cyclohexylene group or 1,4-phenylene group; $Z^{11}$ and $Z^{13}$ independently represent -COO-, $-(CH_2)_2-$, or single bond; $Z^{12}$ represents -CH=CH-, $-C\equiv C-$, -COO-, or single bond; and h is 0 or 1.

**10.** A liquid crystal composition according to claim 7, further comprising, as a part of a second component, at least

one compound selected from the group consisting of the compounds expressed by any one of the general formulas (2), (3) and (4) according to claim 8 and comprising, as another part of the second component, at least one compound selected from the group consisting of the compounds expressed by any one of the general formulas (5), (6), (7), (8) and (9) according to claim 9.

**11.** A liquid crystal display device comprising a liquid crystal composition defined in any one of claims 7 to 10.

**Patentansprüche**

**1.** Flüssigkristalline Verbindung mit einer Fluorsubstituierten Bindegruppe und mit der allgemeinen Formel (1):

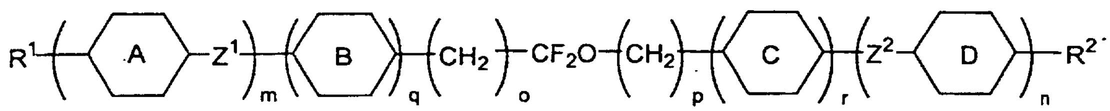

(1)

worin $R^1$ und $R^2$ unabhängig ein geradkettige oder verzweigte Alkylgruppe mit 1 bis 15 Kohlenstoffatomen oder eine Alkenylgruppe mit 2 bis 15 Kohlenstoffatomen, ein Halogenatom, CN-Gruppe, $CF_3$-Gruppe, $OCF_3$-Gruppe oder $OCHF_2$-Gruppe sind, wobei ein oder mehrere Wasserstoffatome in der Alkyl- oder Alkenylgruppe durch ein Halogenatom ersetzt sein können und ein oder nicht benachbarte zwei oder mehrere $CH_2$-Gruppen in der Alkyl- oder Alkenylgruppe durch Sauerstoffatom ersetzt sein können, Ring A, Ring, B, Ring C und Ring D unabhängig 1,4-Cyclohexylengruppe oder 1,4-Phenylengruppe sind, wobei ein oder mehrere Wasserstoffatome am Ring durch ein Fluoratom ersetzt sein können; $Z^1$ und $Z^2$ unabhängig - $(CH_2)_2$-, -CH=CH-, -C=C- oder eine Einfachbindung bedeuten; m und n unabhängig eine ganze Zahl von 0 oder 1 sind; q und r jeweils 1 sind; o und p unabhängig eine ganze Zahl von 0 bis 2 sind, vorausgesetzt, daß o + p = 2; und wobei ein Element, das die Verbindung ausmacht, durch sein Isotop ersetzt sein kann.

**2.** Verbindung nach Anspruch 1, worin in der allgemeinen Formel (1) p 2 und p 0 sind.

**3.** Verbindung nach Anspruch 1, worin in der allgemeinen Formel (1) o 1 und p 1 sind.

**4.** Verbindung nach Anspruch 1, worin in der allgemeinen Formel (1) o 0 und p 2 sind.

**5.** Verbindung nach Anspruch 1, worin der Ring B und Ring C jeweils eine 1,4-Phenylengruppe sind.

**6.** Verbindung nach Anspruch 4, worin in der allgemeinen Formel (1) der Ring B eine 1,4-Phenylengruppe ist.

**7.** Flüssigkristallzusammensetzung, umfassend zumindest zwei Komponenten und zumindest eine flüssigkristalline Verbindung wie in einem der Ansprüche 1 bis 6 definiert.

**8.** Flüssigkristallzusammensetzung nach Anspruch 7, weiterhin umfassend als zweite Komponente zumindest eine Verbindung, ausgewählt aus der Gruppe bestehend aus Verbindungen mit einer der allgemeinen Formeln (2), (3) und (4):

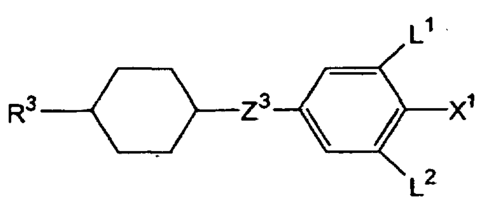

(2)

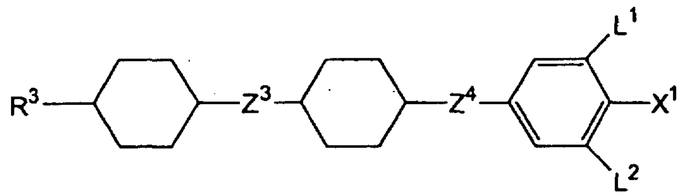

(3)

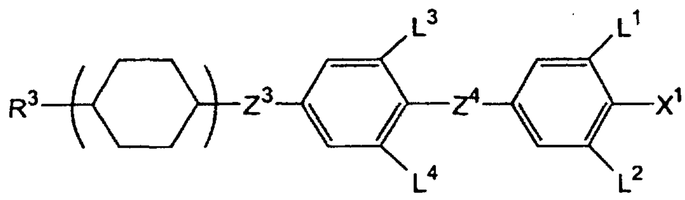

(4)

worin $R^3$ eine Alkylgruppe mit 1 bis 10 Kohlenstoffatomen ist; $X^1$ F, Cl, $OCF_3$, $OCF_2H$, $CF_3$, $CF_2H$ oder $CFH_2$ ist; $L^1$, $L^2$, $L^3$ und $L^4$ unabhängig H oder F sind; $Z^3$ und $Z^4$ unabhängig $-(CH_2)_2-$, -CH=CH- oder eine Einfachbindung sind; und a 1 oder 2 ist.

**9.** Flüssigkristallzusammensetzung nach Anspruch 7, weiterhin umfassend als zweite Verbindung zumindest eine Verbindung, ausgewählt aus der Gruppe bestehend aus Verbindungen mit einer der allgemeinen Formeln (5), (6), (7), (8) und (9):

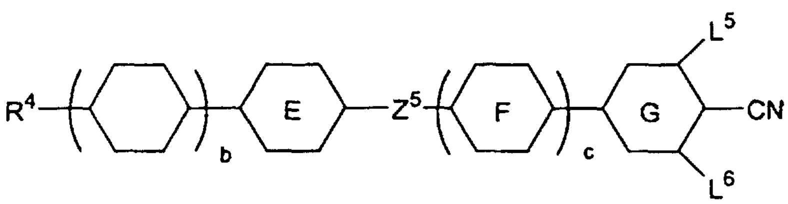

(5)

worin $R^4$ F, eine Alkylgruppe mit 1 bis 10 Kohlenstoffatomen oder Alkenylgruppe mit 2 bis 10 Kohlenstoffatomen ist, wobei jede Methylengruppe ($-CH_2-$) in der Alkyl- oder Alkenylgruppe durch Sauerstoffatom (-O-) ersetzt sein kann, wobei aber in keinem Fall zwei oder mehr Methylengruppen kontinuierlich durch Sauerstoffatom ersetzt sind; Ring E trans-1,4-Cyclohexylengruppe, 1,4-Phenylengruppe, Pyrimidin-2,5-diylgruppe oder 1,3-Dioxan-2,5-diylgruppe ist; Ring F trans-1,4-Cyclohexylengruppe, 1,4-Phenylengruppe oder Pyrimidin-2,5-diylgruppe ist; Ring G trans-1,4-, Cyclohexylengruppe oder 1,4-Phenylengruppe ist; $Z^5$ - $(CH_2)_2-$, -COO- oder Einfachbindung ist; $L^5$ und $L^6$ unabhängig H oder F bedeuten und b und c unabhängig 0 oder 1 sind;

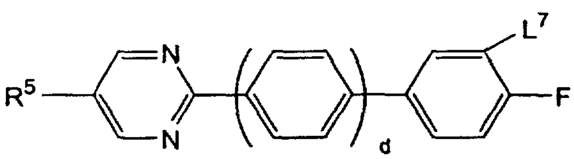

(6)

worin $R^5$ eine Alkylgruppe mit 1 bis 10 Kohlenstoffatomen ist, $L^7$ H oder F ist und d 0 oder 1 ist;

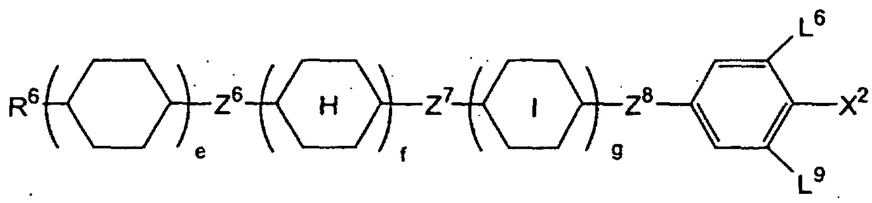

(7)

worin $R^6$ eine Alkylgruppe mit 1 bis 10 Kohlenstoffatomen ist, Ring H und Ring I unabhängig trans-1,4-Cyclohexylengruppe oder 1,4-Phenylengruppe sind; $Z^6$ und $Z^7$ unabhängig -COO- oder Einfachbindung sind; $Z^8$ -COO- oder -C≡C- ist; $L^8$ und $L^9$ unabhängig H oder F sind; $X^2$ F, $OCF_3$, $OCF_2H$, $CF_3$, $CF_2H$ oder $CFH_2$ ist, vorausgesetzt, daß dann, wenn $X^2$ $OCF_3$, $OCF_2H$, $CF_3$, $CF_2H$ oder $CFH_2$ ist, $L^8$ und $L^9$ jeweils H sind; und e, f und g unabhängig 0 oder 1 sind;

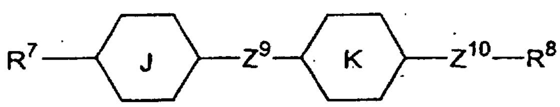

(8)

worin $R^7$ und $R^8$ unabhängig eine Alkylgruppe mit 1 bis 10 Kohlenstoffatomen oder eine Alkenylgruppe mit 2 bis 10 Kohlenstoffatomen sind, wobei jede Methylengruppe ($-CH_2-$) in der Alkyl- oder Alkenylgruppe durch Sauerstoffatom (-O-) ersetzt sein kann, aber in keinem Fall zwei oder mehr Methylengruppen kontinuierlich durch Sauerstoffatom ersetzt sind; Ring J trans-1,4-Cyclohexylengruppe, 1,4-Phenylgruppe oder Pyrimidin-2,5-diylgruppe ist; Ring K trans-1,4-Cyclohexylengruppe oder 1,4-Phenylengruppe ist; $Z^9$ -C≡C-, -COO-, $-(CH_2)_2-$, -CH=CH-C≡C- oder Einfachbindung ist und $Z^{10}$ -COO- oder Einfachbindung ist;

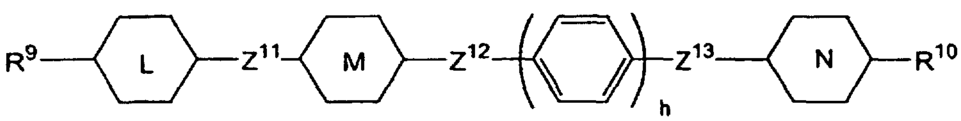

(9)

worin $R^9$ und $R^{10}$ unabhängig eine Alkylgruppe mit 1 bis 10 Kohlenstoffatomen oder eine Alkenylgruppe mit 2 bis 10 Kohlenstoffatomen sind, wobei jede Methylengruppe ($-CH_2-$) in der Alkyl- oder Alkenylgruppe durch Sauerstoffatom (-O-) ersetzt sein kann, aber in keinem Fall zwei oder mehr Methylengruppen kontinuierlich durch Sauerstoffatom ersetzt sind; Ring L trans-1,4-Cyclohexylengruppe, 1,4-Phenylgruppe oder Pyrimidin-2,5-diylgruppe ist; Ring M trans-1,4-Cyclohexylengruppe, 1,4-Phenylgruppe ist, worin ein oder mehrere Wasserstoffatome am Ring durch F ersetzt sein können; oder Pyrimidin-2,5-diylgruppe ist; Ring N trans-1,4-Cyclohexylengruppe oder 1,4-Phenylgruppe ist; $Z^{11}$ und $Z^{13}$ unabhängig -COO-, $-(CH_2)_2-$ oder Einfachbindung sind; $Z^{12}$ -CH=CH-, -C≡C-, -COO- oder Einfachbindung ist; und h 0 oder 1 ist.

10. Flüssigkristallzusammensetzung nach Anspruch 7, weiterhin umfassend als Teil einer zweiten Komponente zumindest eine Verbindung, ausgewählt aus der Gruppe bestehend aus den Verbindungen mit einer der allgemeinen Formeln (2), (3) und (4) nach Anspruch 8 und umfassend als anderen Teil der zweiten Komponente zumindest eine Verbindung, ausgewählt aus der Gruppe bestehend aus den Verbindungen mit einer der allgemeinen Formeln (5), (6), (7), (8) und (9) gemäß Anspruch 9.

11. Flüssigkristall-Anzeigevorrichtung, umfassend eine Flüssigkristallzusammensetzung wie in einem der Ansprüche 7 bis 10 definiert.

**Revendications**

1. Composé cristallin liquide ayant un groupe de liaison substitué avec du fluor et exprimé par la formule générale (1)

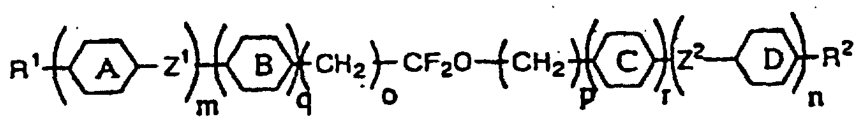

(1)

dans laquelle $R^1$ et $R^2$ représentent indépendamment un groupe alkyle à chaîne linéaire ou ramifiée ayant 1 à 15 atomes de carbone ou un groupa alcényle ayant 2 à 15 atomes de carbone, un atome d'halogène, un groupe CN, un groupe $CF_3$, un groupe $OCF_3$, ou un groupe $OCHF_2$, un ou plusieurs atomes d'hydrogène dans le groupe alkyle ou alcényle peuvent être remplacés par un atome d'halogène, et un ou deux groupes $CH_2$ non adjacents ou plus dans le groupe alkyle ou alcényle peuvent être remplacés par un atome d'oxygène ; le cycle A, le cycle B, le cycle C et le cycle D représentent indépendamment un groupe 1,4-cyclohexylène ou un groupe 1,4-phénylène, un ou plusieurs atomes d'hydrogène sur le cycle peuvent être remplacés par un atome de fluor ; $Z^1$ et $Z^2$ représentent indépendamment $-(CH_2)_2-$, -CH=CH-, -C≡C-, ou une liaison simple ; m et n sont indépendamment un nombre entier de 0 ou 1 ; q et r sont chacun 1 ; o et. p sont indépendamment un nombre entier de 0 à 2 à condition que o + p = 2 ; et un élément qui constitue le composé peut être remplacé par son isotope.

2. Composé selon la revendication 1 dans lequel o est 2 et p est 0 dans la formule générale (1).

3. Composé selon la revendication 1 dans lequel o est 1 et p est 1 dans la formule générale (1).

4. Composé selon la revendication 1 dans lequel o est 0 et p est 2 dans la formule générale (1).

5. Composé selon la revendication 1 dans lequel à la fois le cycle B et le cycle C sont un groupe 1,4-phénylène.

6. Composé selon la revendication 4 dans lequel le cycle B est un groupe 1,4-phénylène dans la formule générale (1).

7. Composition de cristaux liquides comprenant au moins deux composants et comprenant au moins un composé cristallin liquide défini dans l'une quelconque des revendications 1 à 6.

8. Composition de cristaux liquides selon la revendication 7, comprenant en plus, en tant que second composant, au moins un composé choisi dans le groupe constitué par des composés exprimés par l'une quelconque des formules générales (2), (3), et (4)

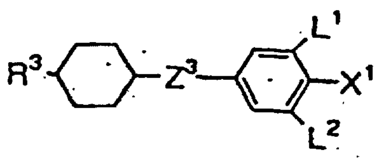

(2)

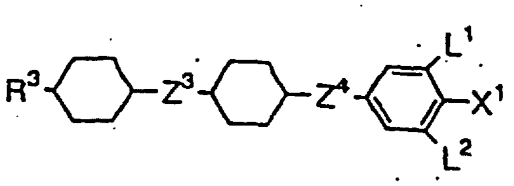

(3)

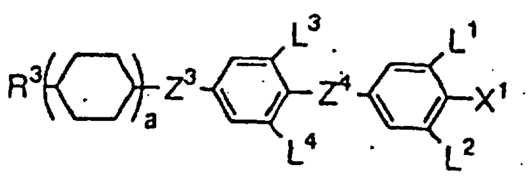

(4)

dans lesquelles $R^3$ représente un groupe alkyle ayant 1 à 10 atomes de carbone ; $X^1$ représente F, Cl, $OCF_3$, $OCF_2H$, $CF_3$, $CF_2H$, ou $CFH_2$ ; $L^1$, $L^2$, $L^3$ et $L^4$ représentent indépendamment H ou F ; $Z^3$ et $Z^4$ représentent indépendamment - $(CH_2)_2$-, -CH=CN-, ou une liaison simple, et a est 1 ou 2.

**9.** Composition de cristaux liquides selon la revendication 7, comprenant en plus, en tant que second composant, au moins un composé choisi dans le groupe constitué par des composés exprimés par l'une quelconque des formules générales (5), (6), (7), (8) et (9)

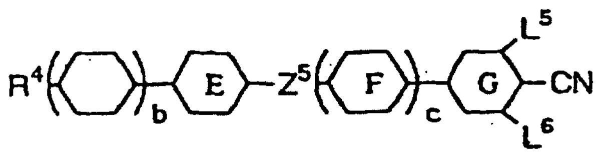

(5)

dans laquelle $R^4$ représente F, un groupe alkyle ayant 1 à 10 atomes de carbone, ou un groupe alcényle ayant 2 à 10 atomes de carbone n'importe quel groupe méthylène ($-CH_2$)- dans le groupe alkyle ou alcényle peut être remplacé par un atome d'oxygène (-O-), mais dans aucun cas deux groupes méthylène ou plus sont remplacés de manière continue par un atome d'oxygène ; le cycle E représente un groupe trans-1,4-cyclohexylène, un groupe 1,4-phénylène, un groupe pyrimidine-2,5-diyle, ou un groupe 1,3-dioxane-2,5-diyle ; le cycle F représente un groupe trans-1,4-cyclohexylène, un groupe 1,4-phénylène ou un groupe pyrimidine-2,5-diyle ; le cycle G représente un groupe trans-1,4-cyclohexylène ou un groupe 1,4-phénylène ; $Z^5$ représente $-(CH_2)_2-$, -COO-, ou une liaison simple ; $L^5$ et $L^6$ représentent indépendamment H ou F ; et b et c sont indépendamment 0 ou 1,

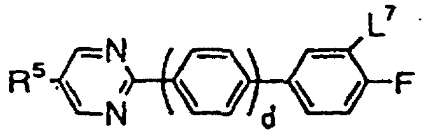

(6)

dans laquelle $R^5$ représente un groupe alkyle ayant 1 à 10 atomes de carbone, $L^7$ représente H ou F ; et d est 0 ou 1,

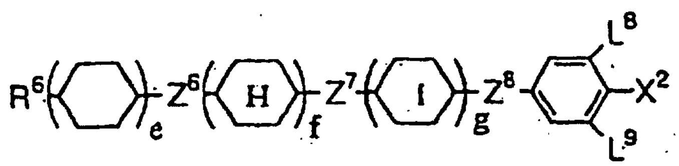

(7)

dans laquelle $R^6$ représente un groupe alkyle ayant 1 à 10 atomes de carbone ; le cycle H et le cycle I représentent indépendamment un groupe trans-1,4-cyclohexylène ou un groupe 1,4-phénylène ; $Z^6$ et $Z^7$ représentent indépendamment - COO- ou une liaison simple ; $Z^8$ représente -COO- ou -C≡C- ; $L^8$ et $L^9$ représentent indépendamment H ou F ; $X^2$ représente F, $OCF_3$, $OCF_2H$, $CF_3$, $CF_2H$, ou $CFH_2$ à condition que lorsque $X^2$ représente F, $OCF_3$, $OCF_2H$, $CF_3$, $CF_2H$, ou $CFH_2$, alors à la fois $L^8$ et $L^9$ représentent H ; et e, f et g sont indépendamment 0 ou 1,

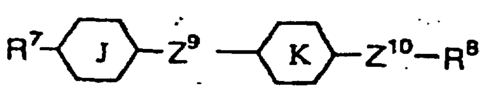

(8)

dans laquelle $R^7$ et $R^8$ représentent indépendamment un groupe alkyle ayant 1 à 10 atomes de carbone ou un groupe alcényle ayant 2 à 10 atomes de carbone n'importe quel groupe méthylène ($-CH_2$)- dans le groupe alkyle ou alcényle peut être remplacé par un atome d'oxygène (-O-), mais dans aucun cas deux groupes méthylène ou plus sont remplacés de manière continue par un atome d'oxygène ; le cycle J représente un groupe trans-1,4-cyclohexylène, un groupe 1,4-phénylène, ou un groupe pyrimidine-2,5-diyle ; le cycle K représente un groupe trans-1,4-cyclohexylène ou un groupe 1,4-phénylène ; $Z^9$ représente -C≡C-, -COO-, $-(CH_2)_2-$, -CH=CH-C≡C-, ou une liaison simple ; et $Z^{10}$ représente -COO- ou une liaison simple,

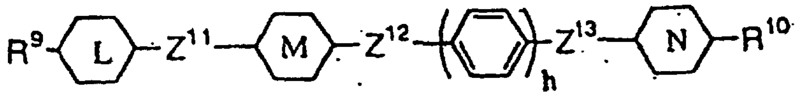

(9)

dans laquelle $R^9$ et $R^{10}$ représentent indépendamment un groupe alkyle ayant 1 à 10 atomes de carbone ou un groupe alcényle ayant 2 à 10 atomes de carbone n'importe quel groupe méthylène $(-CH_2)$- dans le groupe alkyle ou alcényle peut être remplacé par un atome d'oxygène (-O-), mais dans aucun cas deux groupes méthylène ou plus sont remplacés de manière continue par un atome d'oxygène ; le cycle L représente un groupe trans-1,4-cyclohexylène, un groupe 1,4-phénylène, ou un groupe pyrimidine-2,5-diyle ; le cycle M représente un groupe trans-1,4-cyclohexylène, un groupe 1,4-phénylène un ou plusieurs atomes d'hydrogène sur le cycle pouvant être remplacé par F, ou un groupe pyrimidine-2,5-diyle; le cycle N représente un groupe trans-1,4-cyclohexylène ou un groupe 1,4-phénylène ; $Z^{11}$ et $Z^{13}$ représentent indépendamment -COO-, - $(CH_2)_2$-, ou une liaison simple ; $Z^{12}$ représente -CH=CH-, -C≡C-, -COO-, ou une liaison simple ; et h est 0 ou 1.

**10.** Composition de cristaux liquides selon la revendication 7, comprenant en plus, en tant que partie d'un second composant, au moins un composé choisi dans le groupe constitué par des composés exprimés par l'une quelconque des formules générales (2), (3) et (4) selon la revendication 8 et comprenant, en tant qu'une autre partie du second composant, au moins un composé choisi dans le groupe constitué par des composés exprimés par l'une quelconque des formules générales (5), (6), (7), (8) et (9) selon la revendication 9.

**11.** Dispositif d'affichage à cristaux liquides comprenant une composition de cristaux liquides définie dans l'une quelconque des revendications 7 à 10.